(19) Europäisches Patentamt
European Patent Office
Office européen des brevets

(11) EP 3 545 974 A1

(12) **EUROPEAN PATENT APPLICATION**
published in accordance with Art. 153(4) EPC

(43) Date of publication:
**02.10.2019 Bulletin 2019/40**

(21) Application number: **17861068.9**

(22) Date of filing: **13.10.2017**

(51) Int Cl.:
***A61K 39/395*** *(2006.01)* ***A61K 47/68*** *(2017.01)*
***A61K 45/00*** *(2006.01)* ***A61K 31/4745*** *(2006.01)*
***A61K 31/537*** *(2006.01)* ***A61K 38/08*** *(2019.01)*
***A61K 38/06*** *(2006.01)* ***A61P 35/00*** *(2006.01)*

(86) International application number:
**PCT/CN2017/106044**

(87) International publication number:
**WO 2018/068758 (19.04.2018 Gazette 2018/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **14.10.2016 CN 201610898963**

(71) Applicants:
- **Suzhou Suncadia Biopharmaceuticals Co., Ltd.**
  **Suzhou, Jiangsu 215126 (CN)**
- **Jiangsu Hengrui Medicine Co., Ltd.**
  **Jiangsu 222047 (CN)**
- **Shanghai Hengrui Pharmaceutical Co., Ltd.**
  **Shanghai 200245 (CN)**

(72) Inventors:
- **SUN, Xing**
  **Lianyungang**
  **Jiangsu 222047 (CN)**
- **CAO, Guoqing**
  **Lianyungang**
  **Jiangsu 222047 (CN)**
- **TANG, Mi**
  **Lianyungang**
  **Jiangsu 222047 (CN)**
- **JIANG, Jiahua**
  **Lianyungang**
  **Jiangsu 222047 (CN)**
- **YANG, Changyong**
  **Lianyungang**
  **Jiangsu 222047 (CN)**
- **ZHANG, Lianshan**
  **Lianyungang**
  **Jiangsu 222047 (CN)**

(74) Representative: **Potter Clarkson**
**The Belgrave Centre**
**Talbot Street**
**Nottingham NG1 5GG (GB)**

# (54) MEDICAL USE OF ANTI-C MET ANTIBODY-CYTOTOXIC DRUG CONJUGATE

(57) Disclosed is the medical use of an anti-c Met antibody-cytotoxic drug conjugate. In particular, disclosed are an anti-c-Met antibody, an antigen-binding fragment of c-Met, a chimeric antibody and humanized antibody comprising the anti-c-Met antibody CDR region, and an antibody-cytotoxic drug conjugate thereof or pharmaceutically acceptable salt or solvate thereof, and the use of a pharmaceutical composition comprising the humanized anti-c-Met antibody and antigen-binding fragment and the antibody-cytotoxic drug conjugate thereof or the pharmaceutically acceptable salt or solvate thereof as an anti-hepatoma drug.

EP 3 545 974 A1

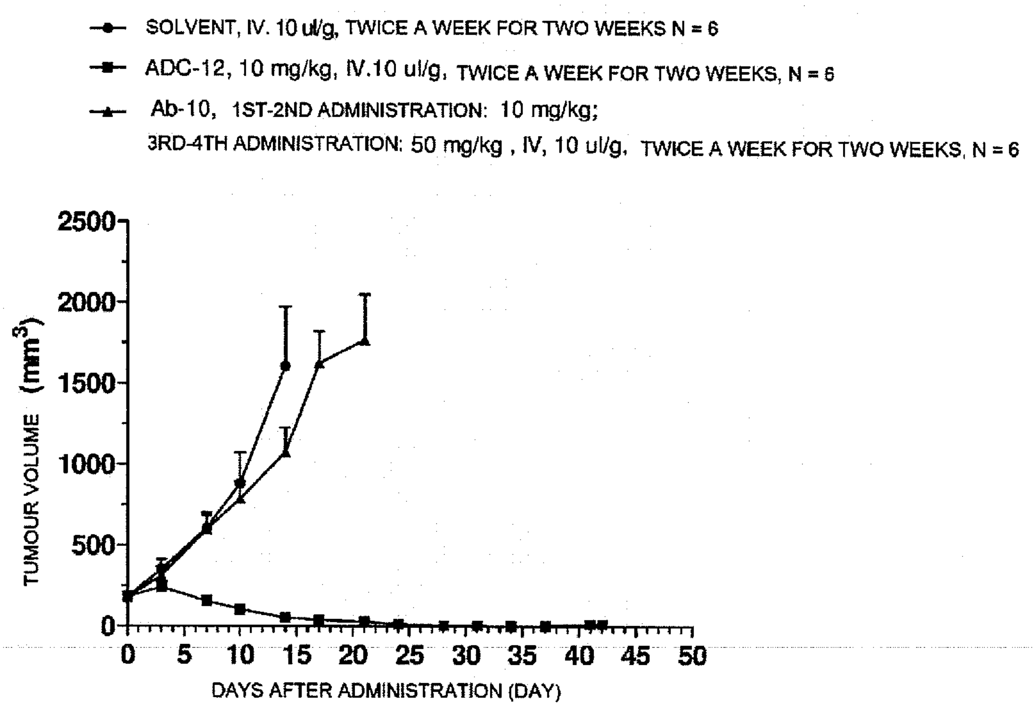
SOLVENT, IV. 10 ul/g, TWICE A WEEK FOR TWO WEEKS N = 6
ADC-12, 10 mg/kg, IV.10 ul/g, TWICE A WEEK FOR TWO WEEKS, N = 6
Ab-10, 1ST-2ND ADMINISTRATION: 10 mg/kg;
3RD-4TH ADMINISTRATION: 50 mg/kg , IV, 10 ul/g, TWICE A WEEK FOR TWO WEEKS, N = 6
TUMOUR VOLUME (mm3)
2500
2000
1500
1000
500
0
0
5
10
15
20
25
30
35
40
45
50
DAYS AFTER ADMINISTRATION (DAY)

Figure 1

## Description

### FIELD OF THE INVENTION

The present invention relates to the use of a c-Met antibody-cytotoxic drug conjugate or pharmaceutically acceptable salt or solvate thereof in the preparation of a medicament for treatment of hepatic carcinoma.

### BACKGROUND OF THE INVENTION

In recent years, molecular biology and tumor pharmacology studies have shown that tyrosine kinase (Protein Tyrosine Kinases, PTKs) related cell signalling pathway plays an extremely important role in tumor formation and development, more than 50% of proto-oncogenes and oncogene products have tyrosine kinase activity. The c-Met proto-oncogene belongs to the Ron subfamily of the PTKs family, and the encoded c-Met protein is a high affinity receptor for the Hepatocyte Growth Factor/Scatter Factor (HGF/SF). HGF/c-Met signaling pathway is closely related to the process of angiogenesis and tumor growth. The sustained activation of the same is an important cause of canceration of tissue cells or of hyperproliferation of cancer cells. The inhibition of this pathway has become a new method of tumor targeted therapy.

The c-Met proto-oncogene is located on human chromosome 7 long arm (7q31), which is more than 120kb in size and encodes c-Met protein precursor with molecular weight of about 150kD, a 170kD glycoprotein is produced by local glycosylation. The glycoprotein is further cleaved into one subunit (50 kDa) and the other subunit (140 kDa), which are linked by disulfide to form mature c-Met protein receptor. The heterodimer contains two strands, one comprises an extracellular domain, a transmembrane region (also called membrane stretch fragment), and an intracellular domain (comprising intracellular tyrosine kinase binding site). The other chain has extracellular portion only, but it is highly glycosylated and attached to the chain by disulfide bond. The extracellular region of the two subunits is the recognition site of corresponding ligand, and the intracellular domain has tyrosine kinase activity.

C-Met activation mechanism is divided into three types: one depends on the activation mechanism of HGF, the second does not depend on HGF activation mechanism, the third is through other membrane pathways, such as through the hyaluronic acid surface receptor CD44, adhesin and RON signalling pathway and so on. One of the most common is that dependent on the activation mechanism of HGF. The N-terminus of HGF binds to c-Met to promote the dimerization and autophosphorylation of Tyr1234 and Tyr1235 on the chain, and the phosphorylation of Tyr1349 and Tyrl356 near the C-terminus produces a binding site for multiple linker proteins which in turn induce P13K/Akt, Ras/Mapk, c-Src and STAT3/5-mediated activation of downstream signaling, and trigger different cellular responses, such as cell survival and activity (closely related to P13K/Akt pathway), tumor metastasis and cell proliferation (mainly mediated by Ras/Mapk). In addition, the cross-talk of c-Met with other membrane receptors has been known to promote tumor formation and metastasis. Since c-Met is the intersection of many pathways leading to tumor formation and metastasis, simultaneously interfering many pathways can be achieved relatively easily with c-Met as the target, and C-Met has become a promising target for antitumor formation and metastasis therapy.

Antibody drug conjugate (ADC) is formed by linking a monoclonal antibody or antibody fragment to a biologically active cytotoxin via a stable chemical linker, which fully utilizes the specificity of the antibody to specific tumor cell or highly expressed antigen, and the high efficiency of cytotoxins, avoid the toxic side effects on normal cells. This means that antibody drug conjugates can bind tumor cells precisely and reduce the effect on normal cells, when compared with conventional chemotherapeutic agents.

ADC consists of three parts: antibodies (target), linkers and toxins. Among them, a good target (antibody part) determines the specificity of the ADC drug, which includes not only specific targeting binding, but also effective endocytosis.

Currently, there are three main types of inhibitors for c-Met kinase targeting: HGF and c-Met biological antagonists, HGF and c-Met antibodies, and c-Met inhibitor small molecules. The existing clinical results show that the antibodies direct targeting HGF and c-Met, or c-Met small molecule inhibition is not ideal. The ADC for c-Met may be the most effective method for treating tumor. At present, there is no clinical research for c-Met ADC.

In PCT/CN2016/078699, the present inventor discloses a type of c-Met ADC drug, and expects its use for treatment of cancer. However, the use for treatment of hepatic carcinoma was not suggested.

### SUMMARY OF THE INVENTION

The technical problem to be solved by the present invention is the use of an antibody-cytotoxic drug conjugate (ADC) or pharmaceutically acceptable salt or solvate thereof in the preparation of medicament for treatment of hepatic carcinoma, wherein said antibody-cytotoxic drug conjugate (ADC) is served as the single component which has prominent anti-tumor activity and inhibits the proliferation of hepatic carcinoma cells effectively, thus providing a better clinical

application.

The technical solution of present invention is provided below:

The present invention provides the use of an antibody-cytotoxic drug conjugate or pharmaceutically acceptable salt or solvate thereof in the preparation of medicament for treatment of hepatic carcinoma, wherein said antibody-cytotoxic drug conjugate has a structure shown as formula (I):

$$Ab\text{-}[(L_2)t\text{-}L_1\text{-}D)]y \qquad (I)$$

wherein:

D is cytotoxic drug;
$L_1$, $L_2$ is linker unit;
t is 0 or 1, preferably 1;
y is 1-8, preferably 2-5;
Ab is antibody or antigen-binding fragment thereof that specifically binds to c-Met receptor, comprising at least one CDR region sequence selected from the following sequences or mutant sequence thereof:

antibody heavy chain variable region HCDR sequence: SEQ ID NO: 6, SEQ ID NO:7 or SEQ ID NO:8; and antibody light chain variable region LCDR sequence: SEQ ID NO: 9, SEQ ID NO: 10 or SEQ ID NO: 11.

Preferably, the antibody heavy chain variable region comprises at least one HCDR region sequence selected from the following sequences or mutant sequence thereof: SEQ ID NO: 6, SEQ ID NO: 7 and SEQ ID NO: 8.

Preferably, the antibody light chain variable region comprises at least one LCDR region sequence selected from the following sequences or mutant sequence thereof: SEQ ID NO: 9, SEQ ID NO: 10 and SEQ ID NO: 11.

In a preferred embodiment of the present invention, the antibody comprises heavy chain variable region sequences SEQ ID NO: 6, SEQ ID NO: 7 and SEQ ID NO: 8, or mutant sequence thereof, and light chain variable region sequences SEQ ID NO: 9, SEQ ID NO: 10 and SEQ ID NO: 11, or mutant sequence thereof.

The mutant sequences are sequences which have 1-3 amino acid mutations in CDRs that optimize antibody activity, wherein the mutant sequence of HCDR2 region preferably is SEQ ID NO: 12.

The antibody or antigen-binding fragment thereof that specifically binds to c-Met receptor is murine antibody or fragment thereof.

The heavy chain variable region sequence of the murine antibody is shown as SEQ ID NO: 4.

The light chain variable region sequence of the murine antibody is shown as SEQ ID NO: 5.

In a preferred embodiment of the present invention, the heavy chain variable region of the murine antibody is shown as SEQ ID NO: 4, and the light chain variable region of the murine antibody is shown as SEQ ID NO: 5.

In a preferred embodiment of the present invention, the antibody or antigen-binding fragment thereof that specifically binds to c-Met receptor is chimeric antibody or humanized antibody or fragment thereof.

The humanized antibody heavy chain variable region comprises a heavy chain FR region derived from human germline heavy chain sequence, preferably human germline heavy chain IGHV 3-33*01; wherein said heavy chain FR region comprises framework sequence of FR1, FR2, FR3 and FR4 regions of human germline heavy chain IGHV 3-33*01, or mutant sequence thereof, preferably the mutant sequence is(are) 0-10 amino acid back-mutation(s).

The humanized antibody comprises a heavy chain variable region sequence selected from SEQ ID NO: 13-15 or variant thereof.

The humanized antibody light chain variable region comprises a light chain FR region derived from human germline light chain sequence, preferably human germline light chain IGKV085 or IGKV4-1*01; wherein said light chain FR region comprises framework sequence of FR1, FR2, FR3 and FR4 regions of human germline light chain IGKV085 and IGKV4-1*01, or mutant sequence thereof, preferably the mutant sequence is(are) 0-10 amino acid back-mutation(s).

In a preferred embodiment of the present invention, the humanized antibody comprises a light chain variable region sequence selected from SEQ ID NO: 16-18 or variant thereof

In a preferred embodiment of the present invention, the humanized antibody comprises a heavy chain variable region sequence selected from SEQ ID NO: 13-15 and a light chain variable region sequence selected from SEQ ID NO: 16-18.

In a preferred embodiment of the present invention, said antibody or antigen-binding fragment thereof that specifically binds to c-Met receptor comprises a combination of heavy chain variable region sequence and light chain variable region sequence selected from any one of a) to c):

a) Heavy chain variable region sequence of SEQ ID NO: 13, and light chain variable region sequence of SEQ ID NO: 16;
b) Heavy chain variable region sequence of SEQ ID NO: 14, and light chain variable region sequence of SEQ ID

NO: 17; or

c) Heavy chain variable region sequence of SEQ ID NO: 15, and light chain variable region sequence of SEQ ID NO: 18.

In a preferred embodiment of the present invention, the heavy chain constant region of the humanized antibody comprises a constant region derived from human IgG1 or variant thereof, human IgG2 or variant thereof, human IgG3 or variant thereof, or human IgG4 or variant thereof, preferably comprises a constant region derived from human IgG1 or variant thereof, human IgG2 or variant thereof or human IgG4 or variant thereof, more preferably a constant region derived from human IgG2 or variant thereof.

In a preferred embodiment of the present invention, said antibody or antigen-binding fragment thereof that specifically binds to c-Met receptor comprises a full length heavy chain sequence selected from SEQ ID NO: 23-25 or sequences having at least 90% identity to SEQ ID NO: 23-25.

In a preferred embodiment of the present invention, the light chain constant region of humanized antibody comprises a constant region selected from human κ or λ or variant thereof.

The antibody or antigen-binding fragment thereof that specifically binds to c-Met receptor comprises a full-length light chain sequence selected from SEQ ID NO: 26-28 or sequences having at least 90% identity to SEQ ID NO: 26-28.

The humanized antibody comprises a combination of full-length light chain sequence and full-length heavy chain sequence selected from:

Ab-9: heavy chain sequence of SEQ ID NO: 23 and light chain sequence of SEQ ID NO: 26;
Ab-10: heavy chain sequence of SEQ ID NO: 24 and light chain sequence of SEQ ID NO: 27; or
Ab-11: heavy chain sequence of SEQ ID NO: 25 and light chain sequence of SEQ ID NO: 28.

The present invention further provides the use of a pharmaceutical composition in the preparation of medicament for treatment of hepatic carcinoma, wherein said pharmaceutical composition comprises the c-Met antibody or antigen-binding fragment thereof described above and one or more pharmaceutically acceptable excipient, diluent or carrier.

In a preferred embodiment of the present invention, wherein said $-L_2-$ is a compound shown as formula ($-L_2-$):

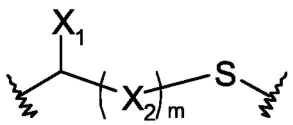

,

wherein:

$X_1$ is selected from the group consisting of hydrogen, halogen, hydroxyl, cyano, $C_{1-6}$ alkyl, $C_{1-6}$ alkoxy or 3-8 membered cycloalkyl;

$X_2$ is selected from the group consisting of $C_{1-6}$ alkyl, 3-8 membered cycloalkyl or 3-8 membered heterocyclyl;

m is 0-5, preferably 1-3; S is sulfur atom.

Preferably, said cytotoxic drug unit of D is a cytotoxic agent selected from toxins, chemotherapeutic agents, antibiotics, radioisotopes or nucleolytic enzyme.

In a preferred embodiment of the present invention, the D is a compound shown as formula (D):

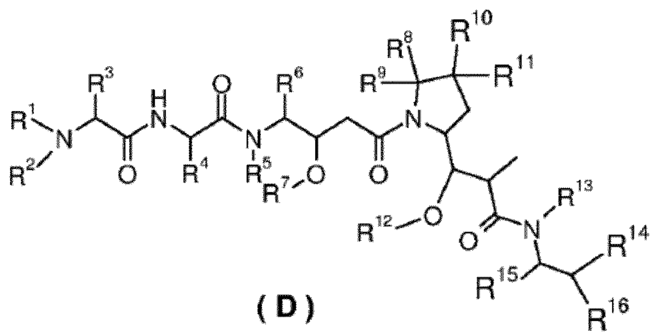

(D)

or tautomer, mesomer, racemate, enantiomer, diastereomer, or mixtures thereof, or pharmaceutically acceptable salt thereof:
wherein:

$R^1$, $R^2$, $R^3$, $R^4$, $R^5$, $R^6$, $R^7$ is each selected from the group consisting of hydrogen, halogen, hydroxyl, cyano, $C_{1-6}$ alkyl, $C_{1-6}$ alkoxy or 3-8 membered cycloalkyl;
$R^8$, $R^9$, $R^{10}$, $R^{11}$ is each selected from the group consisting of hydrogen, halogen, $C_{2-6}$ alkenyl, $C_{1-6}$ alkyl, $C_{1-6}$ alkoxy or 3-8 membered cycloalkyl; preferably at least one group is selected from halogen, $C_{2-6}$ alkenyl, $C_{1-6}$ alkyl or 3-8 membered cycloalkyl; the rest group(s) is(are) hydrogen;
or, any two of $R^8$, $R^9$, $R^{10}$, $R^{11}$ form 3-8 membered cycloalkyl, the rest two are each selected from the group consisting of hydrogen, $C_{1-6}$ alkyl and 3-8 membered cycloalkyl;
$R^{12}$, $R^{13}$ is each selected from the group consisting of hydrogen, $C_{1-6}$ alkyl and halogen;
$R^{14}$ is selected from 6-14 membered aryl or 5-15 membered heteroaryl, wherein the aryl or heteroaryl is optionally further substituted by substituent selected from the group consisting of hydrogen, halogen, hydroxy, $C_{1-6}$ alkyl, $C_{1-6}$ alkoxy and 3-8 membered cycloalkyl;
$R^{15}$ is selected from the group consisting of halogen, $C_{2-6}$ alkenyl, $C_{1-6}$ alkyl, 3-8 membered cycloalkyl, carboxyl, $C_{1-6}$ alkyl carbonyl and $C_{1-6}$ alkoxy carbonyl;
$R^{16}$ is selected from the group consisting of hydrogen, halogen, hydroxy, cyano, alkyl, $C_{1-6}$ alkoxy and 3-8 membered cycloalkyl.

Preferably, $L_2$ comprises a linker selected from the group consisting of Val-Cit, MC, PAB and MC-PAB, preferably MC.
Particularly preferably, D is maytansinoids; preferably DM1, DM3 and DM4; more preferably DM1.
Preferably, $L_2$ is selected from the group consisting of N-succinimidyl 4-(2-pyridylthio) valerate (SPP), N-succinimidyl 4-(N-maleimidomethyl)-cyclohexane-1-carboxylate (SMCC) or N-succinimidyl (4-iodo-acetyl) aminobenzoate (SIAB); preferably N-succinimidyl 4-(2-pyridylthio) valerate or N-succinimidyl 4- (N-maleimidomethyl) -cyclohexane-1-carboxylate.
Further preferably, D is a camptothecin alkaloid which is selected from the group consisting of CPT, 10-hydroxy-CPT, Irinotecan, SN-38 or topotecan, more preferably SN-38.
Particularly preferably, the linker $L_2$ is selected from the group consisting of Val-Cit, MC, PAB or MC-PAB; preferably MC or MC-vc-PAB.
In a preferred embodiment of the present invention, said antibody-cytotoxic drug conjugate is a conjugated drug of formula (II) or pharmaceutically acceptable salt or solvate thereof:

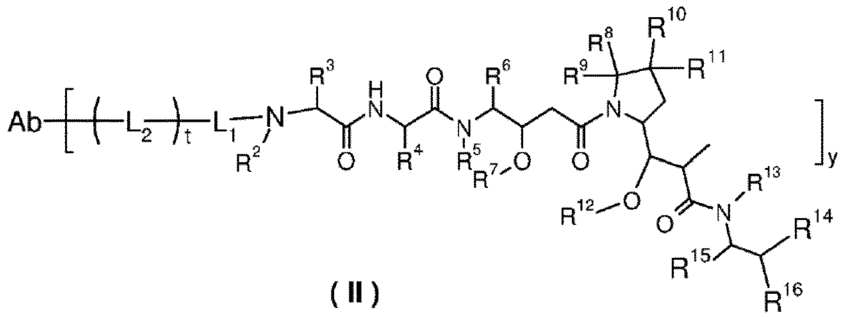

(II)

wherein:

$R^2$-$R^{16}$ are as defined in formula (D);
Ab, t, y, $L_1$, $L_2$ are as defined in formula (I).

In a preferred embodiment of the present invention, said antibody-cytotoxic drug conjugate is a conjugated drug of formula (III) or pharmaceutically acceptable salt or solvate thereof:

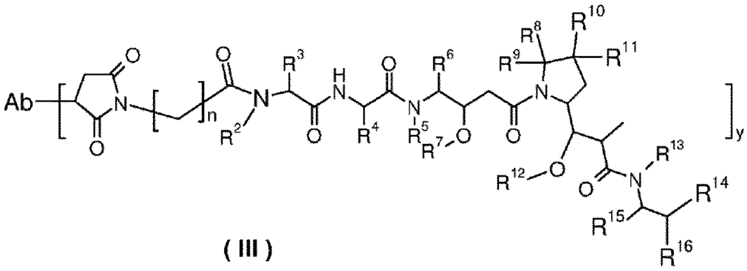

( III )

wherein:

$R^2$-$R^{16}$ are as defined in formula (D);
Ab, y are as defined in formula (I);
n is 3-6, preferably 5.

In a preferred embodiment of the present invention, said antibody-cytotoxic drug conjugate is a conjugated drug of formula (IV) or pharmaceutically acceptable salt or solvate thereof:

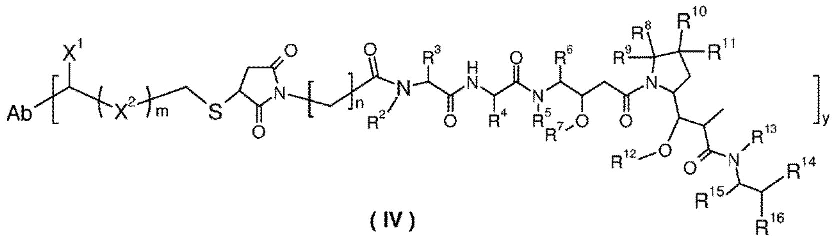

( IV )

wherein:

$R^2$-$R^{16}$ are as defined in formula (D);
Ab, y are as defined in formula (I);
n is as defined in formula (III);
$X^1$, $X^2$, m are as defined in formula L2.

In a preferred embodiment of the present invention, said antibody-cytotoxic drug conjugate is a conjugated drug of formula (V) or pharmaceutically acceptable salt or solvate thereof:

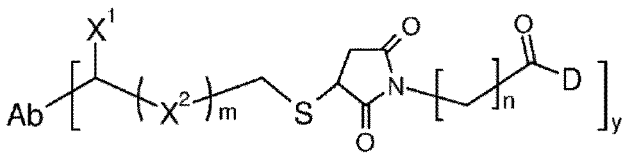

( V )

wherein:

Ab, D, y are as defined in formula (I);

n is as defined in formula (III);

$X^1$, $X^2$, m are as defined in formula L2.

In a preferred embodiment of the present invention, said antibody-cytotoxic drug conjugate or pharmaceutically acceptable salt or solvate thereof is selected from the group consisting of:

| No. | Structure and Name |
|---|---|
| 1 | 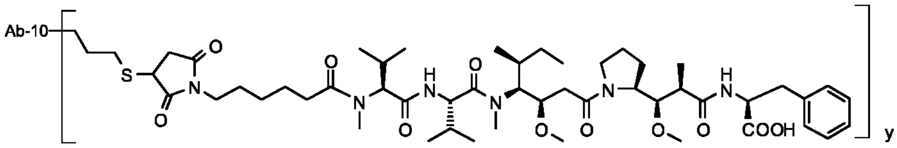 ADC-1 |
| | Anti c-Met antibody Ab-10 conjugated with toxin MC-MMAF |
| 2 | 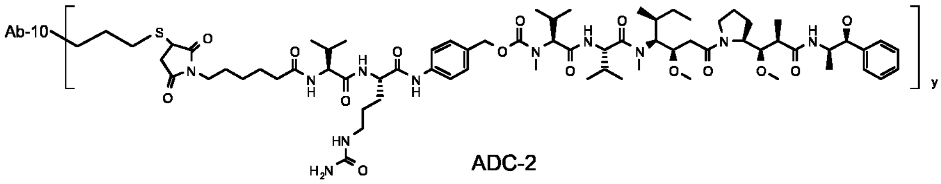 ADC-2 |
| | Anti c-Met antibody Ab-10 conjugated with toxin MC-VC-PAB-MMAE |
| 3 | 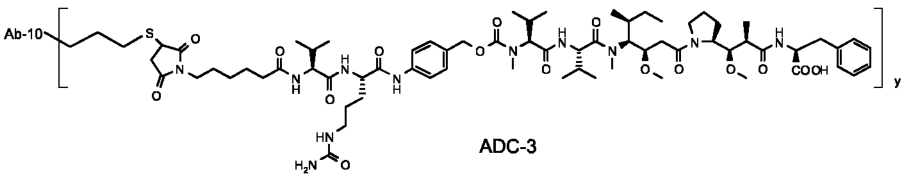 ADC-3 |
| | Anti c-Met antibody Ab-10 conjugated with toxin MC-VC-PAB-MMAF |
| 4 | 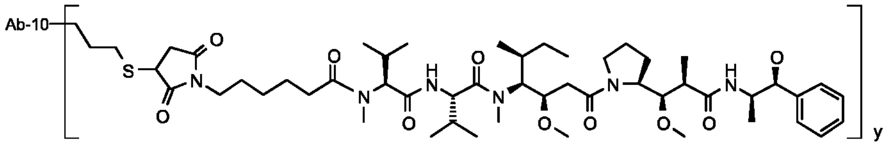 ADC-4 |
| | Anti c-Met antibody Ab-10 conjugated with toxin MC-MMAE |
| 5 | 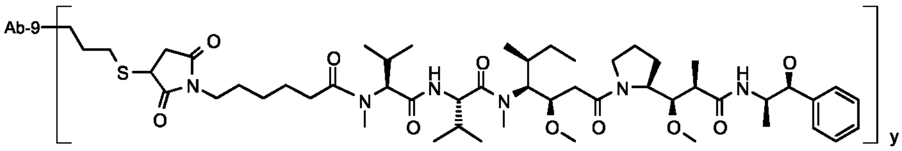 ADC-5 |
| | Anti c-Met antibody Ab-9 conjugated with toxin MC-MMAE |
| 6 | 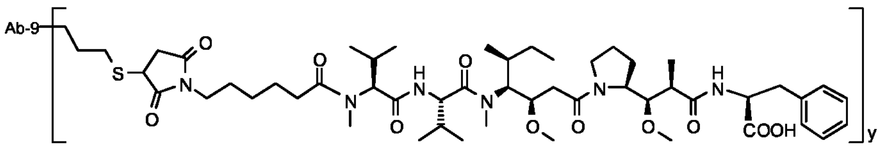 ADC-6 |
| | Anti c-Met antibody Ab-9 conjugated with toxin MC-MMAF |

(continued)

| No. | Structure and Name |
|---|---|
| 7 | 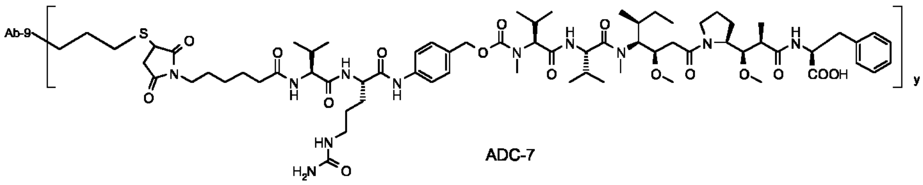 ADC-7 |
| | Anti c-Met antibody Ab-9 conjugated with toxin MC-VC-PAB-MMAF |
| 8 | 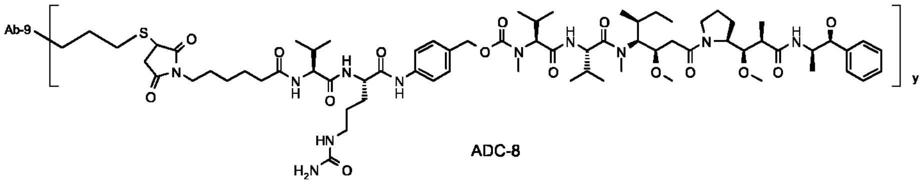 ADC-8 |
| | Anti c-Met antibody Ab-9 conjugated with toxin MC-VC-PAB-MMAE |
| 9 | 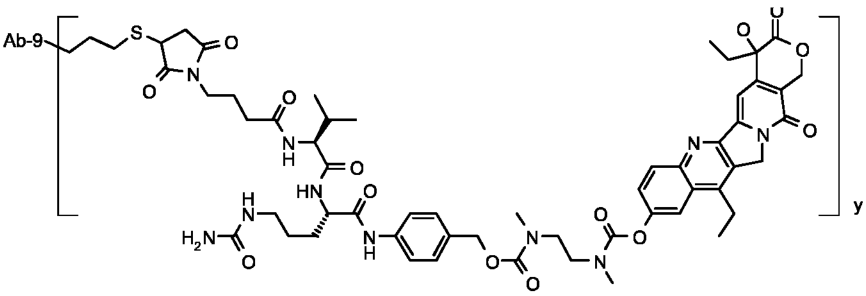 ADC-11 |
| | Anti c-Met antibody Ab-9 conjugated with toxin -SN-38 |
| 10 | 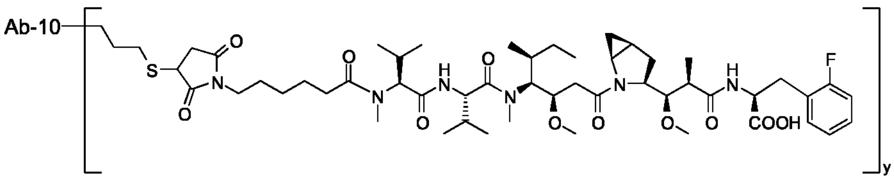 ADC-12 |
| | Anti c-Met antibody Ab-10 conjugated with toxin |
| 11 | 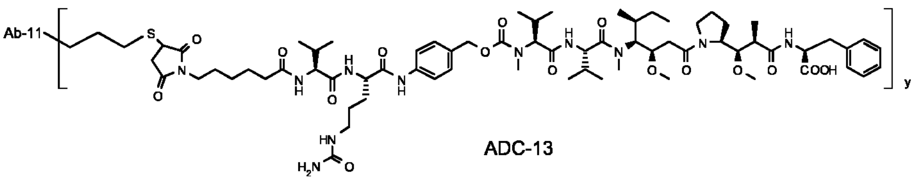 ADC-13 |
| | Anti c-Met antibody Ab-11 conjugated with toxin MC-VC-PAB-MMAF |

(continued)

| No. | Structure and Name |
|---|---|
| 12 | 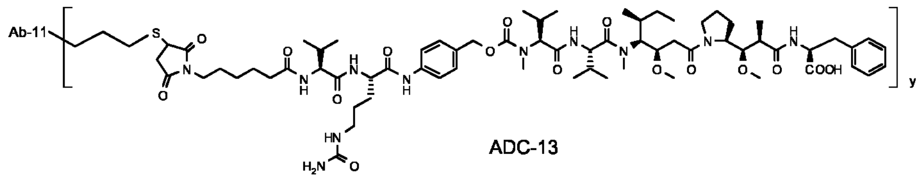 ADC-13 |
| | Anti c-Met antibody Ab-11 conjugated with toxin MC-MMAE |
| Ab-9, Ab-10, Ab-11 are c-Met antibodies described above, y is 1-8, preferably 2-5. Wherein, y ranges from 1-8, preferably 1-4. | |

In a preferred embodiment of the present invention, the cancer cells of hepatic carcinoma is c-Met expression positive or overexpresses c-Met, preferably ≥ 20% of hepatic carcinoma cells are positive/weak positive; further preferably ≥ 25% of hepatic carcinoma cells are positive; more preferably ≥ 50% of hepatic carcinoma cells are strongly positive.

## DETAILED DESCRIPTION OF THE INVENTION

1. Terms

In the specification and claims of present invention, unless specifically defined elsewhere in this document, the scientific and technical terms used herein have the meaning commonly understood by ordinary skilled in the art. However, in order to make the invention more readily understood, the definition and explanation of certain related terms are specifically provided below. Further, when the definition and explanation of the terms provided by the present application are inconsistent with the meanings generally understood by those skilled in the art, the definition and explanation of the terms provided by the present application shall prevail.

As used herein, the three-letter code and single-letter code for amino acids are as described in J. biol. chem, 243, p3558 (1968).

The term "c-Met" or "c-Met polypeptide" or "c-Met receptor" refers to a receptor tyrosine kinase that binds to a hepatocyte growth factor (HGF). In the present invention, unless specified specifically, such as murine c-Met (m-c-Met) or monkey c-Met (cyno-c-Met), the term "c-Met" usually refers to human c-Met (h-c-Met). The human, murine and cynomolgus monkey c-Met used in the present invention are encoded by the nucleotide sequence or polypeptide sequence provided by GenBank, for example, the human polypeptide was encoded by the nucleotide sequence provided in GenBank Accession No. NM_000245, or the human protein or its extracellular domain encoded by the polypeptide sequence provided in GenBank the Accession No. NP_000236. The original single-stranded precursor proteins are cleaved after translation to produce alpha and beta subunits, which are linked by disulfide bonds to form mature receptor. Receptor tyrosine kinase c-Met involves in cell processes including, for example, the process of migration, invasion and morphogenesis of tissue regeneration associated with embryogenesis.

The term "c-Met-related disorder or condition" refers to any disease, disorder or condition originated from adverse expression or lack of c-Met expression, adverse regulation or lack of regulation, or deleterious activity or lack of activity, or refers to any disease, disorder or condition which could be regulated, treated or cured by modulating c-Met expression or activity. The activation of the HGF/c-Met pathway can be expected, for example, in most cancer patients, or in patients whose disease is indeed driven by changes associated with the c-Met pathway. For example, upregulation is due to different mechanisms, such as overexpression of HGF and/or c-Met, or by constitutive activation of c-Met mutations. C-Met-related disorders or conditions include, but are not limited to, such as proliferative diseases and disorders and inflammatory diseases and disorders. Proliferative diseases include, but are not limited to, for example, cancer, including, for example, gastric cancer, esophageal cancer, breast cancer, kidney cancer including papillary renal cell carcinoma, lung cancer, glioma, head and neck cancer, epithelial cancer, skin cancer, leukemia, lymphoma, myeloma, brain cancer, pancreatic cancer, colorectal cancer, gastrointestinal cancer, intestinal cancer, genital cancer, urinary cancer, melanoma, prostate cancer, and other tumors known to those skilled in the art. Inflammatory diseases include, but are not limited to bacterial infections, including infections caused by *Listeria* bacteria.

"Antibody" in this invention refers to immunoglobulin, a four-peptide chain structure formed by two identical heavy chains and two identical light chains connected by interchain disulfide bond. Different immunoglobulin heavy chain constant regions exhibit different amino acid compositions and sequences, hence present different kinds of antigenicity. Accordingly, immunoglobulins can be divided into five categories, also referred as immunoglobulin isotypes, namely

IgM, IgD, IgG, IgA and IgE; the corresponding heavy chains thereof are μ chain, δ chain, y chain, α chain, ε chain, respectively. According to amino acid composition of hinge region and the number and location of heavy chain disulfide bonds, immunoglobulins can be divided into different sub-categories, for example, IgG can be divided into IgG1, IgG2, IgG3, and IgG4. Light chain can be divided into κ or λ chain, based on different constant region. Each category of Ig among these five categories involves κ or λ chain.

Near the N-terminus of the antibody heavy and light chains, about 110 amino acids vary largely, which are known as variable region (V region); the amino acid sequence near the C-terminus is relatively stable, which is known as constant region (C region). Variable region comprises three hypervariable regions (HVR) and four framework regions (FR) with relatively conserved sequence. Three hypervariable regions determine the specificity of the antibody, also known as complementarity determining region (CDR). Each light chain variable region (LCVR) and each heavy chain variable region (HCVR) is composed of three CDR regions and four FR regions, arranged from the amino terminus to the carboxyl terminus is: FR1, CDR1, FR2, CDR2, FR3, CDR3, and FR4. Three light chain CDR regions refer to LCDR1, LCDR2, and LCDR3; three heavy chain CDR regions refer to HCDR1, HCDR2 and HCDR3. The number and location of CDR region amino acid residues in LCVR and HCVR regions of the antibody or antigen binding fragment herein comply with the known Kabat numbering criteria (LCDR1-3, HCDE2-3), or comply with kabat and chothia numbering criteria (HCDR1).

The term "murine antibody" in the present invention refers to anti-human c-Met monoclonal antibody prepared from mouse according to the knowledge and skills in the art. During the preparation, test subject was injected with c-Met antigen, and then isolated the hybridoma expressed the antibody which possesses desired sequence or functional characteristics. In a preferred embodiment of the present invention, the murine c-Met antibody or antigen binding fragment thereof, further comprises light chain constant region of murine κ, λ chain or variant thereof, or further comprises heavy chain constant region of murine IgG1, IgG2, IgG3 or IgG4, or variant thereof.

The term "chimeric antibody", is an antibody which is obtained by fusing the variable region of murine antibody to constant region of human antibody, the chimeric antibody can alleviate the murine antibody-induced immune response. To establish chimeric antibody, hybridoma secreting specific murine monoclonal antibody is established firstly, variable region gene is cloned from murine hybridoma, and then cloned into constant region gene of human antibody for recombinant expression.

The term "humanized antibody", also known as humanized CDR-grafted antibody, refers to an antibody generated by grafting murine CDR sequences onto framework of human antibody variable region, that is to say, the antibodies produced in different types of human germline antibody framework sequences.Humanized antibody avoids the strong immune antibody response due to the chimeric antibody which carries a large number of murine protein components. Such framework sequences can be obtained from public DNA database covering germline antibody gene sequences or published references. For example, germline DNA sequences of human heavy and light chain variable region genes can be found in "VBase" human germline sequence database (available on website www.mrccpe.com.ac.uk/vbase), as well as found in Kabat, EA, et al, 1991 Sequences of Proteins of Immunological Interest, 5th Ed. In a preferred embodiment of the invention, the murine CDRs sequences of c-Met humanized antibody are selected from SEQ ID NO: 6, 7, 8, 9, 10, 11 (please check the #s, in case just copy from sost draft). Human antibody variable region frameworks were designed and selected, wherein the light chain FR region sequence of said antibody light chain variable region is derived from human germline light chain sequences, preferably selected from human germine light chain IGKV085 or IGKV 4-1*01, comprising FR1, FR2, FR3 and FR4 region of human germine light chain IGKV085 and IGKV 4-1*01; the heavy chain FR region sequence of said antibody heavy chain variable region is derived from human germline heavy chain sequences, preferably selected from human germline heavy chain IGHV 3-33*01, comprising FR1, FR2, FR3 and FR4 region of human germline heavy chain IGHV 3-33*01. To avoid decrease of activity caused by decrease of immunogenicity, a minimum of back mutation(s) could be introduced into human antibody variable region to maintain the activity.

There are multiple methods available in the art to generate humanized antibodies. For example, humanized antibodies may be produced by obtaining HCVR and LCVR sequences of anti c-Met antibody (e.g., a murine antibody or antibody produced by a hybridoma), and grafting such sequences onto the selected human framework-encoding sequences. Optionally, a CDR region may be optimized by random mutagenesis or mutagenesis at particular locations in order to substitute one or more amino acids in the CDR with different amino acids prior to grafting the CDR region onto the framework region. Alternatively, a CDR region may be optimized after being inserted into the human framework region by using methods available to one of skilled in the art. Preferably, a "humanized antibody" has CDRs that originate from or are derived from a parent antibody (i.e., a non-human antibody, preferably a mouse monoclonal antibody), while framework and constant region, to the extent it is present, (or significant or substantial portion thereof, i.e., at least about 90%, 92%, 94%, 95%, 96%, 97%, 98% or 99%) are encoded by nucleic acid that occurs in the human germline immunoglobulin region (see, e.g., the International ImMunoGeneTics Database) or in recombined or mutated forms thereof, regardless of whether said antibodies are produced in a human cell. Preferably, at least two, three, four, five or six CDRs of a humanized antibody are optimized from the CDRs of a non-human parent antibody from which the humanized antibody was derived, to generate a desired property, e.g., improved specificity, affinity or neutralization, which may be

identified by a screening assay, e.g., an ELISA assay. Preferably an optimized CDR in the antibody of the invention comprises at least one amino acid substitution when compared with that present in the parent antibody. When compared with CDRs of parent antibodies, certain amino acid substitutions in the CDRs of humanized antibodies of the invention (see example 6 herein) decrease the likelihood of instability of the antibody (e.g., removal of Asn residues from CDR) or decrease the immunogenicity of the antibody when administered to a human subject (e.g., as predicted by IMMUNOFILTERTM Technology).

After the CDR-encoding sequences are grafted onto the selected human framework encoding sequences, the resultant DNA sequences encoding the humanized variable heavy and variable light chain sequences are then expressed to produce a humanized antibody that binds to c-Met. The humanized HCVR and LCVR may be expressed as part of a whole anti-c-Met antibody molecule, i.e., as a fusion protein with human constant domain sequences. However, the HCVR and LCVR sequences can also be expressed in the absence of constant sequences to produce a humanized anti-c-Met scFv.

References further describing methods involved in humanization of a mouse antibody that may be used include e.g., Queen et al., Proc. Natl. Acad. Sci. USA 88: 2869, 1991 and the method of Winter and co-workers [Jones et al., Nature, 321:522 (1986); Riechmann et al., Nature, 332:323-327 (1988); Verhoeyen et al., Science, 239:1534 (1988)].

"Antigen-binding fragment" in the present invention refers to Fab fragment, Fab' fragment, $F(ab')_2$ fragment having antigen-binding activity, as well as Fv fragment scFv fragment binding with human c-Met; it comprises one or more CDR regions of antibodies described in the present invention, selected from the group consisting of SEQ ID NOs:3 to SEQ ID NO:8. Fv fragment is a minimum antibody fragment comprising heavy chain variable region, light chain variable region, and all antigen-binding sites without a constant region. Generally, Fv antibody further comprises a polypeptide linker between the VH and VL domains, and is capable of forming a structure necessary for antigen binding. Also, different linkers can be used to connect the variable regions of two antibodies to form a polypeptide chain, named single chain antibody or single chain Fv (scFv). scFv can also be used with other antibodies such as anti-EGFR antibody to construct bispecific antibody. The term "binding with c-Met" used in this invention, means being capable of interacting with human c-Met. The term "antigen-binding sites" in the present invention, refers to discontinuous, three-dimensional sites on the antigen, recognized by the antibody or the antigen-binding fragment of the present invention. As used herein, the term "ADCC", namely antibody-dependent cell-mediated cytotoxicity, means that the cells expressing Fc receptors directly kill the target cells coated by an antibody by recognizing the Fc segment of the antibody. ADCC effector function of the antibody can be reduced or eliminated by modifying the Fc segment in IgG. The modification refers to mutations performed on the antibody heavy chain constant region, such as mutations selected from N297A, L234A, L235A in IgG1; IgG2/4 chimera; F235E, and L234A/E235A mutations in IgG4.

As used herein, fusion protein described in the present invention is a protein product obtained by co-expressing two genes via recombinant DNA technology. Recombinant c-Met extracellular domain Fc fusion protein is obtained by co-expressing a c-Met extracellular domain and a human antibody Fc fragment via recombinant DNA technology. The c-Met extracellular domain refers to the extracellular moiety of c-Met protein expression.

The engineered antibody or antigen-binding fragment of the present invention may be prepared and purified using conventional methods. For example, cDNA sequences encoding a heavy chain (SEQ ID NO: 4) and a light chain (SEQ ID NO: 5) may be cloned and recombined into pEE6.4 expression vector (Lonza Biologies). The recombinant immunoglobulin expression vector may then be stably transfected into CHO cells. As a more recommended method well known in the art, mammalian expression system will make antibodies glycosylated, typically at the highly conserved N-terminus in the FC region. Stable clones may be obtained through expression of an antibody specifically binding to human c-Met. Positive clones may be expanded in a serum-free culture medium for antibody production in bioreactors. Culture medium, into which an antibody has been secreted, may be purified by conventional techniques. For example, the medium may be conveniently applied to a Protein A or G Sepharose FF column that has been equilibrated with a compatible buffer. The column is washed to remove nonspecific binding components. The bound antibody is eluted by PH gradient and the antibody fragments are detected by SDS-PAGE, and then collected. The antibody may be filtered and concentrated using common techniques. Soluble aggregate and multimers may be effectively removed by common techniques, including size exclusion or ion exchange. The obtained product may be immediately frozen, for example at -70°C, or may be lyophilized.

The term "antibody," in this invention refers to a monoclonal antibody. As used herein, the term "monoclonal antibody" or "mAb" refers to an antibody secreted by a clone derived from a single cell strain. The cell strain is not limited to eukaryotic, prokaryotic, or phage clonal cell lines. Monoclonal antibodies or antigen-binding fragments can be obtained by recombinant method, for example, hybridoma techniques, recombinant techniques, phage display techniques, synthetic techniques (such as CDR-grafting), or other techniques readily known in the art.

"Administration" and "treatment," as it applies to an animal, human, experimental subject, cell, tissue, organ, or biological fluid, refers to contacting an exogenous pharmaceutical, therapeutic, diagnostic agent, or composition with the animal, human, subject, cell, tissue, organ, or biological fluid. "Administration" and "treatment" can refer, e.g., to therapeutic, pharmacokinetic, diagnostic, research, and experimental methods. Treatment of a cell encompasses contacting an agent with the cell, as well as contacting an agent with a fluid, where the fluid is in contact with the cell.

"Treat" means to administer a therapeutic agent, such as a composition comprising any of the binding compounds of the present invention, internally or externally to a patient having one or more disease symptoms for which the agent has known therapeutic activity. Typically, the therapeutic agent is administered in an amount effective to alleviate one or more disease symptoms in the treated patient or population, by inducing the regression of or inhibiting the progression of such symptom(s) to any clinically measurable degree. The amount of a therapeutic agent that is effective to alleviate any particular disease symptom (also referred to "therapeutically effective amount") may vary according to factors such as the disease state, age, and weight of the patient, and the ability of the drug to elicit a desired response in the patient. Whether a disease symptom has been alleviated can be assessed by any clinical measurement typically used by physicians or other skilled healthcare providers to assess the severity or progression status of that symptom. While an embodiment of the present invention (e.g., a treatment method or article of manufacture) may not be effective in alleviating the disease symptom(s) of interest in every patient, it can alleviate the target disease symptom(s) of interest in a statistically significant number of patients as determined by any statistical test known in the art such as the Student's t-test, the chi-square test, the U-test according to Mann and Whitney, the Kruskal-Wallis test (H-test), Jonckheere-Terpstra-test and the Wilcoxon-test.

"Conservative modification" or "conservative replacement or substitution" refers to substitutions of amino acids in a protein with other amino acids having similar characteristics (e.g. charge, side-chain size, hydrophobicity/hydrophilicity, backbone conformation and rigidity, etc.), such that the changes can frequently be made without altering the biological activity of the protein. Those of skilled in this art recognize that, in general, single amino acid substitution in non-essential regions of a polypeptide does not substantially alter biological activity (see, e.g., Watson et al. (1987) Molecular Biology of the Gene, The Benjamin/Cummings Pub. Co., p. 224 (4.th Ed.)). In addition, substitutions of structurally or functionally similar amino acids are less likely to disrupt biological activity.

The term "consisting essentially of' or variation thereof as used throughout the specification and claims, indicates the inclusion of any said elements or group of elements, and optionally inclusion of other elements, of similar or different nature than the recited elements, which do not significantly change the basic or novel properties of the specified dosage regimen, method, or composition. As a non limiting example, a binding compound which consists essentially of a recited amino acid sequence may also include one or more amino acids that do not significantly affect the properties of the binding compound.

"Effective amount" encompasses an amount sufficient to ameliorate or prevent a symptom or sign of a medical condition. Effective amount also refers to an amount sufficient to allow or facilitate diagnosis. An effective amount for particular patient or veterinary subject may vary depending on factors such as the condition being treated, the general health of the patient, the route and dose of administration and the severity of side effects. An effective amount can be the maximal dose or dosing regimen that avoids significant side effects or toxic effects.

"Exogenous" refers to substances that are produced outside an organism, cell, or human body, depending on the context. "Endogenous" refers to substances that are produced within a cell, organism, or human body, depending on the context.

"Homology" refers to sequence similarity between two polynucleotide sequences or between two polypeptides. When a position in both of the two compared sequences is occupied by the same base or amino acid monomer subunit, e.g., if a position in each of two DNA molecules is occupied by adenine, then the molecules are homologous at that position. The percent of homology between two sequences is a function of the number of matching or homologous positions shared by the two sequences divided by the number of positions to be compared and then multiplied by 100. For example, if 6 of 10 positions in two sequences are matched or homologous when the sequences are optimally aligned, then the two sequences are 60% homologous. Generally, the comparison is made when two sequences are aligned to give maximum percent homology.

"Optional" or "optionally" means that the event or situation that follows may but not necessarily occur, and the description includes the instances in which the event or situation does or does not occur. For example, "optionally comprises 1-3 antibody heavy chain variable regions" means that the antibody heavy chain variable region with specific sequence can, but not necessarily, be present.

"Pharmaceutical composition" refers to a mixture comprising one or more compounds according to the present invention or physiologically/pharmaceutically acceptable salt or prodrug thereof with other chemical components, as well as additional components such as physiologically/pharmaceutically acceptable carriers and excipients. The pharmaceutical composition aims at promoting the administration to an organism, facilitating the absorption of the active ingredient and thereby exerting a biological effect.

Preparation of conventional pharmaceutical compositions can be found in Chinese pharmacopoeia.

The term "carrier" is applied for the drug of the present invention, and refers to a system that can change the manner in which a drug enters into the human body, and change the *in vivo* distribution, control the release rate of the drug, and delivery of the drug to the target organ. Drug carrier releasing and targeting systems are capable of reducing drug degradation and loss, decreasing side effects, and improving bioavailability. For example, a macromolecular surfactant used as a carrier can be self-assembled to form aggregates in various forms because of its unique amphiphilic structure, and preferred examples include micelles, emulsions, gels, liquid crystals, vesicles, etc. These aggregates not only have the ability to entrap drug molecules, but also display good permeability to membrane, and can be used as excellent drug carriers.

The term "diluent" is also referred to as filler, and its main purpose is to increase the weight and volume of the tablet. The addition of diluent is not only to ensure a certain volume, but also to reduce the dose deviation of main components and improve the compression moldability of the drug. When pharmaceutical tablets contain an oil component, an absorbent must be added to absorb the oil material, and maintain the "dry" state, which facilitates tablet formation.

The term "pharmaceutically acceptable salt" refers to a salt form of a ligand-cytotoxic drug conjugate of the present invention, the salt is safe and effective, and has the desired biological activity in mammals *in vivo.* The antibody-drug conjugate compound of the present invention comprises at least one amino group, by which the antibody-drug conjugate compound can form a salt with acid, including salt formed with inorganic or organic acids, such as carboxylic acid etc.

The term "solvate" refers to a pharmaceutically acceptable solvate formed by a ligand-drug conjugate of the present invention with one or more solvent molecule(s).

The term "ligand" is a macromolecular compound which is able to recognize and bind to the target cell-associated antigens or receptors. The role of the ligand is to deliver the drug to the target cell population bound to the ligand. The ligand includes, but is not limited to, proteinaceous hormones, lectins, growth factors, antibodies and other molecules capable of binding to cells.

The therapeutic agent is a molecule or atom that is administered separately, simultaneously or successively with a binding moiety, such as an antibody or antibody fragment, or sub-fragment thereof, and is useful for the treatment of the disease. Examples of therapeutic agents include, but are not limited to, antibodies, antibody fragments, conjugates, drugs, cytotoxic agents, apoptotic agents, toxins, nuclease (including DNase and RNase), hormones, immunomodulators, chelating agents, Boron compounds, photosensitizers or dyes, radioisotopes or radionuclides, oligonucleotides, interfering RNAs, peptides, antiangiogenic agents, chemotherapeutic agents, cytokines, chemokines, prodrugs, enzymes, binding proteins or peptides, or combination thereof.

The conjugate is an antibody component or other targeting moiety conjugated to a therapeutic agent as described above. As used herein, the terms "conjugate" and "immunoconjugate" are used interchangeably.

The term "cytotoxic agent" as used herein refers to a substance that inhibits or prevents the function of the cell and/or causes cell death or destruction.

"Toxin" refers to any substance capable of adversely affecting cell growth or proliferation.

"Chemotherapeutic agent" refers to a chemical compound that can be used to treat cancer. The definition also includes anti-hormonal agents that regulate, reduce, block or inhibit the effects of hormones that promote cancer growth, and chemotherapeutic agents are often used for systemic treatment. They per se can be hormones.

Auristatins are completely synthetic drugs with a relatively easily modified chemical structure that facilitates the optimization of physical properties and drug feature. Auristatins derivatives used for antibody conjugation include monomethyl auristatin E (MMAE) and monomethyl auristatin F (MMAF), and MMAE is a synthetic penta-peptide derived from natural tubulin polymerase inhibitor dolastatin-10, synthesized by adding 2-amino-1-phenylpropyl-1-ol at the C-terminus. The inhibitory activities of MMAE against a variety of human tumor cell lines are less than one nanomolar. In order to reduce the cytotoxic activity of MMAE itself, a phenylalanine is introduced at the C-terminus of dolastatin-10 in the case of MMAF. Due to the introduction of a carboxyl group in the structure, MMAF has poor permeability to the membrane, and therefore the biological activity against cells is significantly decreased, but the inhibitory activity against cells is increased substantially after it is conjugated to an antibody (US7750116).

The term "tubulin inhibitor" refers to a class of compounds that exert an anti-tumor effect by inhibiting or promoting polymerization of tublin, and consequently interfering with the cell mitosis process. Non-limiting examples include maytansines, calicheamicins, taxanes, vincristines, colchicines, and Dolastatins/Auristatins, preferably maytansines or Dolastatins/Auristatins; more preferably compounds of formula $D_1$ or $D_M$.

CPT is short for camptothecin, and in this application CPT is used to refer to camptothecin itself or analogs or derivatives of camptothecin. The structure of the camptothecin having the indicated number and the ring labeled with the letter A-E and some analogs thereof is provided in the following formula.

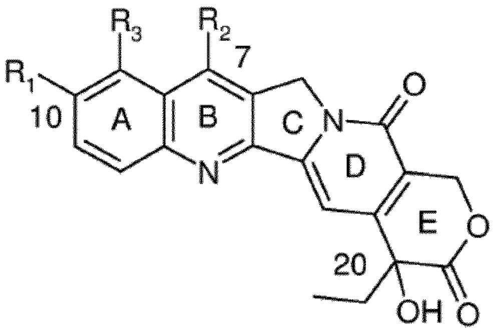

CPT: R1= R2= R3=H
10-hydroxy-CPT:R1=OH; R2= R3=H
Irinotecan: R1=

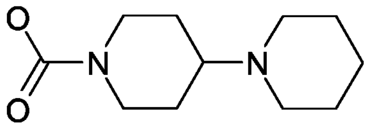

;

R2=ethyl; R3=H
SN-38: R1=OH; R2= ethyl; R3=H
Topotecan: R1=OH; R2=H; R3=CH-N$(CH_3)_2$

The term "intracellular metabolite" refers to a compound produced by intracellular metabolic processes or reactions of antibody-drug conjugates (ADCs). The metabolic process or reaction may be an enzymatic process, such as proteolytic cleavage of a peptide linker of an ADC, or hydrolysis of a functional group such as a hydrazone, ester or amide. Intracellular metabolites include, but are not limited to, antibodies and free drugs that undergo intracellular cleavage after entering, diffusing, ingesting or transporting into cells.

The term "of intracellular cleavage" and "intracellular cleavage" refer to intracellular metabolic processes or reactions of antibody-drug conjugates (ADCs), wherein the covalent attachment between drug moiety (D) and antibody (Ab) is cleaved (i.e. the linker is cleaved), resulting in intracellular dissociation of free drug from the antibody. The module cleaved from ADC is thus an intracellular metabolite.

The term "bioavailability" refers to the systemic availability (i.e., blood/plasma level) of a given amount of drug administered to a patient. Bioavailability is an absolute term that indicates the time (rate) and the total amount (degree) required by the drug to achieve systemic circulation from the administered dose.

The term "cytotoxic activity" refers to cell killing, cytostatic, or growth inhibitory effects of intracellular metabolites of antibody-drug conjugates. Cytotoxic activity can be expressed as the IC50 value, that is, the concentration (molar or mass) per unit volume when half of cells survive.

The "$C_{1-6}$ alkyl" described in the present invention refers to a linear or branched alkyl group having 1 to 6 carbon atoms, and includes for example "$C_{1-4}$ alkyl", "$C_{1-3}$ alkyl" etc., specific examples include but not limited to methyl, ethyl, n-propyl, isopropyl, n-butyl, isobutyl, sec-butyl, tert-butyl, n-pentyl, isopentyl, 2-methylbutyl, neopentyl, 1-ethylpropyl, n-hexyl, isohexyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1,2-dimethylpropyl and the like.

The "$C_{2-6}$ alkenyl" described in the present invention refers to a linear, branched or cyclic alkenyl group having at least one double bond and having 2 to 6 carbon atoms, and includes for example "$C_{2-4}$ alkenyl group" and the like. Examples include, but are not limited to, vinyl, 1-propenyl, 2-propenyl, 1-butenyl, 2-butenyl, 1,3-butadienyl, 1-pentenyl, 2-pentenyl, 3-pentenyl, 1,3-pentadienyl, 1,4-pentadienyl, 1-hexenyl, 2-hexenyl, 3-hexenyl, 1,4-hexadienyl, cyclopentenyl, 1,3-cyclopentadienyl, cyclohexenyl, 1,4-cyclohexadienyl and the like.

The "3-8 membered cycloalkyl" described in the present invention refers to a saturated cyclic alkyl group having 3 to 8 carbon atoms, and includes, for example, "3-6 membered cycloalkyl" and "5-6 membered cycloalkyl" etc. Specific examples include, but are not limited to, cyclopropyl, cyclobutyl, cyclopentyl, cyclohexyl, cycloheptyl, cyclooctyl, and the like. The "5-6 membered cycloalkyl" refers to a saturated cyclic alkyl group having 5 to 6 carbon atoms.

The "$C_{1-6}$ alkoxy" described in the present invention refers to a group which is linked in a form of $C_{1-6}$ alkyl-O-, wherein "$C_{1-6}$ alkyl" is as defined above.

The term "bond" refers to a covalent bond presented as "-".

The term "Hydroxy" refers to an -OH group.

The term "Halogen" refers to fluoro, chloro, bromo or iodo atoms, etc.

The term "Amino" refers to an $-NH_2$ group.

The term "Cyano" refers to a -CN group.

The term "Nitro" refers to a $-NO_2$ group.

The term "Oxo group" refers to a =O group.

The "3-8 membered heterocyclic group" described in the present invention refers to a cyclic group having 3 to 8 ring atoms (at least one of which is a hetero atom such as nitrogen atom, oxygen atom or sulfur atom). Optionally, a ring atom (e.g., carbon atom, nitrogen atom, or sulfur atom) in the cyclic structure can be oxidized. "5-6 membered heterocyclic group" is preferred. Specific examples include, but are not limited to, azacyclopropyl, *2H*-azacyclopropyl, diazacyclopropyl, *3H*-diazacyclopropenyl, azacyclobutyl, 1,4-dioxoheterocyclohexyl, 1,3-dioxoheterocyclohexyl, 1,3-dioxoheterocyclopentyl, 1,4-dioxoheterocyclodiallyl, tetrahydrofuranyl, dihydropyrrolyl, pyrrolidinyl, pyrrolidine-2,5-dione, imidazolidinyl, 4,5-dihydroimidazolyl, pyrazolidinyl, 4,5-dihydropyrazolyl, 2,5-dihydrothiophenyl, tetrahydrothiophenyl, 4,5-dihydrothiazolyl, thiazolidinyl, piperidinyl, tetrahydropyridyl, piperidinone, tetrahydropyridinone, dihydropyridinone, piperazinyl, morpholinyl, 4,5-dihydrooxazolyl, 4,5-dihydroisoxazolyl, 2,3-dihydroisoxazolyl, oxazolidinyl, *2H*-1,2-oxazinyl, *6H*-1,3-oxazinyl, *4H*-1,3-thiazinyl, *6H*-1,3-thiazinyl, *2H*-pyranyl, *2H*-pyranyl-2-one, 3,4-dihydro-*2H*-pyranyl and the like. The "5-6 membered heterocyclic group" refers to a particular example of 3-8 membered heterocyclic group which comprises 5 to 6 ring atoms.

The "6-8 membered aryl" described in the present invention refers to a monocyclic aryl group having 6 to 8 ring carbon atoms, and examples include, but are not limited to, phenyl, cyclooctatetraenyl, and the like.

The "6-15 membered fused aryl" described in the present invention refers to an unsaturated aromatic cyclic group having 6 to 15 ring carbon atoms, which is formed by two or more cyclic structures sharing two adjacent atoms with each other. Specific examples include, but are not limited to, naphthyl, anthryl, phenanthryl and the like. The "6-10 membered fused aryl" refers to a specific example of 6-14 membered fused aryl, which has 6 to 10 ring atoms.

The "5-8 membered heteroaryl" described in the present invention refers to an aromatic cyclic group having 5 to 8 ring atoms (in which at least one ring atom is hetero atom, such as nitrogen atom, oxygen atom or sulfur atom). Optionally, the ring atom in the cyclic structure (e.g., carbon atom, nitrogen atom or sulfur atom) can be oxidized. "5-6 membered heteroaryl" is preferred. Specific examples include, but are not limited to, furyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl , 1,3,4-oxadiazolyl, pyridyl, 2-pyridinone, 4-pyridinone, pyrimidinyl, pyridazinyl, pyrazinyl, 1,2,3-triazinyl, 1,3,5-triazinyl, 1,2,4,5-tetrazinyl, azacycloheptatrienyl, 1,3-diazacycloheptatrienyl, azacyclooctatetraenyl and the like. "5-6 membered heteroaryl" refers to a specific example of 5-8 membered heteroaryl, which has 5 to 6 ring atoms.

The "carbon atom, nitrogen atom or sulfur atom is oxidized" described in the present invention refers to a structure in which C=O, N=O, S=O or $SO_2$ is formed.

The term "optional" or "optionally" means that the event or circumstance described subsequently may, but not necessarily, occur, and the description includes the instances in which the event or circumstance does or does not occur. For example, "the heterocyclic group optionally substituted with an alkyl" means that an alkyl group can be, but not necessarily, present, and the description includes a case wherein the heterocyclic group is substituted with an alkyl and a case wherein the heterocyclic group is not substituted with an alkyl.

"Substituted" refers to one or more hydrogen atoms in the group, preferably up to 5, more preferably 1 to 3 hydrogen atoms, each independently substituted with corresponding number of substituents. It is clear that the substituents only occur in their possible chemical position. The person skilled in the art is able to determine if the substitution is possible or impossible without paying excessive efforts by experiment or theory. For example, the conjugation between amino or hydroxy group having free hydrogen and carbon atoms having unsaturated bonds (such as alkene) may be unstable.

"Linker or linker unit" refers to a chemical module comprising a covalent or atomic chain that covalently attaches the antibody to the drug module. In various embodiments, the linker includes: divalent radicals such as alkyldiyl, arylene, heteroarylene, such as unit like $-(CR_2)_nO(CR_2)_n-$, hydrocarbyloxy repeat units (e.g., polyethyleneamino, PEG, polymethyleneoxy) and aminoalkyl (e.g., polyvinylamino, Jeffamine™), and the like; and diesters and amides including succinate, succinamide, bis-glycolate, malonate and caproamide.

**Abbreviation**

Linker unit

MC=6- maleimido-caproyl
Val-Cit or "vc"= valine-citrulline (an exemplary dipeptide of a protease cleavable linker)
Citrulline=2-Amino-5-ureido pentanoic acid
PAB= p-aminobenzyloxycarbonyl (examples of "self-immolative " linker unit)
Me-Val-Cit=N-methyl-valine-citrulline (wherein the linker peptide bond has been modified to prevent from being cleaved by cathepsin B)
$MC(PEG)_6$-OH= maleimido-caproyl-polyethylene glycol (which can be attached to antibody cysteine)
SPP=N-Succinimidyl 4-(2-pyridylthio) valerate
SPDP=N-Succinimidyl 3-(2-pyridyldithio) propionate
SMCC= Succinimidyl-4-(N-maleimidomethyl) cyclohexane-1-carboxylate
IT= imino sulfane

Cytotoxic drugs:

MMAE= Monomethyl aurantatin E (MW 718)
MMAF=variant of aurantatin E (MMAE), which has phenylalanine at the C-terminus of the drug (MW731.5)
MMAF-DMAEA= DMAEA (dimethylaminoethylamine) linked to the phenylalanine at C-terminus of MMAF (MW 801.5) via amide
MMAF-TEG= tetraethylene glycol is esterified to phenylalanine of MMAF
MMAF-NtBu= N-tert-butyl as an amide attached to the C-terminus of the MMAF
DM1=N(2')-deacetyl-N (2')-(3-mercapto-1-oxopropyl)-maytansine
DM3=N(2')-deacetyl-N2-(4-mercapto-1-oxopentyl)-maytansine
DM4=N(2')-deacetyl-N2-(4-mercapto-4-methyl-1-oxopentyl)-maytansine

The present invention also provides an antibody-cytotoxic drug conjugate comprising any anti-c-Met antibody of the invention or other c-Met antibody showing endocytosis activity (eg, LY-2875358) conjugated to one or more cytotoxic agents, or pharmaceutically acceptable salt or solvate thereof (interchangeable as "antibody-drug conjugate" or "ADC"), wherein the cytotoxic agents include, for example, chemotherapeutic agents, drugs, growth inhibitors, toxins (e.g., bacterial, fungal, plant or animal-derived enzyme-active toxins or fragments thereof) or radioisotopes (i.e., radioconjugates).
In certain embodiments, the antibody-cytotoxic drug conjugate or pharmaceutically acceptable salt or solvate thereof comprises an anti-c-Met antibody and a chemotherapeutic agent or other toxin. The chemotherapeutic agents that can be used to produce an antibody-cytotoxic drug conjugate or pharmaceutically acceptable salt or solvate thereof have been described herein (described above). Enzyme-active toxins and fragments thereof are also used, which are described in the specification.
In certain embodiments, the antibody-cytotoxic drug conjugate or pharmaceutically acceptable salt or solvate thereof comprises an anti-c-Met antibody and one or more small molecule toxins including, but not limited to small molecule drugs such as camptothecin derivatives, calicheamicin, maytansinoids, dolastatin, oricotine, trichothecene and CC1065, and cytotoxic fragments of these drugs.
Exemplary linker $L_2$ includes 6-maleimidocaproyl ("MC"), maleimidopropionyl ("MP"), valine-citrulline ("val-cit" or "vc"), alanine-phenylalanine (ala-phe), p-aminobenzyloxycarbonyl ("PAB"), N-succinimidyl 4- (2-pyridylthio) pentanoate "SPP"), N-succinimidyl 4- (N-maleimidomethyl) cyclohexane-1 carboxylate ("SMCC"), and N-succinimidyl (4-iodo-acetyl) aminobenzoate ("SIAB"). A variety of linkers are known in the art and are described below.
The linker may be a "cleavable linker" that facilitates the release of the drug in the cell. For example, an acid-labile linker (e.g., hydrazone), a protease-sensitive (e.g., peptidase-sensitive) linker, a light-labile linker, a dimethyl linker, or disulfide-containing linker may be used (Chari et al, Cancer Research 52: 127-131(1992); US patent No.5,208,020).
In some embodiments, the linker element may be a "stretcher unit" that connects the antibody to another linker element or drug module. Exemplary stretcher units are shown below (where the wave line indicates the site to which the antibody covalently attached):

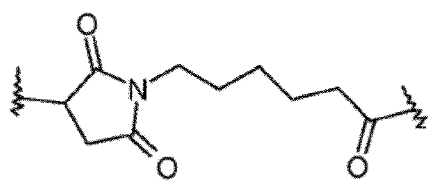
MC

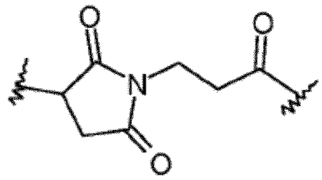

MP

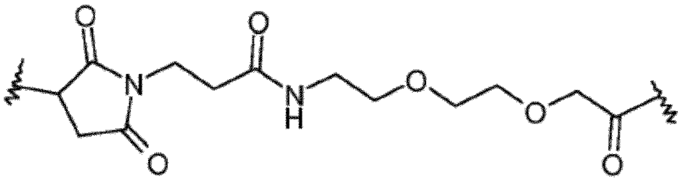

MPEG

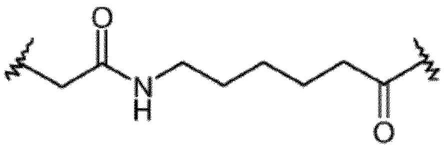

In some embodiments, the linker unit may be an amino acid unit. In one such embodiment, the amino acid unit allows the linker to be cleaved by the protease, thereby facilitating to release the drug from the antibody-cytotoxic drug conjugate or its pharmaceutically acceptable salt or solvate after exposure to intracellular proteases, such as lysosomal enzymes. See for example Doronina et al (2003) Nat. Biotechnol. 21: 778-784. Exemplary amino acid units include, but are not limited to, dipeptides, tripeptides, tetrapeptides, and pentapeptides. Exemplary dipeptides include: valine-citrulline (VC or val-cit); alanine-phenylalanine (AF or ala-phe); phenylalanine-lysine (FK or phe-lys); or N-Methyl-valine-citrulline (Me-val-cit). Exemplary tripeptides include glycine-valine-citrulline (gly-val-cit) and glycine-glycine-glycine (gly-gly-gly). The amino acid units may comprise naturally occurring amino acid residues, as well as minor amino acids and non-naturally occurring amino acid analogs, such as citrulline. Amino acid units can be designed and optimized for their selectivity to enzymatic cleavage by specific enzymes, such as tumor-associated proteases, cathepsin B, C and D, or plasma proteases.

In some embodiments, the linker unit may be a "spacer" unit that connects the antibody (either directly or through the stretcher unit and/or the amino acid unit) to the drug module. The spacer unit may be "self-immolative" or "non-self immolative". The "non-self immolative" spacer unit refers to a spacer unit of which portion or the whole remains binding to the drug module after enzymatic cleavage (protein hydrolysis) of the ADC. Examples of non-self immolative spacer units include, but are not limited to, glycine spacer units and glycine-glycine spacer units. Other combinations of peptide spacers susceptible to sequence-specific enzymatic cleavage are also contemplated. For example, enzymatic cleavage of glycine-glycine spacer unit-containing ADC by tumor-cell-associated protease will result in the release of the glycine-glycine-drug module from the remainder of the ADC. In one such embodiment, the glycine-glycine-drug module is then subjected to a separate hydrolysis step in the tumor cells, thereby the glycine-glycine spacer unit is cleaved from the drug module.

The "self-immolative" spacer unit allows the release of the drug module without separate hydrolysis steps. In certain embodiments, the spacer unit of the linker comprises a p-aminobenzyl unit. In one such embodiment, p-aminobenzyl alcohol is attached to the amino acid unit via an amide bond, thereby forming a carbamate, methyl carbamate, or carbonate between benzyl alcohol and the cytotoxic agent. See, for example, in Hamann et al, (2005) Expert Opin. Ther. Patents (2005) 15: 1087-1103. In one embodiment, the spacer unit is p-aminobenzyloxycarbonyl (PAB).

Exemplary linkers in the present invention are as follows:

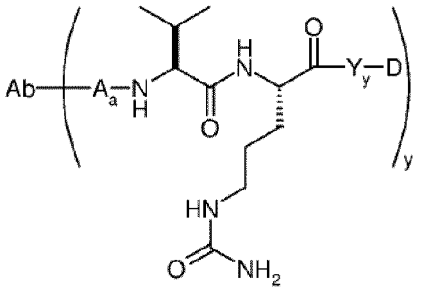

Val-Cit or VC

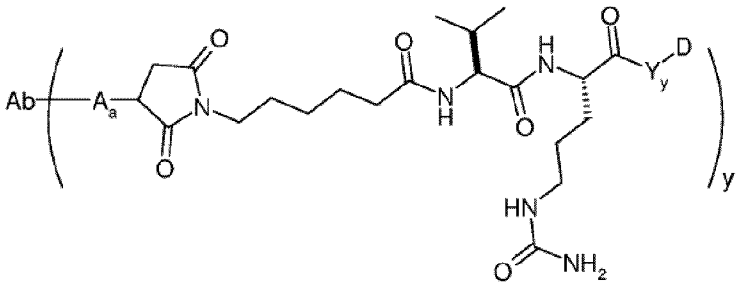

MC-Val-Cit

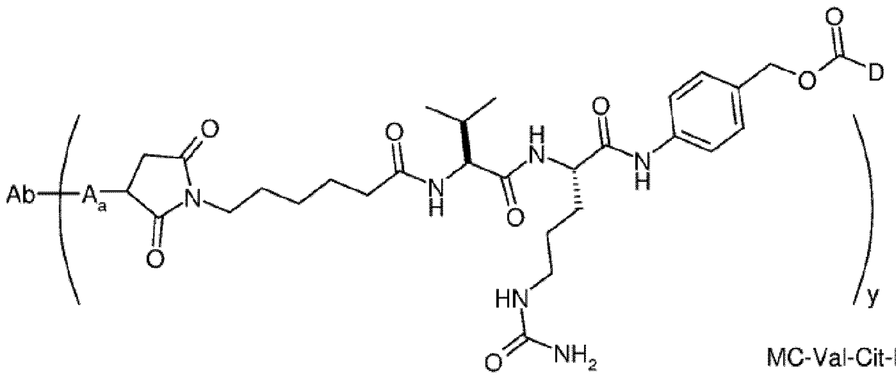

MC-Val-Cit-PAB

The linker, including stretcher, spacer, and amino acid unit, can be synthesized by methods known in the art, such as those described in US2005-0238649A1.

**Exemplary drug modules:**

Maytansine and maytansinoids.

In some embodiments, the antibody-cytotoxic drug conjugate or pharmaceutically acceptable salt or solvate thereof comprises an antibody of the invention conjugated to one or more maytansinoid molecules. The maytansinoid is a mitotic inhibitor that acts by inhibiting tubulin multimerization. Maytansine was originally isolated from the Maytansine tree (Maytenus serrata) from the East African shrubs (US patent No.3,896,111). It was subsequently found that certain microorganisms also generate maytansinoids such as maytansinol and C-3 maytansinol ester (US patent No.4,151,042).

The maytansinoid drug modules are attractive drug modules in antibody-drug conjugates because they: (i) are relatively easy to be prepared from fermentation ,or prepared from modification or derivation from the fermentation products; (ii) readily derived with a functional group suitable for coupling to an antibody through a non-disulfide linker; (iii) stable in plasma; and (iv) effective for variety of tumor cell lines.

Maytansine compounds suitable for use as the maytansinoid drug modules are well known in the art and can be isolated from natural sources according to known methods or produced using genetic engineering technique (See Yu et al (2002) PNAS 99: 7968-7973). The maytansinol and maytansinol analogs can also be prepared according to known methods.

Exemplary embodiments of the maytansinoid drug module include: DM1, DM3 and DM4, as disclosed herein.

In some embodiments, the antibody-cytotoxic drug conjugate or pharmaceutically acceptable salt or solvate

thereof comprises an antibody of the invention conjugated to dolastatin or dolastatin peptide analog or derivative (e.g., auristatin) (U.S. Patent No. 5,635,483; 5,780,588). Dolastatin and auristatin have been shown to interfere with microtubule kinetics, GTP hydrolysis, and nuclear and cell division (Woyke et al. (2001) Antimicrob. Agents and Chemother. 45 (12): 3580-3584), and anti-cancer activity (U.S. Patent No. 5,663,149) and antifungal activity (Pettit et al. (1998) Antimicrob. Agents Chemother. 42: 2961-2965). Dolastatin or auristatin drug modules may be attached to the antibody via the N (amino) terminus or the C (carboxy) terminus of the peptide drug module (WO02/088172).

Exemplary embodiment of auristatin include N-terminus linked monomethyl auristatin drug modules DE and DF, which are disclosed by Senter et al, Proceedings of the American Association for CancerResearch, volume 45, abstract number 623, March 28, 2004, the disclosure of which is expressly incorporated herein by reference in its entirety. The peptide drug module may be selected from the general formulas $D_E$ and $D_F$ as below:

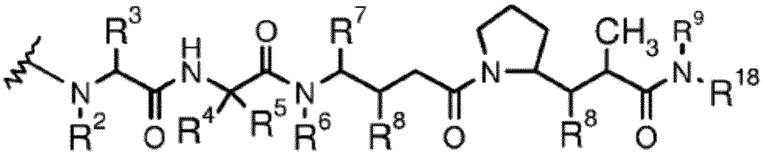

$D_E$

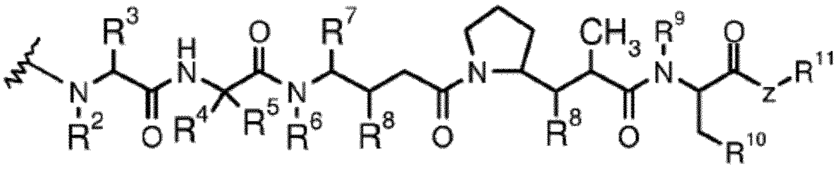

$D_F$

wherein the wavy lines of the $D_E$ and $D_F$ indicate the covalent attachment sites of the antibody or antibody-linker, and each site is independent from each other:

$R^2$ is selected from H and C1-C8 hydrocarbyl;
$R^3$ is selected from the group consisting of H, C1-C8 hydrocarbyl, C3-C8 carbocycle, aryl, C1-C8 hydrocarbyl-aryl, C1-C8 hydrocarbyl-(C3-C8 carbocycle), C3-C8 heterocycle and C1-C8 hydrocarbyl -(C3-C8 heterocycle);
$R^4$ is selected from the group consisting of H, C1-C8 hydrocarbyl, C3-C8 carbocycle, aryl, C1-C8 hydrocarbyl-aryl, C1-C8 hydrocarbyl-(C3-C8 carbocycle), C3-C8 heterocycle and C1-C8 hydrocarbyl-(C3-C8 heterocycle);
$R^5$ is selected from H or methyl;
or $R^4$ and $R^5$ form a carbocycle of formula -(CRaRb)n-, wherein $R^a$ and $R^b$ are each independently selected from the group consisting of H, C1-C8 hydrocarbyl and C3-C8 carbocycle, and n is selected from 2, 3, 4, 5 and 6;
$R^6$ is selected from H or C1-C8 hydrocarbyl;
$R^7$ is selected from the group consisting of H, C1-C8 hydrocarbyl, C3-C8 carbocycle, aryl, C1-C8 hydrocarbyl-aryl, C1-C8 hydrocarbyl-(C3-C8 carbocycle), C3-C8 heterocycle and C1-C8 hydrocarbyl-(C3-C8 heterocycle);
Each $R^8$ is independently selected from the group consisting of H, OH, C1-C8 hydrocarbyl, C3-C8 carbocycle and O-(C1-C8 hydrocarbyl);
$R^9$ is selected from H or C1-C8 hydrocarbyl;
$R^{10}$ is selected from aryl or C3-C8 heterocycle;
Z is selected from O, S, NH or $NR^{12}$, wherein $R^{12}$ is C1-C8 hydrocarbyl;
$R^{11}$ is selected from the group consisting of H, C1-C20 hydrocarbyl, aryl, C3-C8 heterocycle, $-(R^{13}O)m-R^{14}$ and $-(R^{13}O)m-CH(R^{15})_2$;
m is an integer selected from 1-1000;
$R^{13}$ is C2-C8 hydrocarbyl;
$R^{14}$ is H or C1-C8 hydrocarbyl;
$R^{15}$ is independently selected from the group consisting of H, COOH, $-(CH_2)n-N(R16)_2$, $-(CH_2)n-SO_3H$ and-$(CH_2)n-SO_3$-C1-C8 hydrocarbyl;
$R^{16}$ is independently selected from the group consisting of H, C1-C8 hydrocarbyl and $-(CH_2)n-COOH$;
$R^{18}$ is selected from the group consisting of $-C(R8)_2-C(R8)_2$-aryl, $-C(R8)_2-C(R8)_2$-(C3-C8 heterocycle) and $-C(R8)_2-C(R8)_2$-(C3-C8 carbocycle); and
n is an integer selected from 0 to 6.

MMAE is an exemplary auristatin of the formula $D_E$, wherein the wavy line indicates a linker (L) covalently attached to the antibody-drug conjugate:

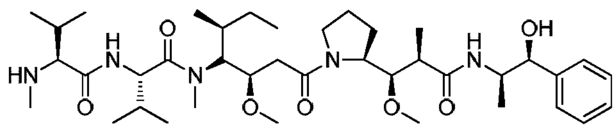

MMAE

MMAF is an exemplary auristatin of the formula DF, wherein the wavy line indicates a linker (L) covalently attached to the antibody-drug conjugate (see US2005/0238649 and Doronina et al (2006) Bioconjugate Chem. 17: 114-124):

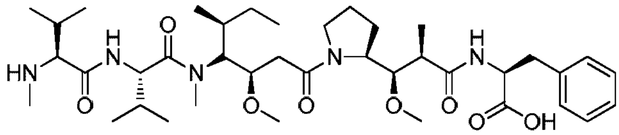

MMAF

The other drug modules comprise MMAF derivative selected from the following, wherein the wavy line indicates a linker (L) covalently attached to the antibody-drug conjugate:

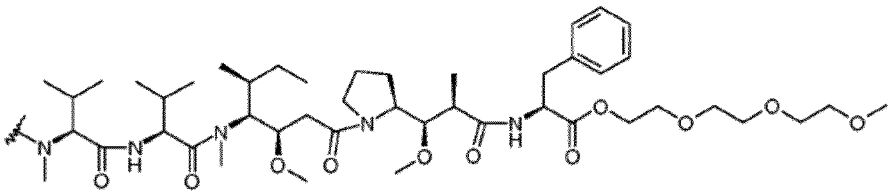

,

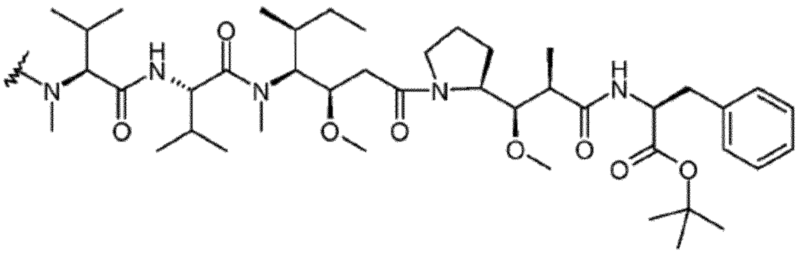

,

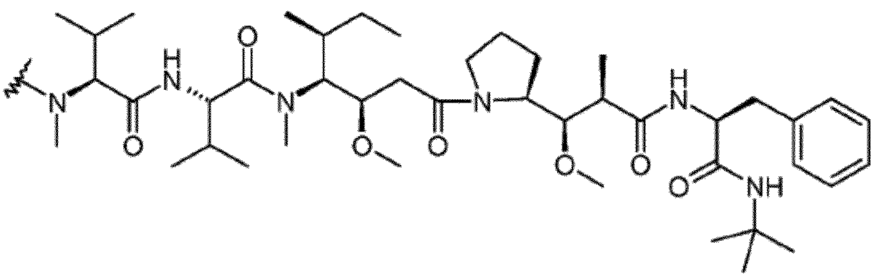

,

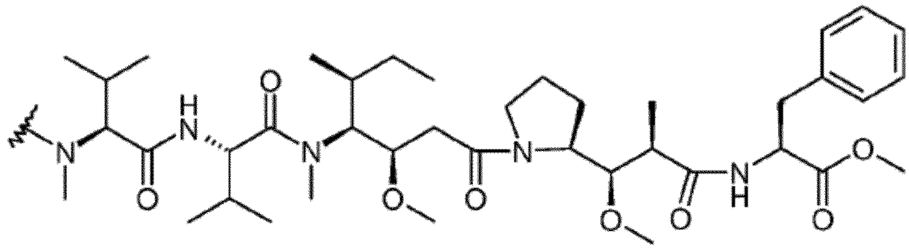

,

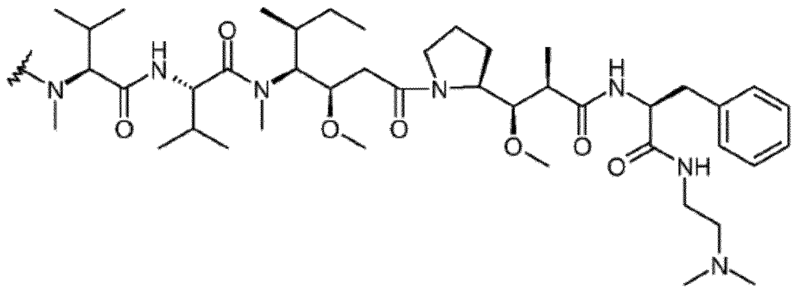

,

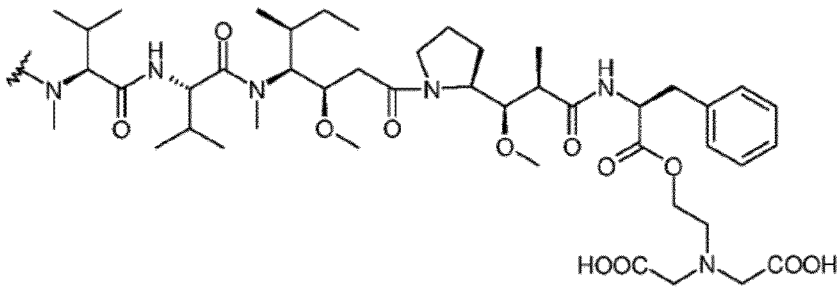

,

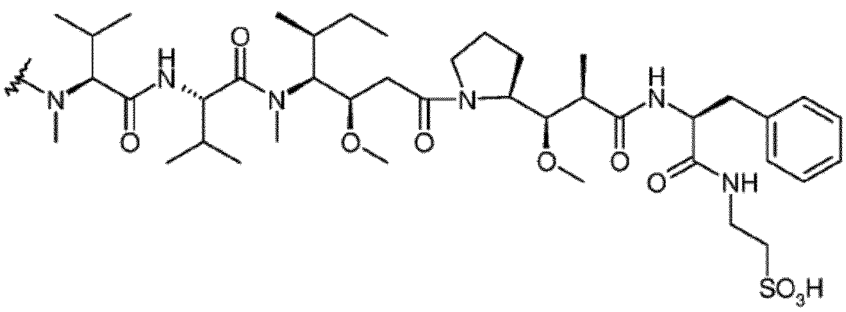

,

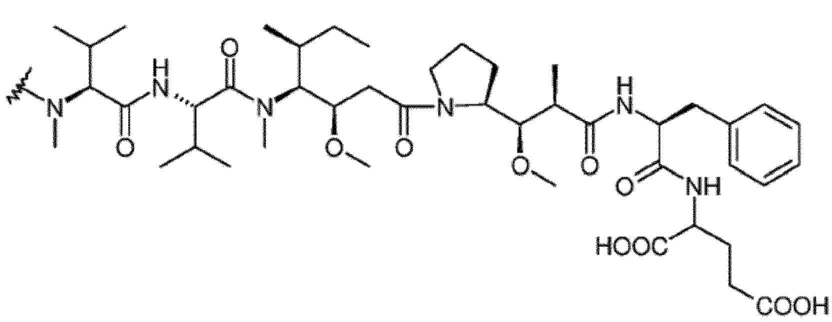

,

and

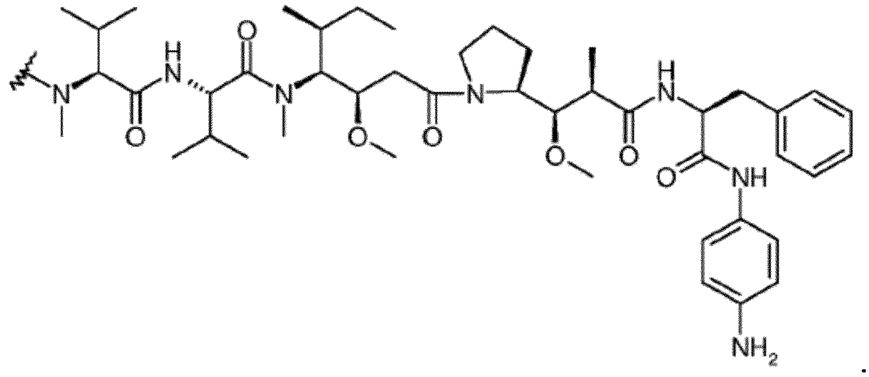

In one aspect, a hydrophilic group may be attached to a drug module at $R^{11}$, wherein said hydrophilic group includes, but not limited to, triethylene glycol ester (TEG), as described above. Without being limited to any particular theory, the hydrophilic groups contribute to the internalization and non-agglomeration of the drug modules. Exemplary embodiments of ADC of general formula I comprising auristatin/dolastatin or derivative thereof are described in US2005-0238649A1 and Doronina et al (2006) Bioconjugate Chem. 17:114-124, which is expressly incorporated herein by reference. Exemplary embodiments of ADCs of general formula I comprising MMAE or MMAF and various linkers have the following structure and abbreviations (wherein "Ab" is antibody; p ranges from 1 to about 8; "Val-Cit" is Valine-citrulline dipeptide; and "S" is sulfur atom):

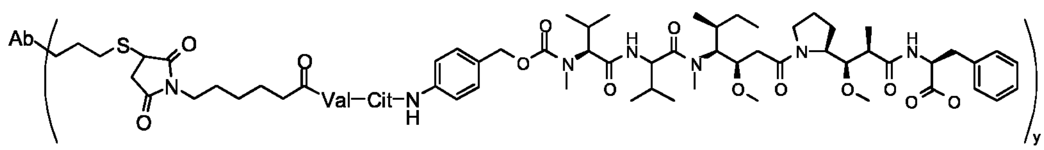

Ab- linker 1-MC-vc-PAB-MMAF

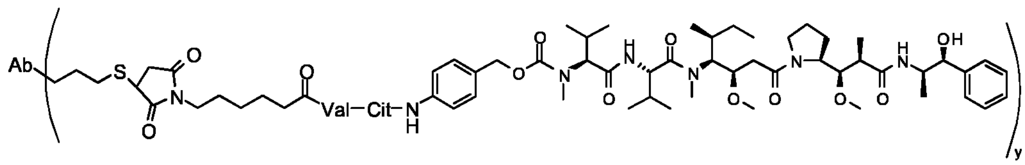

Ab- linker 1-MC-vc-PAB-MMAE

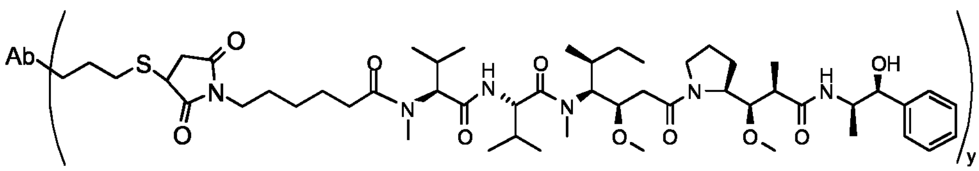

Ab- linker 1-MC- MMAE

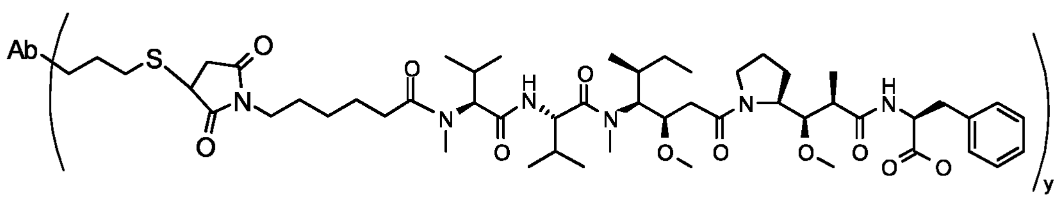

Ab-linkerl-MC- MMAF

Typically, peptide-based drug modules can be prepared by forming peptide bonds between two or more amino acids and/or peptide fragments. Such peptide bonds can be prepared according to, for example, liquid phase synthesis methods well known in the art of peptide chemistry (see E. Schroder and K.Lübke, The Peptides, volume 1, pp 76-136, 1965, Academic Press). The auristatin/dolastatin drug modules can be prepared according to the methods described in the following literatures: US20050238649A1; US patent No.5635483; US patent No.5780588; Pettit et al(1989) J. Am. Chem. Soc. 111: 5463-5465; Pettit et al (1998) Anti-Cancer Drug Design 13: 243-277; Pettit, G. R. et al, Synthesis, 1996, 719-725; Pettit et al (1996) J. Chem. Soc. Perkin Trans. 15: 859-863; and Doronina(2003) Nat. Biotechnol. 21(7): 778-784.

In particular, the auristatin/dolastatin drug modules of the general formula DF, such as MMAF and derivatives thereof, can be prepared using the methods described in US20050238649A1 and Doronina et al. (2006) Bioconjugate Chem. 17: 114-124. The auristatin/dolastatin drug modules of the general formula DE, such as MMAE and derivatives thereof, can be prepared by the method described in Doronina et al. (2003) Nat. Biotech. 21: 778-784. The drug-linker modules of MC-MMAF, MC-MMAE, MC-vc-PAB-MMAF and MC-vc-PAB-MMAE can be conveniently synthesized by conventional methods such as those described in Doronina et al. (2003) Nat. Biotech. 21: 778-784 and U.S. Patent Application Publication No. US2005/0238649A1, and then conjugated to the antibody of interest.

The Immunohistochemistry IHC of the present invention is used for detecting overexpression of c-Met protein in c-Met-positive tumor cells, and the expression amount is reflected based on the staining of cancer cells, gray density analysis was performed with reference to percep test scoring or by using software.

The expression of c-Met is divided into two types: high expression and low expression, 3+ (≥50% of tumor cells are strongly positive), 2+ (≥50% of tumor cells are positive/weak positive, and <50% of tumor cells are strongly positive), 1+ (≥50% of tumor cells are weakly positive, and <50% of cells are positive), 0 (no staining or tumor cells with any intensity <50%), 2+ or 3+ is defined as high expression.

The c-Met positive marker scoring criteria are as follows:

(1) According to the degree of cell positive staining (antigen content), it is divided into: weak positive (+), 1 score; medium positive (++), 2 score; strongly positive (+++), 3 score;

(2) According to the number of positive cells, it is divided into: weakly positive (+, refers to the total number of positive cells below 25%), medium positive (++, refers to the total number of positive cells between 25%-49%), strongly positive (+++, refers to the total number of positive cells above 50%);

At present, weighted integral measurement is used. The calculation formula is: (÷)%×1+(++)%×2+(+++)%×3; a score of 0-0.3 is weakly positive, 0.3-1.5 is medium positive, and 1.5-3 is strongly positive.

The c-Met positive or overexpression of c-Met described in the present invention means that ≥20% of the hepatic carcinoma cells are positive/weakly positive, preferably ≥25% of the hepatic carcinoma cells are positive, more preferably ≥50% of the hepatic carcinoma cells are strongly positive.

Drug loading

The drug loading is expressed as y, that is the mean number of drug modules per antibody in the molecule of formula I. The drug loading can range from 1 to 20 drug modules (D) per antibody. The ADC of Formula I includes a collection of antibodies conjugated to a range of (1-20) drug modules. The mean number of drug modules per antibody in ADC preparation obtained from coupling reaction can be characterized by conventional means such as mass spectrometry, ELISA assay, and HPLC. It is also possible to determine the quantitative distribution of ADCs in the aspect of y. In some cases, homogeneous ADCs with certain p values are isolated from ADCs of other drug loads, and then purified and characterized, this can be achieved by means such as reverse phase HPLC or electrophoresis.

For some antibody-drug conjugates, y may be limited by the number of attachment sites on the antibody. For example, if the cysteine thiol is attached, as in the above illustrative embodiment, the antibody may have only one or several cysteine thiol groups, or may only have one or more reactive thiol group which can be attached to the linker. In certain embodiments, a higher drug loading, such as y >5, may cause aggregation, insolubility, toxicity, or loss of cell permeability of certain antibody-drug conjugates. In certain embodiments, the drug loading of the ADC of the invention ranges from 1 to about 8; from about 2 to about 6; from about 3 to about 5; from about 3 to about 4; from about 3.1 to about 3.9; from about 3.2 to about 3.8; about 3.2 to about 3.7; about 3.2 to about 3.6; about 3.3 to about 3.8; or about 3.3 to about 3.7. In fact, for some ADCs, it has been shown that the optimal ratio of each antibody drug module may be less than 8 and may be from about 2 to about 5. See US20050238649A1 (incorporated herein by reference in its entirety).

In certain embodiments, a drug module having less than the theoretical maximum is coupled to the antibody in the coupling reaction. The antibody may comprise, for example, a lysine residue that does not react with a drug-linker intermediate or linker agent, as discussed below. Only the most reactive lysine groups can react with amine-reactive linker agents. In general, the antibody does not contain a number of free and reactive cysteine thiol groups, which can

be linked to a drug module; in fact, most of the cysteine thiol groups in the antibody are present in the form of a disulfide bridge. In certain embodiments, the antibody may be reduced with a reducing agent such as dithiothreitol (DTT) or tricarbonyl ethyl phosphine (TCEP) under partially or completely reductive conditions to produce a reactive cysteine thiol group. In certain embodiments, the antibody is placed under denaturing conditions to expose a reactive nucleophilic group, such as lysine or cysteine.

The drug load (drug/antibody ratio, DAR) of the ADC can be controlled in different ways, for example by: (i) limiting the molar excess of the drug-linker intermediate or linker agent; (ii) limiting the time or temperature of the coupling reaction; (iii) limiting the thiol modification of the cysteine or restricting the reductive condition; (iv) engineering the amino acid sequence of the antibody by recombinant techniques, such that the number and location of cysteine residues are altered in order to control the number and/or position of the linker-drug attachment (such as the thioMab or the thioFab prepared as those described in the present invention and WO2006/034488 (incorporated herein by reference in its entirety)).

It is to be understood that if more than one nucleophilic group is reacted with a drug-linker intermediate or with a linker and subsequent drug module agents, the resulting product is an ADC compound mixture having one or more drug modules attached to the antibody. The mean number of drugs per antibody can be calculated from the mixture by ELISA assay which involves antibody-specific and drug-specific antibodies. The various ADC molecules in the mixture can be identified by mass spectrometry, and separated by HPLC, for example, hydrophobic interaction chromatography. In certain embodiments, a homogeneous ADC with a single load value can be isolated from the coupling mixture by electrophoresis or chromatography.

Methods for preparing antibody-cytotoxic drug conjugates or pharmaceutically acceptable salts or solvates thereof

The ADC of general formula I can be prepared by several routes using organic chemical reactions, conditions and agents known to those skilled in the art, including: (1) the nucleophilic group of the antibody reacts with the divalent linker agent via a covalent bond to form Ab-L, followed by reaction with the drug module D; and (2) the nucleophilic group of the drug module reacts with the divalent linker agent via a covalent bond to form D-L, followed by reaction with the nucleophilic group of the antibody. The exemplary method for preparing the ADC of Formula I via the latter route is described in US2005-0238649A1, which is expressly incorporated herein by reference.

Nucleophilic groups of antibodies include, but are not limited to: (i) an N-terminal amine group; (ii) a side chain amine group such as lysine; (iii) a side chain thiol group such as cysteine; and (iv) a hydroxyl or amino group of saccharide in the glycosylated antibody. Amines, thiols and hydroxyl groups are nucleophilic and are capable of reacting with the electrophilic groups on the linker module to form covalent bonds, and the linker agents include: (i) active esters such as NHS esters, HOBt esters, haloformates, and acid halides; (ii) hydrocarbyl and benzyl halides, such as haloacetamides; (iii) aldehydes, ketones, carboxyl groups and maleimide groups. Some antibodies have a reducible interchain disulfide, that is, a cysteine bridge. The antibody can be completely or partially reduced by treatment with a reducing agent such as DTT (dithiothreitol) or tricarbonyl ethylphosphine (TCEP) to provide coupling reactivity with the linker. Each cysteine bridge will theoretically form two reactive thiol nucleophiles. Alternatively, the sulfhydryl group may be introduced into the antibody via modification of the lysine residue, for example by reacting the lysine residue with 2-imin sulfane (Traut reagent), resulting in the conversion of the amine to the thiol.

The antibody-drug conjugates of the present invention can also be produced by the reaction between an electrophilic group on an antibody (such as an aldehyde or ketone carbonyl group) and a linker or nucleophilic group on a drug. Useful nucleophilic groups on the linker include, but are not limited to: hydrazide, oxime, amino, hydrazine, thiosemicarbazone, hydrazine carboxylate and arylhydrazide. In one embodiment, the saccharide of the glycosylated antibody can be oxidized with, for example, a periodate oxidant to form an aldehyde or ketone group that can react with the amine group of the linker or drug module. The resulting imine Schiff base may form a stable linkage or may be reduced with, for example, a borohydride agent to form a stable amine linkage. In one embodiment, the reaction of the carbohydrate moiety of the glycosylated antibody with galactose oxidase or sodium metaperiodate may produce a carbonyl group (aldehyde group and keto group) in the antibody, which may be reacted with a suitable group on the drug (Hermanson, Bioconjugate Techniques). In another embodiment, an antibody comprising an N-terminal serine or threonine residue may react with sodium metaperiodate, resulting in the formation of an aldehyde at the first amino acid (Geoghegan and Stroh, (1992) Bioconjugate Chem. 3: 138-146; US5362852). Such aldehydes can react with the drug module or the linker nucleophile.

Nucleophilic groups on the drug module include, but are not limited to: amine, thiol, hydroxy, hydrazide, oxime, hydrazine, thiosemicarbazone, hydrazine carboxylate and arylhydrazide groups, which can react with the electrophilic groups on the linker module to form covalent bonds. And the linker agents include: (i) active esters such as NHS esters, HOBt esters, haloformates, and acid halides; (ii) hydrocarbyl and benzyl halides, such as haloacetamides; (iii) aldehydes, ketones, carboxyl groups, and maleimide groups.

The compounds of the present invention clearly cover but are not limited to the ADC prepared by the following crosslinking agents: BMPS, EMCS, GMBS, HBVS, LC-SMCC, MBS, MPBH, SBAP, SIA, SIAB, SMCC, SMPB, SMPH,

sulfo-EMCS, sulfo-GMBS, sulfo-KMUS, sulfo-MBS, sulfo-SIAB, sulfo-SMCC, sulfo-SMPB and SVSB (succinimidyl-(4-vinylsulfone) benzoate), which are commercially available (such as Pierce Biotechnology, Inc., Rockford, IL., U.S.A, □refer to the 2003-2004 Application Manual and product catalog (2003-2004 Applications Handbook and Catalog) pages 467-498).

Antibody-cytotoxic drug conjugates or their pharmaceutically acceptable salts or solvates containing antibodies and cytotoxic agents can also be prepared using a variety of bifunctional protein coupling agents, such as N-succinimidyl 3- (2-pyridyldithio) propionate (SPDP), succinimidyl-4-(N-maleimidomethyl) cyclohexane-1-carboxylate (SMCC), aminosulfane (IT), imidates (such as dimethyl adipamide HCl), active esters (such as disuccinimidyl suberate), aldehydes (such as glutaraldehyde), bis azide compounds (such as bis (p-azidobenzoyl) hexamethylene diamine), bis diazo derivatives (such as bis (p-diazo benzoyl) -ethylenediamine), diisothiocyanate (such as toluene 2,6-Diisocyanate) and dual active fluorine compounds (such as 1,5-difluoro-2,4-dinitrobenzene). For example, ricin immunotoxins can be prepared as described in Vitetta et al., Science 238: 1098 (1987). The carbon-14 labeled 1-isothiocyanate benzyl-3-methyl diethylene triamine pentaacetic acid (MX-DTPA) is an exemplary chelating agent for coupling a radioactive nucleotide to an antibody. See WO 94/11026.

Alternatively, a fusion protein comprising an antibody and a cytotoxic agent can be prepared by, for example, recombinant techniques or peptide synthesis. The recombinant DNA molecule may comprise regions encoding the antibody and cytotoxic moiety of the conjugate respectively, either adjacent to each other or separated by a region encoding a linker peptide, wherein said linker peptide does not destroy the desired properties of the conjugate.

In yet another embodiment, the antibody may be conjugated to a "receptor"; (such as streptavidin) for pre-targeting the tumor, the antibody-receptor conjugate is administered to a patient, followed by the use of a scavenger which removes the unbound conjugates from circulation. Then, a "ligand" (e.g., avidin) coupled to a cytotoxic agent (such as a radioactive nucleotide) is administrated. The following examples are provided for illustrative purposes only and are not intended to limit the scope of the invention.

## Beneficial effects of the invention

When compared with the prior art, the technical solution of the present invention has the following advantages:

(1) The anti-c-Met antibody-cytotoxic drug conjugate (ADC) of the present invention, as the single component, has a significant inhibitory effect on hepatic carcinoma cells, effectively inhibits the proliferation thereof, can inhibit the growth and volume of hepatic carcinoma cells for a long time. On the other hand, the antibody alone has a poor inhibitory effect on hepatic carcinoma cells, and the difference between the two is remarkable. Therefore, the ADC drug of the present invention can be preferably used as a pharmaceutically active ingredient.

(2) Studies have shown that the anti-c-Met antibody-cytotoxic drug conjugate (ADC) of the present invention is well tolerated in animals, and the anti-c-Met antibody-cytotoxic drug conjugate obtained by the technical solution of the present invention (ADC) can be applied into industrial production.

## BRIEF DESCRIPTION OF THE DRAWINGS

Figure 1 is the curve tracing the tumor volume, showing the inhibition of hepatic carcinoma cells by ADC molecule of the present invention in LI-03-0022 HCC PDX tumor model. The results show that the ADC molecule can achieve complete tumor inhibition by the introduced toxin, whereas the antibody alone cannot. The ADC drug of the invention has good tolerance in tumor-bearing animals; the data represent the mean value of group, and the error bar represents the standard error of mean value (SEM);

Figure 2 is IHC (immunohistochemical staining) score of LI-03-0010 (negative control) in LI-03-0022 HCC PDX tumor model;

Figure 3 is IHC (immunohistochemical staining) score of LI-03-0022 tissue in LI-03-0022 HCC PDX tumor model;

Figure 4 is the curve tracing the tumor volume, showing the inhibition of hepatic carcinoma cells by ADC molecule of the present invention in LI-03-0240 HCC PDX tumor model;

Figure 5 is IHC (immunohistochemical staining) score of LI-03-0010 (negative control) in LI-03-0240 HCC PDX tumor model;

Figure 6 is IHC (immunohistochemical staining) score of LI-03-0240 tissue in LI-03-0240 HCC PDX tumor model.

## DETAILED DESCRIPTION OF THE INVENTION

Hereinafter, the present invention is illustrated in more details with reference to examples. The examples of

the invention are merely used to exemplify the technical solution of the invention, the merits and scope of the invention is not limited thereto.

In the examples or test examples of the present invention, where specific conditions are not described, the experiments are generally conducted under conventional conditions, or under conditions proposed by the manufacturers of material or product. See Sambrook et al., Molecular Cloning, A Laboratory Manual, Cold Spring Harbor Laboratory; Current Protocols in Molecular Biology, Ausubel et al, Greene Publishing Associates, Wiley Interscience, NY. Where the source of the agents is not specifically given, the agents are commercially available.

## EXAMPLES

### Example 1. Clonal expression of antigen and antibody

The antibodies (light and heavy chains) and antigens used in the present invention are constructed by overlapping extension PCR methods known in the art. The DNA fragment obtained by overlapping extension PCR was inserted into the expression vector pEE6.4 (Lonza Biologies) using HindIII/BstBI restriction sites, and expressed in 293F cells (Invitrogen, Cat # R790-07). The resulting recombinant protein is used for immunization or screening. The c-Met gene template is derived from origene Corporation (number RC217003). The DNA sequences to be cloned and expressed are as follows:

DNA sequence for fusion protein of human c-Met extracellular region (ECD) and murine Fc region (human c-Met ECD-mFc):

atgaaggcccccgctgtgcttgcacctggcatcctcgtgctcctgtttaccttggtgcagaggagcaatggggagtgtaaag
aggcactagcaaagtccgagatgaatgtgaatatgaagtatcagcttcccaacttcaccgcggaaacacccatccagaatg
tcattctacatgagcatcacattttccttggtgccactaactacatttatgttttaaatgaggaagaccttcagaaggttgctgagt
acaagactgggcctgtgctggaacacccagattgtttcccatgtcaggactgcagcagcaaagccaatttatcaggaggtg
tttggaaagataacatcaacatggctctagttgtcgacacctactatgatgatcaactcattagctgtggcagcgtcaacaga

gggacctgccagcgacatgtctttccccacaatcatactgctgacatacagtcggaggttcactgcatattctccccacagat
agaagagcccagccagtgtcctgactgtgtggtgagcgccctgggagccaaagtcctttcatctgtaaaggaccggttcat
caacttctttgtaggcaataccataaattcttcttatttcccagatcatccattgcattcgatatcagtgagaaggctaaaggaaa
cgaaagatggttttatgtttttgacggaccagtcctacattgatgttttacctgagttcagagattcttaccccattaagtatgtcc
atgcctttgaaagcaacaattttatttacttcttgacggtccaaagggaaactctagatgctcagacttttcacacaagaataatc
aggttctgttccataaactctggattgcattcctacatggaaatgcctctggagtgtattctcacagaaaagagaaaaaagag
atccacaaagaaggaagtgtttaatatacttcaggctgcgtatgtcagcaagcctggggcccagcttgctagacaaatagga
gccagcctgaatgatgacattcttttcggggtgttcgcacaaagcaagccagattctgccgaaccaatggatcgatctgcca
tgtgtgcattccctatcaaatatgtcaacgacttcttcaacaagatcgtcaacaaaaacaatgtgagatgtctccagcatttttac
ggacccaatcatgagcactgctttaataggacacttctgagaaattcatcaggctgtgaagcgcgccgtgatgaatatcgaa
cagagtttaccacagctttgcagcgcgttgacttattcatgggtcaattcagcgaagtcctcttaacatctatatccaccttcatt
aaaggagacctcaccatagctaatcttgggacatcagagggtcgcttcatgcaggttgtggtttctcgatcaggaccatcaa
cccctcatgtgaattttctcctggactcccatccagtgtctccagaagtgattgtggagcatacattaaaccaaaatggctaca
cactggttatcactgggaagaagatcacgaagatcccattgaatggcttgggctgcagacatttccagtcctgcagtcaatg
cctctctgccccacccttgttcagtgtggctggtgccacgacaaatgtgtgcgatcggaggaatgcctgagcgggacatg
gactcaacagatctgtctgcctgcaatctacaaggttttcccaaatagtgcaccccttgaaggagggacaaggctgaccata
tgtggctgggactttggatttcggaggaataataaatttgatttaaagaaaactagagttctccttggaaatgagagctgcacc
ttgactttaagtgagagcacgatgaatacattgaaatgcacagttggtcctgccatgaataagcatttcaatatgtccataattat
ttcaaatggccacgggacaacacaatacagtacattctcctatgtggatcctgtaataacaagtatttcgccgaaatacggtc
ctatggctggtggcactttacttactttaactggaaattacctaaacagtgggaattctagacacatttcaattggtggaaaaac
atgtactttaaaaagtgtgtcaaacagtattcttgaatgttataccccagcccaaaccatttcaactgagtttgctgttaaattgaa
aattgacttagccaaccgagagacaagcatcttcagttaccgtgaagatcccattgtctatgaaattcatccaaccaaatctttt
attagtggtgggagcacaataacaggtgttgggaaaaacctgaattcagttagtgtcccgagaatggtcataaatgtgcatg
aagcaggaaggaactttacagtggcatgtcaacatcgctctaattcagagataatctgttgtaccactccttccctgcaacag
ctgaatctgcaactccccctgaaaaccaaagcctttttcatgttagatgggatcctttccaaatactttgatctcatttatgtacat
aatcctgtgtttaagccttttgaaaagccagtgatgatctcaatgggcaatgaaaatgtactggaaattaagggaaatgatatt
gaccctgaagcagttaaaggtgaagtgttaaaagttggaaataagagctgtgagaatatacacttacattctgaagccgtttta
tgcacggtccccaatgacctgctgaaattgaacagcgagctaaatatagagtggaagcaagcaatttcttcaaccgtccttg
gaaaagtaatagttcaaccagatcagaatttcaca (SEQ ID NO: 1)

DNA sequence for human c-Met extracellular Sema region and Flag-His tag (Human c-Met Sema-Flis):

atgaaggcccccgctgtgcttgcacctggcatcctcgtgctcctgtttaccttggtgcagaggagcaatggggagtgtaaag
aggcactagcaaagtccgagatgaatgtgaatatgaagtatcagcttcccaacttcaccgcggaaacacccatccagaatg
tcattctacatgagcatcacattttccttggtgccactaactacatttatgttttaaatgaggaagaccttcagaaggttgctgagt
acaagactgggcctgtgctggaacacccagattgtttcccatgtcaggactgcagcagcaaagccaatttatcaggaggtg
tttggaaagataacatcaacatggctctagttgtcgacacctactatgatgatcaactcattagctgtggcagcgtcaacaga
gggacctgccagcgacatgtctttccccacaatcatactgctgacatacagtcggaggttcactgcatattctccccacagat
agaagagcccagccagtgtcctgactgtgtggtgagcgccctgggagccaaagtcctttcatctgtaaaggaccggttcat
caacttctttgtaggcaataccataaattcttcttatttcccagatcatccattgcattcgatatcagtgagaaggctaaaggaaa
cgaaagatggttttatgtttttgacggaccagtcctacattgatgttttacctgagttcagagattcttaccccattaagtatgtcc
atgcctttgaaagcaacaattttatttacttcttgacggtccaaagggaaactctagatgctcagacttttcacacaagaataatc
aggttctgttccataaactctggattgcattcctacatggaaatgcctctggagtgtattctcacagaaaagagaaaaaagag
atccacaaagaaggaagtgtttaatatacttcaggctgcgtatgtcagcaagcctggggcccagcttgctagacaaatagga
gccagcctgaatgatgacattcttttcggggtgttcgcacaaagcaagccagattctgccgaaccaatggatcgatctgcca
tgtgtgcattccctatcaaatatgtcaacgacttcttcaacaagatcgtcaacaaaaacaatgtgagatgtctccagcatttttac
ggacccaatcatgagcactgctttaataggacacttctgagaaattcatcaggctgtgaagcgcgccgtgatgaatatcgaa
cagagtttaccacagctttgcagcgcgttgacttattcatgggtcaattcagcgaagtcctcttaacatctatatccaccttcatt
aaaggagacctcaccatagctaatcttgggacatcagagggtcgcttcatgcaggttgtggtttctcgatcaggaccatcaa
cccctcatgtgaattttctcctggactcccatccagtgtctccagaagtgattgtggagcatacattaaaccaaaatggctaca
cactggttatcactgggaagaagatcacgaagatcccattgaatggcttgggctgcagacatttccagtcctgcagtcaatg
cctctctgccccaccctttgttcagtgtggctggtgccacgacaaatgtgtgcgatcggaggaatgcctgagcgggacatg
gactcaacagatctgtctgcctgcaatctacaaggactacaaggacgacgacgacaagcatgtccaccatcatcaccatca
ctgattcgaa (SEQ ID NO: 2)

DNA sequence for human c-Met ECD his tag (Human c-Met ECD-His) recombinant protein:

atgaaggcccccgctgtgcttgcacctggcatcctcgtgctcctgtttaccttggtgcagaggagcaatggggagtgtaaag
aggcactagcaaagtccgagatgaatgtgaatatgaagtatcagcttcccaacttcaccgcggaaacacccatccagaatg
tcattctacatgagcatcacattttccttggtgccactaactacatttatgttttaaatgaggaagaccttcagaaggttgctgagt
acaagactgggcctgtgctggaacacccagattgtttcccatgtcaggactgcagcagcaaagccaatttatcaggaggtg
tttggaaagataacatcaacatggctctagttgtcgacacctactatgatgatcaactcattagctgtggcagcgtcaacaga
gggacctgccagcgacatgtctttccccacaatcatactgctgacatacagtcggaggttcactgcatattctccccacagat

agaagagcccagccagtgtcctgactgtgtggtgagcgccctgggagccaaagtcctttcatctgtaaaggaccggttcat
caacttctttgtaggcaataccataaattcttcttatttcccagatcatccattgcattcgatatcagtgagaaggctaaaggaaa
cgaaagatggttttatgtttttgacggaccagtcctacattgatgttttacctgagttcagagattcttaccccattaagtatgtcc
atgcctttgaaagcaacaattttatttacttcttgacggtccaaagggaaactctagatgctcagacttttcacacaagaataatc
aggttctgttccataaactctggattgcattcctacatggaaatgcctctggagtgtattctcacagaaaagagaaaaaagag
atccacaaagaaggaagtgtttaatatacttcaggctgcgtatgtcagcaagcctggggcccagcttgctagacaaatagga
gccagcctgaatgatgacattcttttcggggtgttcgcacaaagcaagccagattctgccgaaccaatggatcgatctgcca
tgtgtgcattccctatcaaatatgtcaacgacttcttcaacaagatcgtcaacaaaaacaatgtgagatgtctccagcatttttac
ggacccaatcatgagcactgctttaataggacacttctgagaaattcatcaggctgtgaagcgcgccgtgatgaatatcgaa
cagagtttaccacagctttgcagcgcgttgacttattcatgggtcaattcagcgaagtcctcttaacatctatatccaccttcatt
aaaggagacctcaccatagctaatcttgggacatcagagggtcgcttcatgcaggttgtggtttctcgatcaggaccatcaa
cccctcatgtgaattttctcctggactcccatccagtgtctccagaagtgattgtggagcatacattaaaccaaaatggctaca
cactggttatcactgggaagaagatcacgaagatcccattgaatggcttgggctgcagacatttccagtcctgcagtcaatg
cctctctgccccacccttttgttcagtgtggctggtgccacgacaaatgtgtgcgatcggaggaatgcctgagcgggacatg
gactcaacagatctgtctgcctgcaatctacaaggttttcccaaatagtgcaccccttgaaggagggacaaggctgaccata
tgtggctgggactttggatttcggaggaataataaatttgatttaaagaaaactagagttctccttggaaatgagagctgcacc
ttgactttaagtgagagcacgatgaatacattgaaatgcacagttggtcctgccatgaataagcatttcaatatgtccataattat
ttcaaatggccacgggacaacacaatacagtacattctcctatgtggatcctgtaataacaagtatttcgccgaaatacggtc
ctatggctggtggcactttacttactttaactggaaattacctaaacagtgggaattctagacacatttcaattggtggaaaaac
atgtactttaaaaagtgtgtcaaacagtattcttgaatgttataccccagcccaaaccatttcaactgagtttgctgttaaattgaa
aattgacttagccaaccgagagacaagcatcttcagttaccgtgaagatcccattgtctatgaaattcatccaaccaaatctttt
attagtggtgggagcacaataacaggtgttgggaaaaacctgaattcagttagtgtcccgagaatggtcataaatgtgcatg
aagcaggaaggaactttacagtggcatgtcaacatcgctctaattcagagataatctgttgtaccactccttccctgcaacag
ctgaatctgcaactccccctgaaaaccaaagcctttttcatgttagatgggatcctttccaaatactttgatctcatttatgtacat
aatcctgtgtttaagccttttgaaaagccagtgatgatctcaatgggcaatgaaaatgtactggaaattaagggaaatgatatt
gaccctgaagcagttaaaggtgaagtgttaaaagttggaaataagagctgtgagaatatacacttacattctgaagccgtttta
tgcacggtccccaatgacctgctgaaattgaacagcgagctaaatatagagtggaagcaagcaatttcttcaaccgtccttg
gaaaagtaatagttcaaccagatcagaatttcacacaccatcatcaccatcactgattcgaa (SEQ ID NO: 3)

**Example 2. Binding assay of antibody and antigen** (ELISA)

This experiment uses enzyme linked immunosorbent assay to detect affinity of c-Met antibody (including supernatant of hybridoma or recombinant expressed monoclonal antibodies)to c-Met antigen *in vitro.*

Experimental procedures: Coating buffer (PBS; Hyclone, Cat No.: SH30256.01B) was used to dilute antigen (human c-Met-His, example 1) to 2μg/mL, which was added to 96-well microplate with 100μL/well (Costar 9018, Cat No. :03113024) and incubated overnight at 4°C. The next day, the antibody-coated 96-well microplate was restored to room temperature and was washed three times with washing buffer (PBS+0.05%Tween 20 (Sigma, Cat No.:P1379).

Blocking buffer was added at 200μL/well (PBS+1%BSA (Roche, Cat No.:738328) and the plate was incubated at 37°C for 1 hour. The plate was then washed three times with washing buffer. Anti c-Met antibody to be tested was added to the 96-well microplate and was incubated for 1 hour at room temperature. The plate was then washed three times with washing buffer. Secondary antibody (Goat anti-Mouse IgG(H+L)(HRP) (Thermo, No.:31432) diluted with blocking buffer (10000× dilution) was added to the 96-well microplate at 100μL/well and the plate was incubated for 1 hour at room temperature. The plate was then washed three times and TMB chromogenic substrate (eBioscience REF:00-4201-56) was added to the 96-well microplate at 100μL/well. Stop solution 2N $H_2SO_4$ was added to the 96-well microplate at 100μL/well. Read the plate with plate reader at 450nm.

**Example 3. Production of murine monoclonal antibody cell strain against human c-Met**

In this invention, murine anti-huamn c-Met monoclonal cell strain was obtained by immunizing mice, fusion of spleen cell and screening of hybridoma. This method is well-known in this field. Recombinant expressed antigen (human c-Met ECD-mFc, human c-Met Sema-flis, see example 1) was diluted to 1mg/mL with PBS (Hyclone, Cat No.:SH30256.01B) and emulsified with Freund's adjuvant (the first immunization was performed with Freund's complete adjuvant, and the booster immunizations were performed with Freund's incomplete adjuvant); and injected into Balb/C mice subcutaneously (5 mice/group) with each mouse inoculated 100μg antigen, booster immunizations were given every two weeks. Since the first booster immunization, mice serum was collected during 7 to 10 days after each booster immunization, and the titer was detected by ELISA (Methods were described in Example 2).

After immunization, mice with serum titer higher than 1:$10^5$ were selected for cell fusion. Mice B-cells and myeloma cells (SP2/0, ATCC number:CRL-1581™) were prepared respectively in aseptic condition and counted. The two kinds of cells were mixed at a ratio of B-cells : SP2/0 of 1:4 and then were centrifuged (1500r/min,7min). Supernatant was discarded and 1mL of 50% polyethylene glycol (Supplier: SIGMA, Catalogue# RNBB306) was added. Next, 1mL serum-free RPMI1640 (Supplier: GIBCO, Catalogue#C22400) was used for termination and centrifuged for 10 minutes. Supernatant was then discarded. The pellet was resuspended in RPMI1640 which comprises hybridoma cell growth factor (Supplier: Roche, Catalogue# 1363735001), serum (Supplier: GIBCO, Catalogue#C20270) and HAT (Supplier: Invitrogen, Catalogue# 21060-017). B-cells were plated on the plate at $10^5$cells/well and each well is 100μL. The plate was placed in cell incubator at 37°C. Three days later, 100μL of RPMI1640 which comprises hybridoma cell growth factor, serum and HT (Supplier: Invitrogen, Catalogue# 11067-030) was added in each well. After 2 to 4 days, each well was replaced for 150μL RPMI1640 comprising hybridoma cell growth factor, serum and HT. Next day, positive clone was detected by ELISA (see Methods in Example 2).

Experimental results:

Table 1. Detection of hybridoma fusion of mice immunized with human c-Met

| Clone No. | Detection Results (OD450) |
|---|---|
| Negative control | 0.07 |
| Ab-1 | 1.48 |
| Ab-2 | 1.38 |
| Ab-3 | 1.29 |
| Ab-4 | 1.6 |
| Ab-5 | 1.64 |
| Ab-6 | 1.75 |
| Ab-7 | 1.58 |
| Ab-8 | 1.24 |

**Example 4. Cloning of anti c-Met antibody sequence**

The single cell stain Ab-5 was selected for cDNA sequence cloning. Mab was recombinant expressed and subjected to various activity tests. The variable regions of heavy chain and light chain of antibody gene were amplified by reverse transcription PCR, and ligated to vector to obtain heavy and light chain sequence of monoclonal antibody by

sequencing. First of all, RNA purification kit (Qiagen company, Cat. No.74134, see the instructions for this procedure) was used to extract total RNAs from the active single cell stain from example 3. Next, cDNA single chain was prepared by the cDNA synthesis kit (Invitrogen company, Cat. No. 18080-051), that is, Oligo-dT primers cDNA reverse transcription. The product was served as template, and the variable region sequence of the antibody heavy and light chain was synthesized by PCR method. The products of PCR were cloned to TA vector pMD-18T and then sequenced. Obtained heavy and light chain sequence of antibody were separately cloned to expression vectors (see example 1), and the recombinant monoclonal antibody was expressed to prove its activity (see examples 2 and 3), followed by humanization.

Sequence of mice hybridoma cell monoclonal antibody Ab-5:

Heavy chain variable region:

QVQLKQSGPGLVQPSQSLSITCTVSGFSLP**NYGVH**WVRQSPGKGLEWLG**VIW SGGSTNYAAAFVS**RLRISKDNSKSQVFFEMNSLQADDTAVYYCAR**NHDNPY NYAMDY**WGQGTTVTVSS (SEQ ID NO: 4)

Light chain variable region:

DIVLTQSPGSLAVYLGQRATISC**RANKSVSTSTYNYLH**WYQQKPGQPPKLLI Y**LASNLAS**GVPARFSGSGSGTDFTLNIHPLEEEDAATYYC**QHSRDLPPT**FGA GTKLELKR (SEQ ID NO: 5)

The amino acid residues of VH/VL CDR of anti-human c-Met antibodies are determined and annotated by the Kabat numbering system.

CDR sequences of murine in the invention are shown in Table below:

Table 2. CDR sequence of Murine anti-sclerostin antibody

| **Antibody** | Ab-5 | |
|---|---|---|
| **Heavy chain CDR1** | NYGVH | (SEQ ID NO: 6) |
| **Heavy chain CDR2** | VIWSGGSTNYAAAFVS | (SEQ ID NO: 7) |
| **Heavy chain CDR3** | NHDNPYNYAMDY | (SEQ ID NO: 8) |
| **Light chain CDR1** | RANKSVSTSTYNYLH | (SEQ ID NO: 9) |
| **Light chain CDR2** | LASNLAS | (SEQ ID NO: 10) |
| **Light chain CDR3** | QHSRDLPPT | (SEQ ID NO: 11) |

**Example 5. Humanization of anti c-Met antibody**

The murine anti c-Met monoclonal antibody heavy and light chain sequence obtained from example 4 was aligned against antibody data base for homology, and a humanized antibody model was established then. The optimal humanized c-Met monoclonal antibody was selected as the preferred molecule of the invention according to the model for back-mutation. Crystal structure showing similar homology with the obtained murine candidate molecules was selected from the published database of mice Fab crystal structure model (e.g. PDB database), and Fab crystal structure with high resolution (such as, less than 2.5 Å) was selected; and mouse Fab model was established. The murine antibody heavy and light chain sequences of the invention were aligned against the sequences in model, the consistent sequence was maintained and then the structure model of mouse antibody of the invention would be obtained. The inconsistent amino acids might be potential sites for back-mutation. Swiss-pdb viewer software was used to run the mouse antibody structure model to optimize energy (minimization). Back-mutation was performed at different amino acid sites other than those in CDRs of the model, and the activities of the resultant humanized antibody and of the antibody without humanization was compared. Humanized antibody with good activity was maintained. And then, the CDR region was further optimized, including avoiding glycosylation, deamidization, oxidation sites and so on. CDR region of the optimized humanized anti c-Met antibody is shown in table below:

Table 3. CDR sequence of the optimized anti c-Met antibody

| Antibody | Optimized humanized antibody | |
|---|---|---|
| **Heavy Chain CDR1** | NYGVH | (SEQ ID NO: 6) |
| **Heavy Chain CDR2** | VIWSGGSTNYAAAFVS | (SEQ ID NO: 7) |
| **Heavy Chain CDR3** | NHDNPYNYAMDY | (SEQ ID NO: 8) |
| **Light Chain CDR1** | RADKSVSTSTYNYLH | (SEQ ID NO: 12) |
| **Light Chain CDR2** | LASNLAS | (SEQ ID NO: 10) |
| **Light Chain CDR3** | QHSRDLPPT | (SEQ ID NO: 11) |

Variable regions of humanized heavy and light chain sequences are shown below:

1. Heavy chain variable regions

Ab-9

QVTLKESGPVLVKPTETLTLTCTVSGFSLP**NYGVH**WVRQPPGKALEWLA**VIWSGGSTNYAAAFVS**RLRISKDTSKSQVVFTMNNMDPVDTATYYCAR**NHDNPYNYAMDY**WGQGTTVTVSS (SEQ ID NO: 13)

Ab-10

QVQLVESGGGVVQPGRSLRLSCAASGFSLS**NYGVH**WVRQAPGKGLEWLA**VIWSGGSTNYAAAFVS**RLTISKDNSKNTVYLQMNSLRAEDTAVYYCAR**NHDNPYNYAMDY**WGQGTTVTVSS (SEQ ID NO: 14)

Ab-11

QVQLVESGGGVVQPGRSLRLSCAASGFTLP**NYGVH**WVRQAPGKGLEWLA**VIWSGGSTNYAAAFVS**RLTISKDNSKNTVYLQMNSLRAEDTAVYYCAR**NHDNPYNYAMDY**WGQGTTVTVSS (SEQ ID NO: 15)

2. Light chain variable regions

Ab-9

DIVLTQSPASLAVSPGQRATITC**RANKSVSTSTYNYLH**WYQQKPGQPPKLLIY**LASNLAS**GVPARFSGSGSGTDFTLTINPVEANDTANYYC**QHSRDLPPT**FGQGTKLEIKR (SEQ ID NO: 16)

Ab-10

DIVLTQSPDSLAVSLGERATINC**RADKSVSTSTYNYLH**WYQQKPGQPPKLLIY **LASNLAS**GVPDRFSGSGSGTDFTLTISSLQAEDVAVYYC**QHSRDLPPT**FGQG TKLEIKR (SEQ ID NO: 17)

Ab-11

DIVLTQSPDSLAVSLGERATINC**RANKSVSTSTYNYLH**WYQQKPGQPPKLLIY **LASNLAS**GVPDRFSGSGSGTDFTLTISSLQAEDVAVYYC**QHSRDLPPT**FGQG TKLEIKR (SEQ ID NO: 18)

The humanized heavy and light chain sequences are recombined with IgG Fc regions to obtain the humanized anti c-Met monoclonal antibody of the invention. The Fc sequence used was selected optionally from the following sequences:

Heavy chain constant region:

ASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKVEPKSCDKT HTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNK ALPAPIEKTISKAKGQPREPQVYTLPPSREEMTKNQVSLTCLVKGFYPSDIAVE WESNGQPENNYKTTPPVLDSDGSFFLYSKLTVDKSRWQQGNVFSCSVMHEA LHNHYTQKSLSLSPGK (SEQ ID NO: 19)

ASTKGPSVFPLAPCSRSTSESTAALGCLVKDYFPEPVTVSWNSGALTSGVHTF PAVLQSSGLYSLSSVVTVPSSNFGTQTYTCNVDHKPSNTKVDKTVERKCCVE CPPCPAPPVAGPSVFLFPPKPKDTLMISRTPEVTCVVVDVSHEDPEVQFNWYV DGVEVHNAKTKPREEQFNSTFRVVSVLTVVHQDWLNGKEYKCKVSNKGLPA PIEKTISKTKGQPREPQVYTLPPSREEMTKNQVSLTCLVKGFYPSDIAVEWESN GQPENNYKTTPPMLDSDGSFFLYSKLTVDKSRWQQGNVFSCSVMHEALHNH YTQKSLSLSPGK (SEQ ID NO: 20)

ASTKGPSVFPLAPCSRSTSESTAALGCLVKDYFPEPVTVSWNSGALTSGVHTF
PAVLQSSGLYSLSSVVTVPSSSLGTKTYTCNVDHKPSNTKVDKRVESKYGPPC
PPCPAPEFLGGPSVFLFPPKPKDTLMISRTPEVTCVVVDVSQEDPEVQFNWYV
DGVEVHNAKTKPREEQFNSTYRVVSVLTVLHQDWLNGKEYKCKVSNKGLPS
SIEKTISKAKGQPREPQVYTLPPSQEEMTKNQVSLTCLVKGFYPSDIAVEWES
NGQPENNYKTTPPVLDSDGSFFLYSRLTVDKSRWQEGNVFSCSVMHEALHN
HYTQKSLSLSLGK (SEQ ID NO: 21)

Light chain constant region:

TVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGE
C (SEQ ID NO: 22)

The above antibodies were cloned, expressed and purified by gene cloning and recombinant expression, respectively. The humanized antibodies Ab-9, Ab-10 and Ab-11 with best activity were finally selected through ELISA (Example 2) and *in vitro* binding activity assay (Example 6). The sequences are shown as below:

Ab-9 humanized antibody:

Heavy chain:

QVTLKESGPVLVKPTETLTLTCTVSGFSLPNYGVHWVRQPPGKALEWLA
VIWSGGSTNYAAAFVSRLRISKDTSKSQVVFTMNNMDPVDTATYYCARNHD
NPYNYAMDYWGQGTTVTVSSASTKGPSVFPLAPCSRSTSESTAALGCLVKDY
FPEPVTVSWNSGALTSGVHTFPAVLQSSGLYSLSSVVTVPSSNFGTQTYTCNV
DHKPSNTKVDKTVERKCCVECPPCPAPPVAGPSVFLFPPKPKDTLMISRTPEV
TCVVVDVSHEDPEVQFNWYVDGVEVHNAKTKPREEQFNSTFRVVSVLTVVH
QDWLNGKEYKCKVSNKGLPAPIEKTISKTKGQPREPQVYTLPPSREEMTKNQ
VSLTCLVKGFYPSDIAVEWESNGQPENNYKTTPPMLDSDGSFFLYSKLTVDKS
RWQQGNVFSCSVMHEALHNHYTQKSLSLSPGK (SEQ ID NO: 23)

Light chain:

DIVLTQSPASLAVSPGQRATITCRANKSVSTSTYNYLHWYQQKPGQPPKL
LIYLASNLASGVPARFSGSGSGTDFTLTINPVEANDTANYYCQHSRDLPPTFG
QGTKLEIKRTVAAPSVFIFPPSDEQLKSGTASVVCLLNNFYPREAKVQWKVDN
ALQSGNSQESVTEQDSKDSTYSLSSTLTLSKADYEKHKVYACEVTHQGLSSP
VTKSFNRGEC (SEQ ID NO: 26)

**Ab-10 humanized antibody:**

Heavy chain:

QVQLVESGGGVVQPGRSLRLSCAASGFSLSNYGVHWVRQAPGKGLEWL
AVIWSGGSTNYAAAFVSRLTISKDNSKNTVYLQMNSLRAEDTAVYYCARNH
DNPYNYAMDYWGQGTTVTVSSASTKGPSVFPLAPCSRSTSESTAALGCLVKD
YFPEPVTVSWNSGALTSGVHTFPAVLQSSGLYSLSSVVTVPSSNFGTQTYTCN
VDHKPSNTKVDKTVERKCCVECPPCPAPPVAGPSVFLFPPKPKDTLMISRTPE
VTCVVVDVSHEDPEVQFNWYVDGVEVHNAKTKPREEQFNSTFRVVSVLTVV
HQDWLNGKEYKCKVSNKGLPAPIEKTISKTKGQPREPQVYTLPPSREEMTKN
QVSLTCLVKGFYPSDIAVEWESNGQPENNYKTTPPMLDSDGSFFLYSKLTVD
KSRWQQGNVFSCSVMHEALHNHYTQKSLSLSPGK (SEQ ID NO: 24)

Light chain:

DIVLTQSPDSLAVSLGERATINCRADKSVSTSTYNYLHWYQQKPGQPPKL
LIYLASNLASGVPDRFSGSGSGTDFTLTISSLQAEDVAVYYCQHSRDLPPTFGQ
GTKLEIKRTVAAPSVFIFPPSDEQLKSGTASVVCLLNNFYPREAKVQWKVDNA
LQSGNSQESVTEQDSKDSTYSLSSTLTLSKADYEKHKVYACEVTHQGLSSPVT
KSFNRGEC (SEQ ID NO: 27)

**Ab-11 humanized antibody:**

Heavy chain:

QVQLVESGGGVVQPGRSLRLSCAASGFTLPNYGVHWVRQAPGKGLEWL
AVIWSGGSTNYAAAFVSRLTISKDNSKNTVYLQMNSLRAEDTAVYYCARNH
DNPYNYAMDYWGQGTTVTVSSASTKGPSVFPLAPCSRSTSESTAALGCLVKD
YFPEPVTVSWNSGALTSGVHTFPAVLQSSGLYSLSSVVTVPSSNFGTQTYTCN
VDHKPSNTKVDKTVERKCCVECPPCPAPPVAGPSVFLFPPKPKDTLMISRTPE
VTCVVVDVSHEDPEVQFNWYVDGVEVHNAKTKPREEQFNSTFRVVSVLTVV
HQDWLNGKEYKCKVSNKGLPAPIEKTISKTKGQPREPQVYTLPPSREEMTKN
QVSLTCLVKGFYPSDIAVEWESNGQPENNYKTTPPMLDSDGSFFLYSKLTVD
KSRWQQGNVFSCSVMHEALHNHYTQKSLSLSPGK (SEQ ID NO: 25)

Light chain:

DIVLTQSPDSLAVSLGERATINCRANKSVSTSTYNYLHWYQQKPGQPPKL
LIYLASNLASGVPDRFSGSGSGTDFTLTISSLQAEDVAVYYCQHSRDLPPTFGQ
GTKLEIKRTVAAPSVFIFPPSDEQLKSGTASVVCLLNNFYPREAKVQWKVDNA
LQSGNSQESVTEQDSKDSTYSLSSTLTLSKADYEKHKVYACEVTHQGLSSPVT
KSFNRGEC (SEQ ID NO: 28)

**Example 6. Detection of *In vitro* binding activity of anti c-Met humanized antibody**

The humanized antibody of the invention was detected for its *in vitro* activity by ELISA (Example 2), and also detected for its binding with cell stain MKN45 highly expressing c-Met, and for its affinity to c-Met antigen (BIACore assay).

FACS method was used to detect the binding activity of c-Met humanized antibody with cell stain MKN45 highly expressing c-Met.

MKN45 cells (JCRB, Cat No.: JCRB0254) were resuspended in RPMI1640 medium (GIBCO, Cat No.: 11835-030) which contains 10% (v/v) fetal calf serum (FBS GIBCO, Cat No.: 10099-141) and Penicillin/Streptomycin (GIBCO, Cat No.: 15070-063) to reach 10000,000 cells/mL. 2mL of resuspended MKN45 cells was added to 96-well microtiter plate (Corning, Cat No.: 3799) at 150000 cells/well, 8 concentrations of c-Met antibody (5-fold gradient diluted, starting from 20μg/mL) were added to the corresponding wells and the final volume was 100μL, the plate was incubated for 1 hour at 4°C. FACS buffer (PBS comprising 2.5% (v/v) FBS (Hyclone, Cat: SH30256.01B)) was added. It was centrifuged under 4°C at 1300rmp for 4 minutes, then the supernatant was discarded. This procedure was repeated for three times. 100μL of secondary antibodies (Fluorescence labeled goat-anti-mouse secondary antibodies with 1:200 dilution, Biolegend, Cat No. 405307; Fluorescence labeled anti-human secondary antibody with 1:30 dilution, Biolegend, Cat No. 409304) were added to each well, and the plate was incubated for 1 hour at 4°C. FACS buffer was added and centrifuged under 4°C at 1300rpm for 4 minutes, and then the supernatants were discarded; this procedure was repeated for three times. 200μL FACS buffer was added to resuspend the cells, the prepared sample was detected by flow cytometry (BD FACS Array).

The affinity of c-Met antibody to c-Met antigen Sema-His was detected by surface plasmon resonance (SPR) in the invention.

Anti-mouse IgG (GE Life Sciences catalog # BR-1008-38) or anti-human IgG (GE Life Sciences catalog # BR-1008-39) antibodies were respectively diluted to 30μg/mL and 50μg/mL by sodium acetate solution pH 5.0 (GE Healthcare, Cat#BR-1003-51). Amino coupling kit (GE Life Sciences, Cat#BR100050) was immobilized onto the test channels and control channels on CM5 chip (GE Life Sciences catalog # BR-1000-12), and the coupling level was set at 15000RU. The c-Met antibody was diluted with Running buffer PBS (Hyclone, Cat#SH30256.01B) + 0.05% P20 (GE Life Sciences, Cat#BR-1000-54) to 1.5μg/mL. Antigen Sema-His was diluted to 200nM with running buffer, and then diluted at 1:2 dilution with the same buffer until 0.78nM is reached. The diluted antibody passed through test channel for 1 minute at a speed of 30μL/min, and the antigen passed through the test channels and control channels for 3 minutes at the same speed. 10 minutes after dissociation, the flow speed was adjusted to 10μL/min, and regeneration buffer passed through test channels and control channels for 3 minutes. Data was fitted by BiaEvaluation 4.1 after double deduction, and the fitting model is 1:1 Langmuir model.

Experimental results:

Table 4. Binding activity of humanized anti c-Met antibody

| Humanized antibody | Ab-9 | Ab-10 | Ab-11 |
|---|---|---|---|
| ELISA assay ($EC_{50}$, nM) | 0.13 | 0.39 | 0.2 |

Table 5. Binding activity and affinity of humanized anti c-Met antibody to MKN45 and to antigen

| Humanized antibody | MKN45/FCAS Binding activity (nM) | affinity to antigen Biacore(nM) |
|---|---|---|
| Ab-9 | 1.6 | 4 |

(continued)

| Humanized antibody | MKN45/FCAS Binding activity (nM) | affinity to antigen Biacore(nM) |
|---|---|---|
| Ab-10 | 1.23 | 8 |

Conclusion: The above experimental results show that the binding activity of humanized antibody with antigen is within 0.13-8 nM, and the results may vary depending on the detection methods used. The results show that humanized anti c-Met antibody maintains binding activity of parent antibody prior to humanization.

**Example 7. Endocytosis of anti c-Met antibody**

The antibody of the invention binds to human c-Met, and has very good *in vitro* activity and good activity in inhibiting tumor activity *in vivo.* In addition, the antibody does not have agonist activity, or has very weak agonist activity. In order to detect whether the antibody would be internalized into the cell along with human c-Met once bound to human c-Met, human gastric cancer cell MKN45 (JCRB, Cat No.: JCRB0254) expressing c-Met was used for evaluation.

MKN45 cells were resuspended to 10000,000 cells/mL in RPMI 1640 medium (GIBCO, Cat No.: 11835-030), which contains 10% (v/v) FBS (GIBCO, Cat No.: 10099-141) and penicillin/streptomycin (GIBCO, Cat No.: 15070-063). 2mL resuspended MKN45 cells were added to 96-well microtiter plate with 250,000 cells/well, and 10μg/mL of c-Met antibody was added to the corresponding wells and the final volume is 100μL, the plate was incubated at 4°C for 1 hour. FACS buffer (phosphate buffer solution including 2.5% fetal bovine serum; Hyclone, Cat: SH30256.01B) was added and centrifuged at 4°C, 1300rpm for 4 minutes. The supernatant was discarded and this procedure was repeated three times. 100μL secondary antibody solution (Fluorescence labeled goat anti mouse secondary antibodies at 1:200 dilution, Biolegend, Cat#405307; Fluorescence labeled anti-human secondary antibody at 1:30 dilution, Biolegend, Cat#409304) was added into each well. The plate was incubated at 4°C for 1 hour. FACS buffer was added and centrifuged at 4°C, 1300rpm for 4 minutes. The supernatant was discarded and this procedure was repeated three times. Complete cell culture medium (RPMI 1640 medium with 10% FBS) was added and was incubated at 37°C in 5% $CO_2$ for 0, 0.5, 1, 2, 4 hours. 5μL 7-AAD (Biolegend, Cat:420403) was added to 100μL FACS buffer each well, and was incubated at 4°C for 30 minutes. FACS buffer was added and was centrifuged at 4°C, 1300rpm for 4 minutes. The supernatant was discarded and this procedure was repeated three times. 200μL Stripping buffer (0.05 M glycine, pH 3.0; 0.1 M NaCl, mixed at 1:1 (v/v)) was added to each well. The cells were resuspended and were incubated for 7 minutes at room temperature. The cells were centrifuged at room temperature at 1300 rpm for 4 minutes, and the supernatant was discarded. 200μL neutralizing wash buffer (0.15M trihydroxymethyl aminomethane, pH 7.4) was added to each well, The cells were resuspended and were centrifuged at room temperature at 1300rpm for 4 minutes, and the supernatant was discarded. 200μL FACS buffer was added and cells were resuspended. The prepared aamples were detected by flow cytometry (BD FACS Calibur). The results are shown in table below.

$$\text{Endocytosis of c-Met antibody \%} = (\text{intensity of fluorescence at each time point} - \text{mean intensity of fluorescence at time 0})/\text{mean intensity of fluorescence at time 0}.$$

Experimental results:

Table 6. Evaluation of endocytosis of humanized anti c-Met antibody of the present invention (endocytosis%)

| Humanized antibody | 0h | 0.5h | 1h | 2h | 4h |
|---|---|---|---|---|---|
| hIgG (control)* | 0 | -0.9 | -4.4 | -4.9 | 3.6 |
| Ab-9 | 0 | 26 | 32 | 32 | 31 |
| Ab-10 | 0 | 24 | 38 | 53 | 59 |
| * -4.9% and -3.6% in control group are due to experimental error (background value), and it is considered as no endocytosis. | | | | | |

Experimental conclusion:

The experimental results in the table above show that the antibody of the invention has good endocytosis while it does not have agonist activity. Once bound with target cells, both antibodies and receptors would be rapidly internalized into target cells, and maximum value was reached within 2-4 hours.

**Example 8. Analysis of biophysical stability of anti c-Met antibody**

To evaluate the biophysical stability of the antibody of the invention, such as the presence of glycosylation and deamidization sites and stability, LC-MS analysis was used to evaluate the anti c-Met antibody of the invention.

Molecular weight of heavy and light chain was directly detected by LC-MS for the analysis of glycosylation. Deamidization was analyzed by LC-MS at 4°C for long time (at least 3 months), or at 40°C for 21 days under accelerated condition. Samples treated with different conditions were taken and diluted to 2mg/mL with pH7.2 Tris-HCl; final concentration of 10mM of TCEP and 6M of urea (AMRESCO, Cat# $0378)_3$ were added, then the samples were incubated for 20 minutes at 37°C. IAA(Sigma-Aldrich, Cat#I1149) with a final concentration of 20mM was added and was incubated for 15 minutes in darkness to protect sulfhydryl group. pH of sample was adjusted by dilution with Tris-HCl, pH 7.2, and protease (Sigma-Aldrich, Cat# T6567) was added at a ratio by weight of 10:1 (protein: enzyme). The samples were incubated at 37°C for 25 minutes, and then formic acid with a final concentration of 0.1% (Fluca, Cat#94318) was added to terminate the reactions. Samples were centrifuged and analyzed by LC-MS.

BiopharmaLynx was used to analyze the presence of deamidization. Extracted Ion Chromatogram (EIC) was obtained from MS data by searching native peptide comprising deamidization site and modified product, and then extracting parent ion. Peak area was obtained by integration, and the percentages of deamidization and oxidation product were calculated.

Experimental results:

Table 7. Evaluation of physical stability of the humanized anti c-Met antibody of the invention

| The antibody of the invention | Molecular weight of light chain* | | Analysis of deamidization# | |
|---|---|---|---|---|
| | Detected value | Estimated value | 4°C, 3.5 months | 40°C, 21 days |
| Ab-9 | 25940 | 23907 | 0.66 | - |
| Ab-10 | 23828 | 23832 | - | 0.3 |
| *: Heavy chains all involve glycosylation, and molecular weight was consistent with the expected value. #: Percentage of deaminated molecules (%). 0.66-1.0% is within the background of detection. -: not tested. | | | | |

Experimental conclusion: The above results show that the antibody of the invention is stable and has good physical properties.

**Example 9. anti-c-Met antibody Ab-10 conjugated to toxin MC-MMAF (No.1)**

The anti-c-Met antibodies of the present invention have inhibitory activity of receptor binding, without agonist activity, show activity of endocytosis into targeted cells as well as physical stability. These properties make the antibodies of the invention particularly suitable for the preparation of ADC drugs when conjugated to toxins for the treatment of c-Met expressing cancer. The coupling process is shown below:

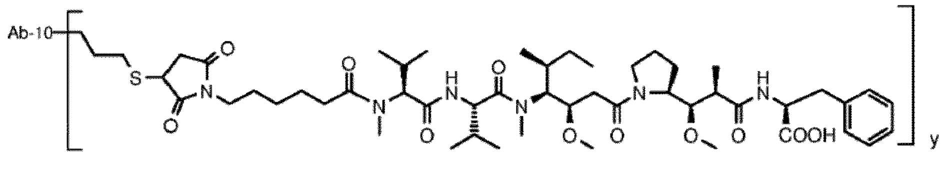

ADC-1

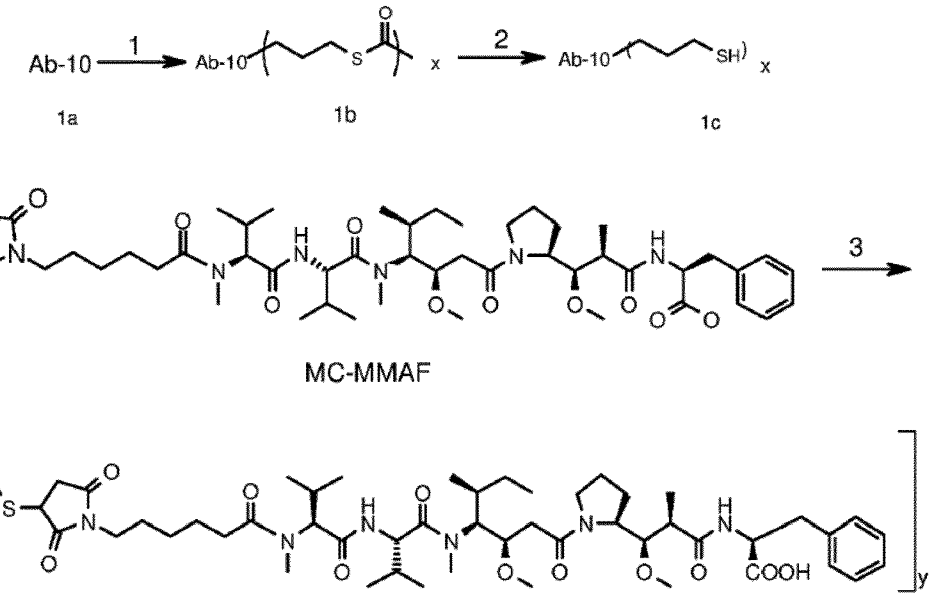


1a 1b 1c

MC-MMAF

ADC-1

Step1. Thioacetic acid S-(3-carbonyl propyl) ester (0.7 mg, 5.3μmol) was dissolved in acetonitrile solution (0.9 mL), for use. The thioacetic acid S-(3-carbonyl propyl) ester in acetonitrile prepared above was added into acetic acid/sodium acetate buffer pH=4.3 (10.35mg/mL, 9.0mL, 0.97mmol) containing Ab-10 monoclonal antibody, and sodium borohydride aqueous solution (14.1mg, 224μmol, 1.0mL) was added dropwise with shaking for 2 hours at 25°C. At the end of reaction, desalination and purification were done on Sephadex G25 gel column (Elution phase: 0.05*M* of PBS solution pH 6.5), product **1b** solution was collected and was concentrated to 10mg/mL directly for the next reaction.

Step 2. hydroxylamine hydrochloride solution (2.0M, 0.35mL) was added into 11.0mL of **1b** solution with shaking for 30 minutes at 25°C, and then desalination and purification were done on Sephadex G25 gel column (Elution phase: 0.05*M* of PBS solution pH 6.5), the captioned product Ab-10 monoclonal antibody-propyl mercaptan **1c** solution was collected (6.17mg/mL, 14.7mL).

Step 3. The compound MC-MMAF (1.1mg, 1.2μmol; prepared by method published in PCT patent WO2005081711) was dissolved in acetonitrile (0.3mL) and was added in Ab-10 monoclonal antibody-propyl mercaptan **1c** solution (6.17mg/mL, 3.0mL) with shaking for 4 hours at 25°C, and then desalination and purification were done on Sephadex G25 gel column (Elution phase: 0.05M of PBS solution pH 6.5). The captioned product ADC-**1** (3.7mg/mL, 4.7mL) in PBS buffer was obtained by filtration through 0.2μm filter under aseptic condition, and then frozen stored at 4°C.

Q-TOF LC/MS: characteristic peak: 148119.2 ($M_{Ab}$+0D), 149278.1 ($M_{Ab}$+1D), 150308.1 ($M_{Ab}$+2D), 151314.1 ($M_{Ab}$+3D). The amount of the conjugated toxin per antibody (DAR) was calculated by analysis and the mean value is y=1.7.

**Example 10. Anti-c-Met antibody Ab-10 conjugated toxin MC-VC-PAB-MMAE (No.2)**

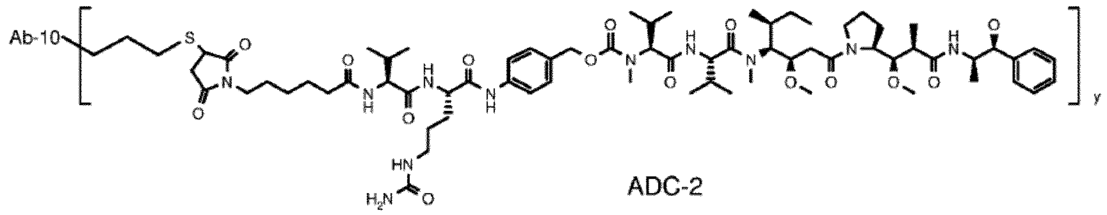

ADC-2

The compound MC-VC-PAB-MMAE (1.6mg, 1.2μmol; prepared as method disclosed in PCT patent WO2004010957) was dissolved in acetonitrile (0.3mL) and was added into Ab-10 monoclonal antibody-propyl mercaptan **1c** solution (6.17mg/mL, 3.0mL) with shaking for 4 hours at 25°C, and then desalination and purification were done on Sephadex G25 gel column (Elution phase: 0.05*M* of PBS solution, pH 6.5). The captioned product ADC-**2** in PBS buffer (3.6mg/mL, 4.8mL) was obtained by filtration through 0.2μm filter under aseptic condition, and then frozen stored at 4°C.

Q-TOF LC/MS: characteristic peak: 148118.4 ($M_{Ab}$+0D), 149509.2 ($M_{Ab}$+1D), 150903.1 ($M_{Ab}$+2D), 152290.4 ($M_{Ab}$+3D), 153680.7 ($M_{Ab}$+4D). The amount of the conjugated toxin per antibody (DAR) was calculated by analysis and the mean value is y=1.8.

**Example 11. Anti-c-Met antibody Ab-10 conjugated toxin MC-VC-PAB-MMAF (No.3)**

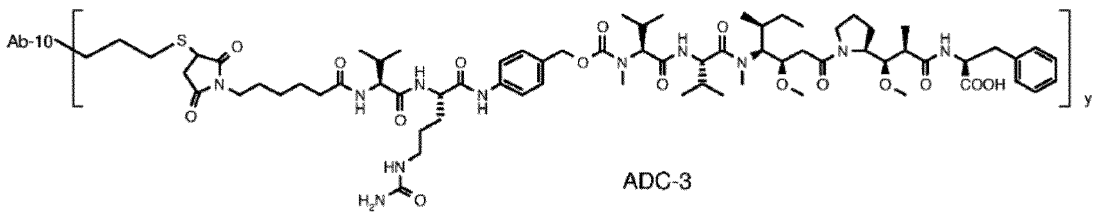

ADC-3

The compound MC-VC-PAB-MMAF (1.6mg, 1.2μmol; prepared as method disclosed in PCT patent WO2005081711) was dissolved in acetonitrile (0.3mL) and was added in Ab-10 monoclonal antibody-propyl mercaptan **1c** solution (6.17mg/mL, 3.0mL) with shaking for 4 hours at 25°C, and then desalination and purification were done on Sephadex G25 gel column (Elution phase: 0.05*M* of PBS solution which pH is 6.5). The captioned product ADC-**3** (3.5mg/mL, 4.9mL) in PBS buffer was obtained by filtration through 0.2μm filter under aseptic condition, and then frozen stored at 4°C.

Q-TOF LC/MS: characteristic peak: 148119.1 ($M_{Ab}$+0D), 149525.3 ($M_{Ab}$+1D), 150930.7 ($M_{Ab}$+2D), 152335.2 ($M_{Ab}$+3D), 153739.8 ($M_{Ab}$+4D). The amount of the conjugated toxin per antibody (DAR) was calculated by analysis and the mean value is y=1.6.

**Example 12. Anti-c-Met antibody Ab-10 conjugated toxin MC-MMAE (No.4)**

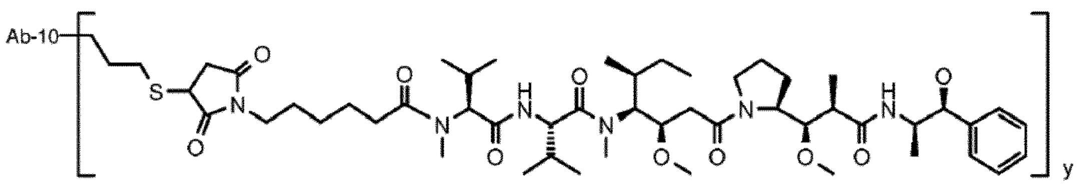

ADC-4

The compound MC-MMAE (1.2mg, 1.2μmol; prepared as method disclosed in patent application US7/750/116B1) was dissolved in acetonitrile (0.3mL) and was added in Ab-10 monoclonal antibody-propyl mercaptan 1c solution (6.17mg/mL, 3.0mL) with shaking for 4 hours at 25°C, and then desalination and purification were done on Sephadex G25 gel column (Elution phase: 0.05*M* of PBS solution which pH is 6.5). The captioned product ADC-**4** in PBS buffer (3.4mg/mL, 5.0 mL) was obtained by filtration through 0.2μm filter under aseptic condition, and then frozen stored at 4°C.

Q-TOF LC/MS: characteristic peak: 148118.6 ($M_{Ab}$+0D), 149104.3 ($M_{Ab}$+1D), 150090.1 ($M_{Ab}$+2D), 151075.8 ($M_{Ab}$+3D). The amount of the conjugated toxin per antibody (DAR) was calculated by analysis and the mean value is y=1.6.

**Example 13. Anti-c-Met antibody Ab-9 conjugated toxin MC-MMAE (No.5)**

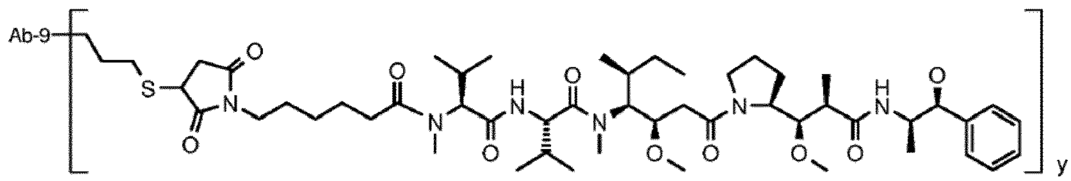

ADC-5

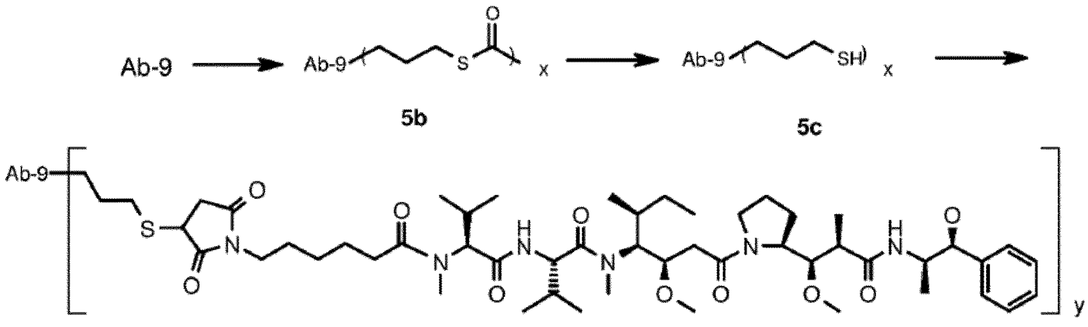

5b

5c

ADC-5

Step1. Thioacetic acid S-(3-carbonyl propyl) ester (0.7 mg, 5.3μmol) was dissolved in 0.9mL acetonitrile solution, for use. The thioacetic acid S-(3-carbonyl propyl) ester in acetonitrile prepared above was added into acetic acid/sodium acetate buffer containing Ab-9 monoclonal antibody (10.85mg/mL, 9.0mL, 0.976mmol), and sodium borohydride aqueous solution (14.1mg, 224μmol, 1.0mL) was added dropwise with shaking for 2 hours at 25°C. At the end of reaction, desalination and purification were done on Sephadex G25 gel column (Elution phase: 0.05*M* of PBS solution which pH is 6.5), and the captioned product **5b** solution was collected and concentrated to 10mg/mL directly for the next reaction.

Step 2. hydroxylamine hydrochloride solution (2.0M, 0.35mL) was added into 5b solution (11.0mL) with shaking for 30 minutes at 25°C, and then desalination and purification were done on Sephadex G25 gel column (Elution phase: 0.05*M* of PBS solution which pH is 6.5), the captioned product Ab-9 monoclonal antibody-propyl mercaptan **5c** solution was collected (6.2mg/mL, 15.0mL).

Step3. the compound MC-MMAE (1.1mg, 1.2μmol) was dissolved in acetonitrile (0.3mL) and was added in Ab-9 monoclonal antibody-propyl mercaptan **5c** solution (6.2mg/mL, 3.0mL) with shaking for 4 hours at 25°C, and then desalination and purification were done on Sephadex G25 gel column (Elution phase: 0.05*M* of PBS solution which pH is 6.5). The captioned product ADC-**5** in PBS buffer (3.8 mg/mL, 4.6 mL) was obtained by filtration through 0.2μm filter under aseptic condition, and then frozen stored at 4°C.

Q-TOF LC/MS: characteristic peak: 150530.9 ($M_{Ab}$+0D), 151915.7 ($M_{Ab}$+1D), 153333.6 ($M_{Ab}$+2D), 154763.4 ($M_{Ab}$+3D), 156271.9 ($M_{Ab}$+4D). The amount of conjugated toxin per antibody (DAR) was calculated by analysis and the mean value is y=1.5.

**Example 14. Anti-c-Met antibody Ab-9 conjugated toxin MC-MMAF (No.6)**

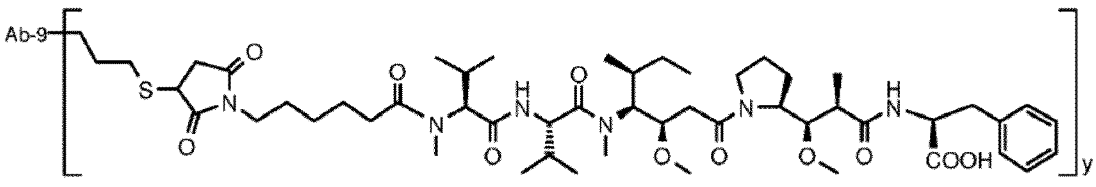

ADC-6

The compound MC-MMAF (1.1mg, 1.2μmol) was dissolved in acetonitrile (0.3mL) and was added in Ab-9 monoclonal antibody-propyl mercaptan **5c** solution (6.17mg/mL, 3.0mL) with shaking for 4 hours at 25°C, and then desalination and purification were done on Sephadex G25 gel column (Elution phase: 0.05*M* of PBS solution which pH is 6.5). The captioned product ADC-**6** in PBS buffer (3.8mg/mL, 4.6mL) was obtained by filtration through 0.2μm filter under aseptic condition, and then frozen stored at 4°C.

Q-TOF LC/MS: characteristic peak: 150537.8 ($M_{Ab}$+0D), 152087.9 ($M_{Ab}$+1D), 153486.5 ($M_{Ab}$+2D), 154911.7 ($M_{Ab}$+3D), 156499.9 ($M_{Ab}$+4D). The amount of conjugated toxin per antibody (DAR) was calculated by analysis and the mean value is y=1.7.

**Example 15. Anti-c-Met antibody Ab-9 conjugated toxin MC-VC-PAB-MMAF (No.7)**

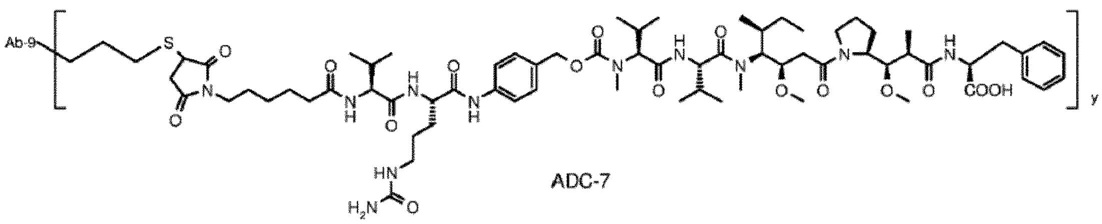

ADC-7

The compound MC-VC-PAB-MMAF (1.6mg, 1.2μmol) was dissolved in acetonitrile (0.3mL) and was added in Ab-9 monoclonal antibody-propyl mercaptan **5c** solution (6.2mg/mL, 3.0mL) with shaking for 4 hours at 25°C, and then desalination and purification were done on Sephadex G25 gel column (Elution phase: 0.05*M* of PBS solution which pH is 6.5). The captioned product ADC-**7** in PBS buffer (3.8mg/mL, 4.6mL) was obtained by filtration through 0.2μm filter under aseptic condition, and then stored at 4°C.

Q-TOF LC/MS: characteristic peak: 150537.8 ($M_{Ab}$+0D), 152087.9 ($M_{Ab}$+1D), 153486.5 ($M_{Ab}$+2D), 154911.7 ($M_{Ab}$+3D), 156499.9 ($M_{Ab}$+4D). The amount of conjugated toxin per antibody (DAR) was gained by analysis and the mean value is y=1.8.

**Example 16. Anti-c-Met antibody Ab-9 conjugated toxin MC-VC-PAB-MMAE (No.8)**

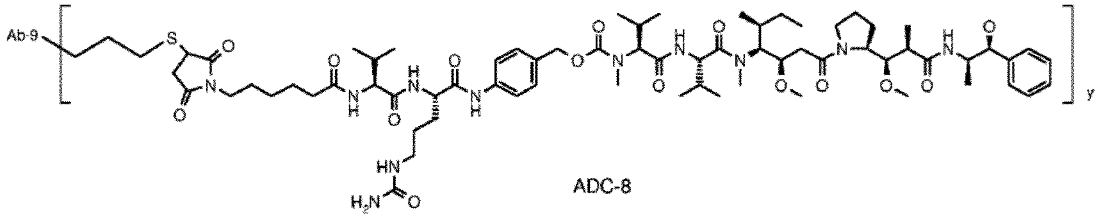

ADC-8

The compound MC-VC-PAB-MMAE (1.6mg, 1.2μmol) was dissolved in acetonitrile (0.3mL) and was added in Ab-9 monoclonal antibody-propyl mercaptan **5c** solution (6.2mg/mL, 3.0mL) with shaking for 4 hours at 25°C, and then desalination and purification were done on Sephadex G25 gel column (Elution phase: 0.05*M* of PBS solution which pH is 6.5). The captioned product ADC-**8** in PBS buffer (3.8mg/mL, 4.6mL) was obtained by filtration through 0.2μm filter under aseptic condition, and then frozen stored at 4°C.

Q-TOF LC/MS: characteristic peak: 150508.6 ($M_{Ab}$+0D), 151903.6 ($M_{Ab}$+1D), 153314.5 ($M_{Ab}$+2D), 154747.8 ($M_{Ab}$+3D), 156039.5 ($M_{Ab}$+4D). The amount of conjugated toxin per antibody (DAR) was gained by analysis and the mean value is y=1.6.

**Example 17. Anti-c-Met antibody Ab-9 conjugated toxin SN-38 (No.9)**

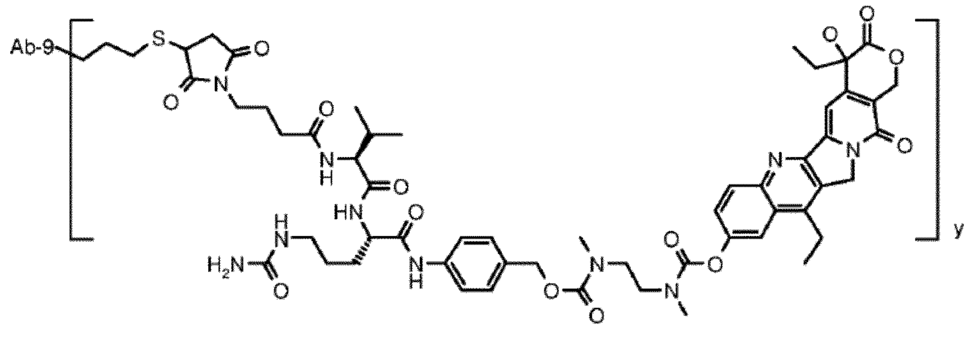

ADC-11

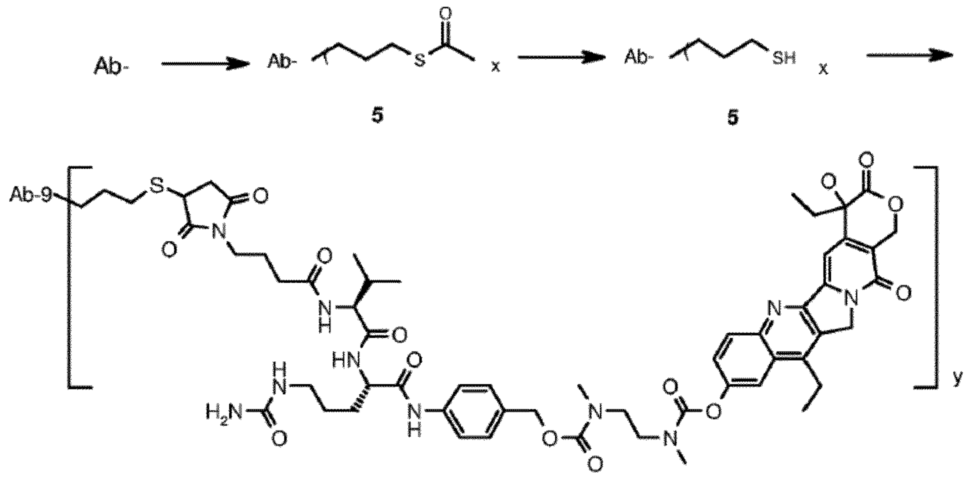

5

5

ADC-11

The compound MC-VC-PAB-SN-38 (1.3mg, 1.2μmol) was dissolved in acetonitrile (0.3mL) and was added in Ab-9 monoclonal antibody-propyl mercaptan **5c** solution (6.2mg/mL, 3.0mL) with shaking for 4 hours at 25°C, and then desalination and purification were done on Sephadex G25 gel column (Elution phase: 0.05*M* of PBS solution which pH is 6.5). The captioned product ADC-**11** in PBS buffer (3.7mg/mL, 4.5mL) was obtained by filtration through 0.2μm filter under aseptic condition, and then stored at 4°C.

Q-TOF LC/MS: characteristic peak: 150537.1 ($M_{Ab}$+0D), 151786.6 ($M_{Ab}$+1D), 152948.6 ($M_{Ab}$+2D), 154161.7 ($M_{Ab}$+3D), 155365.9 ($M_{Ab}$+4D), 156477.8 ($M_{Ab}$+5D).

The mean value is y=2.6.

**Example 18. Anti-c-Met antibody Ab-10 conjugated toxin (No.10)**

1. Preparation of toxin

(*S*)-2-((2*R*,3*R*)-3-((1*S*,3*S*,5*S*)-2-((3*R*,4*S*,5*S*)-4-((*S*)-*N*,3-dimethyl-2-((*S*)-3-methyl-2-(methyl amino)butyramide)butyramide)-3-methoxy-5-methylheptanoyl)-2-azabicyclo[3.1.0]hexane-3-yl)-3-methoxy-2-methylpropanamide)-3-(2-fluorophenyl) propionic acid

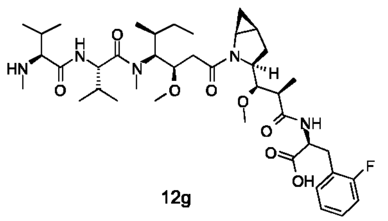

12g

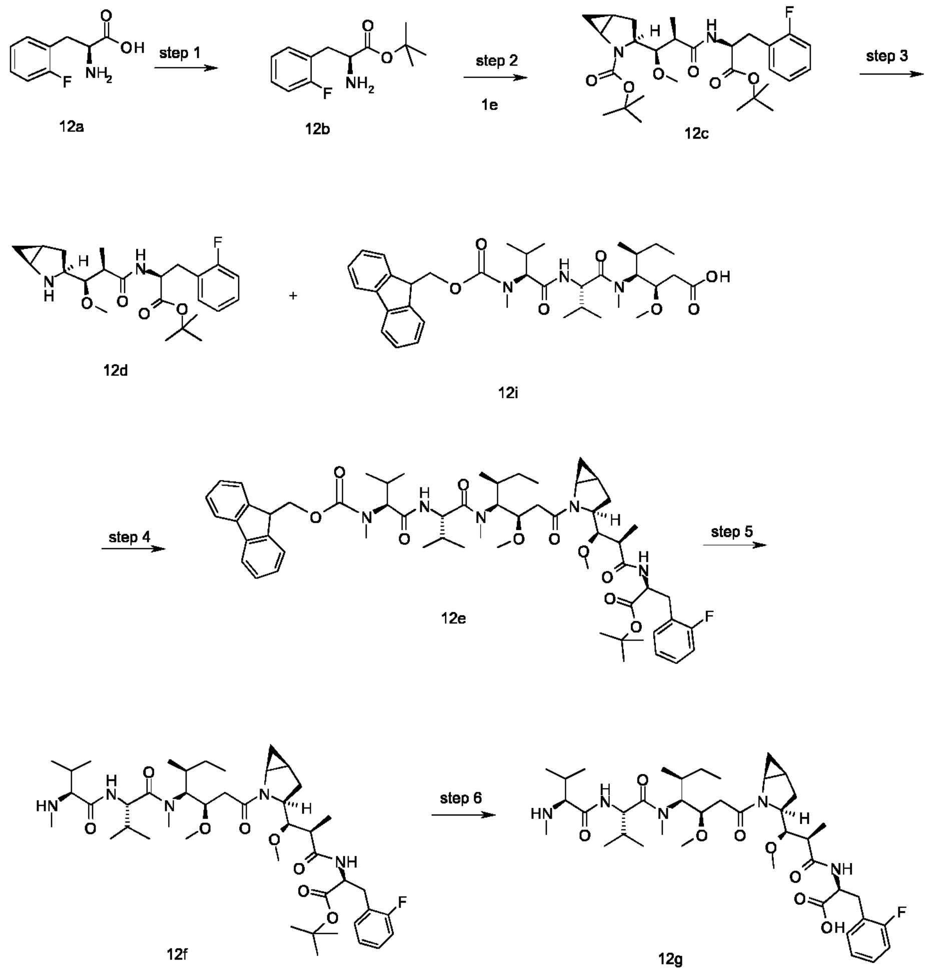


12a 12b 12c 12d 12i 12e 12f 12g

Step 1.

Preparation of (*S*)-tert-butyl-2-amino-3-(2-fluorophenyl) propanoic acid

Starting material (*S*)-2-amino-3-(2-fluorophenyl)propanoic acid **12a** (400 mg, 2.18 mmol, prepared according to the known method in "Advanced Synthesis & Catalysis, 2012, 354(17), 3327-3332") was dissolved in 10mL of tert-butyl acetate. Perchloric acid (300 mg (70%), 3.3 mmol) was added and stirred at room temperature for 16 hours. Water (6mL) was added after reaction, and solution was seprated. The organic phase was washed with saturated sodium bicarbonate solution (5mL). The aqueous phase was adjusted to pH=8 with saturated sodium bicarbonate solution, and then was extracted with dichloromethane (5mL×3), and the organic phase was combined. The reaction mixture was then washed successively with water (3mL) and saturated sodium chloride solution (5mL), dried with anhydrous sodium sulfate, filtered; the filtrate was concentrated under reduced pressure. The crude product compound **12b** was obtained (390mg, yellow, oily) which was subjected to the next reaction directly without purification.

Step 2.

Preparation of (1S,3S,5S)-tert-butyl 3-((1R,2R)-3(((S)-1-(t-butoxy)-3-(2-fluorophenyl)-1-carbonylpropyl-2-yl)amino)-1 -methoxy-2-methyl-3 -carbonyl propyl) -2-azabicyclo [3.1.0] hexane-2-carboxylic acid

The starting material (2R,3R)-3-((1S,3S,5S)-2-(tert-butoxycarbonyl)- 2-azabicyclo [3.1.0] hexane-3-yl)-3-methoxy-2-methyl propionate **12e** (100mg, 0.334mmol) was dissolved in the mixture of dichloromethane (6mL) and dimethylformamide (V/V=5:1), and then crude product (S)-tert-butyl 2-amino-3-(2-fluorophenyl) propionate **12b** (80mg, 0.334mmol) was added. *N,N*-diisopropylethylamine (0.29mL, 1.67mmol) and 2-(7-azabenzotriazol)-N,N,N',N'-tetramethyluronium hexafluorophosphate (152.3mg, 0.40mmol) were added into that mixture. The mixture was stirred for 1 hour under argon atmosphere at room temperature. After reaction, water (10mL) was added and stirred, layers were separated. Layer of dichloromethane was washed by saturated sodium chloride solution (10 mL), and dried with anhydrous sodium sulfate, filtered and the filtrate was concentrated under reduced pressure. The residues were purified by silica gel column chromatography using eluent system B to obtain the captioned product compound **12c** (173 mg, clear liquid, the yield is 99.5%).
MS m/z (ESI): 521.2 [M+1]

Step 3.

Preparation of *(S)*-tert-butyl-2-((2*R*,3*R*)-3-((*1S,*3S,5*S*),-2-azabicyclo[3.1.0] hexane-3-yl)-3-methoxy-2-methylpropionamide)-3-(2-fluorophenyl) propionic acid

*The* starting material *(1S,3S,5S*)-tert-butyl-3-((*1R,2R*)-3-(((S)-1-(t-butoxy)-3 (2-fluorophenyl)-1-carbonylpropyl-2-yl)amino)-1-methoxy-2-methyl-3-carbonyl propyl)-2-azabicyclo [3.1.0] hexane-2-carboxylic acid **12c** (173mg, 0.33mmol) was dissolved in dioxane (2mL), and hydrogen chloride dioxane solution (5.6M, 0.21mL, 1.16mmol) was added. The mixture was stirred for 1 hour under argon atmosphere at room temperature, and was placed in 0°C refrigerator for 12 hours. After reaction, the reaction mixture was concentrated under reduced pressure, and dichloromethane (5mL) was added to dilute the reaction mixture. Saturated sodium bicarbonate solution (10mL) was added and the mixture was stirred for 10 minutes. The product was layered and the aqueous phase was extracted by dichloromethane (5mL×3). Dichloromethane layers were combined and were washed by saturated sodium chloride solution (10mL), dried with anhydrous sodium sulfate, filtered. The filtrate was concentrated under reduced pressure. The crude product of the captioned compound **12d** (77mg, yellow liquid) was obtained and directly subjected to the next reaction without purification.
MS m/z (ESI):421.2 [M+1]

Step 4.

Preparation of (S)-tert-butyl-2-((*2R*,*3R*)-3-((*1S,3S,5S*)-2-(*5S,8S,11S,12R)* -11-((S)-sec-butly)-1-(9H-fluorene-9-yl)-5,8-diisopropyl-12-methoxy-4,10-dimethyl-3,6,9-tricarbonyl-2-oxygen-4,7,10-triazatetradecyl-14-acyl)-2-azabicyclo[3.1.0]hexane-3-yl)-3-methoxy-2-methylpropionamide)-3-(2-fluorophenyl) propionic acid

Crude product *(S)*-tert-butyl-2-((*2R,3R*)-3-((*1S,2S,5S*)-2-azabicyclo[3.1.0] hexane-3-yl)-3-methoxy-2-methylpropionamide)-3-(2-fluorophenyl) propionic acid **12d** (77mg, 0.183mmol) and (5S,8S,11S,12R)-11-((S)-sec-butyl)-1-(9H-fluorene-9-yl) -5,8-diisopropyl-12-methoxy-4,10-dimethyl-3,6,9-tricarbonyl-2-oxo-4,7,10-triazatetradecyl-14-carboxylic acid **12i** (116.8mg, 0.183mmol, prepared by methods published in patent application "WO 2013072813") were dissolved in mixture of dichloromethane (6mL) and dimethylformamide (V/V=5:1). *N,N*-diisopropylethylamine (0.16mL, 0.915mmol) and 2-(7-azabenzotriazol)- *N,N,N',N'*-tetramethyluronium hexafluorophosphate (84mg, 0.22 mmol) were added into that mixture. The reaction mixture was stirred for 1 hour under argon atmosphere at room temperature. After reaction, water (10mL) was added and stirred, layered. Layer of dichloromethane was washed by saturated sodium chloride solution (10 mL), and dried with anhydrous sodium sulfate, filtered and the filtrate was concentrated under reduced pressure. The residues were purified by silica gel column chromatography using eluent system B to obtain the captioned product compound **12e** (190.5mg, yellow viscous) with yield of 100%.
MS m/z (ESI): 1040.6 [M+1]

Step5.

Preparation of *(S)*-tert-butyl-2-((*2R,3R*)-3-((*1S,3S,5S*)-2-((*3R,4S,5S*)-4-((*S*)-N,3-dimethyl-2-((*S*)-3-methyl-2-(methylamino)butanamide)butanamide)-3-methoxy-5-methylheptanoyl)-2-azabicyclo[3.1.0]hexane-3-yl)-3-methoxy-2-methylpropanamide)-3-(2-fluorophenyl) propionic acid

The starting material (*S*)-tert-butyl-2-(*(2R,3R*)-3-((*1S,3S,5S*)-2-((*5S,8S,11S,12R)*-11-((*S*)-sec-butly)-1 -(9H-fluorene-9-yl)-5,8-diisopropyl-12-methoxy-4,10-dimethyl-3,6,9-tricarbonyl-2-oxo-4,7,10-triazatetradecyl-14-acyl)-2-azabicyclo[3.1.0]hexane-3-yl)-3-methoxy-2-methyl propionamide)-3-(2-fluorophenyl) propionic acid **12e** (190.5mg, 0.183mmol) was dissolved in dichloromethane (1.5mL) and diethylamine (2 mL) was added. The mixture was stirred for 3 hours under argon atmosphere at room temperature. After reaction, the reaction mixture was concentrated under reduced pressure and the crude captioned product compound **12f** (150mg, yellow viscous) was obtained. Products were directly subjected to the next reaction without purification.
MS m/z (ESI): 818.5 [M+1]

Step6.

(*S*)-2-((2*R*,3*R*)-3-((1*S*,3*S*,5*S*)-2-((3*R*,4*S*,5*S*)-4-((*S*)-*N*,3-dimethyl-2-((*S*)-3-methyl-2-(methylamino)butanamide)butanamide)-3-methoxy-5-methylheptanoyl)-2-azabicyclo[3.1.0]hexane-3-yl)-3-methoxy-2-methylpropanamide)-3-(2-fluorophenyl)propionic acid

The crude product compound (S)-tert-butyl-2-((2R,3R)-3-((1S,3S,5S)-2-((3R,4S,5S)-4-((S)-N,3-dimethyl-2-((S)-3-methyl-2-(methylamino)butanamide)butanamide)-3-methoxy-5-methylheptanoyl)-2-azabicyclo[3.1.0]hexane-3-yl)-3-methoxy-2-methylpropanamide)-3-(2-fluoro phenyl)propionic acid **12f** (150mg, 0.183mmol) was dissolved in dioxane (1mL), hydrogen chloride in dioxane (5.6M, 3mL) was added. The mixture was stirred for 12 hours under argon atmosphere at room temperature. After reaction, the reaction solution was concentrated under reduced pressure with ether solvent. The residues were purified by high performance liquid chromatography to obtain the captioned product compound **12g** (28mg, white powder with yield of 20%).
MS m/z (ESI): 762.7[M+1] $^1$H NMR (400 MHz, $CD_3OD$): $\delta$ 7.38-7.18(m, 2H), 7.13-7.01(m, 2H), 4.80-4.67(m, 2H), 4.30-4.15(m, 1H), 4.13-4.01(m, 1H), 3.96-3.83(m, 2H), 3.75-3.60(m, 2H), 3.42-3.11(m, 9H), 3.06-2.95(m, 1H), 2.70-2.58(m, 4H), 2.28-2.01 (m, 4H), 1.88-1.70(m, 3H), 1.57-1.25 (m, 4H), 1.22-0.95(m, 18H), 0.92-0.80(m, 4H), 0.78-0.65(m, 1H).

2. Preparation of toxin intermediates

(*S*)-2-((2*R*,3*R*)-3-((1*S*,3*S*,5*S*)-2-((3*R*,4*S*,5*S*)-4-((*S*)-2-((*S*)-2-(6-(2,5-dicarbonyl-2,5-dihydro-1H-pyrrol-1-yl)-*N*-methyl hexanamide)-3-methyl butanamide)-*N*,3-dimethyl butanamide)-3-methoxy-5-methylheptanoyl)-2-azabicyclo [3.1.0]hexane-3-yl)-3-methoxy-2-methylpropanamide)-3-(2-fluorophenyl)propionic acid

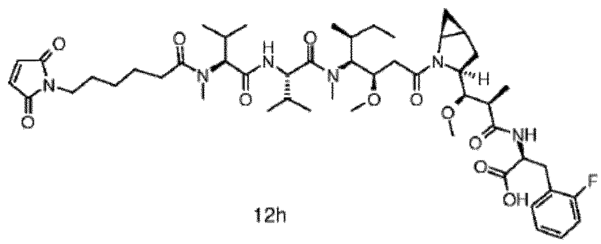

12h

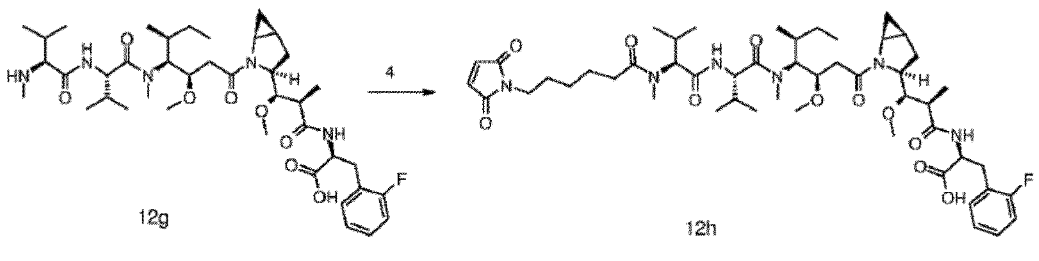


12g

12h

The starting material (*S*)-2-((2*R*,3*R*)-3-((1*S*,3*S*,5*S*)-2-((3*R*,4*S*,5*S*)-((*S*)-*N*,3-dimethyl-2-((*S*)-3-methyl-2-(methylamino)butanamide)butanamide)-3-methoxy-5-methylheptanoyl)-2-azabicyclo[3.1.0]hexane-3-yl)-3-methoxy-2-methylpropanamide)-3-(2-fluorophenyl) propionic acid **12g** (25mg, 0.033mmol) was dissolved in dichloromethane (3mL) and *N,N*-diisopropylethylamine (0.029mL, 0.164mmol) was added. The reaction system was dropwise added with 6-(2,5-dicarbonyl-2,5-dihydro-1*H*-pyrrol-1-yl)hexanoyl chloride **4b** in dichloromethane (11.3mg, 0.049mmol) prepared previously under argon atmosphere, in ice-bath, and the reaction was performed for 3 hours at room temperature. After the reaction, water (5mL) was added and the mixture was stirred for 20 minutes, until layered, and the organic layer was dried with anhydrous sodium sulfate, filtered and the filtrate was concentrated under reduced pressure. The residues were purified by high performance liquid chromatography to obtained the captioned product compound **12h** (7mg, yellow viscous, with the yield of 22.4%).
MS m/z (ESI): 955.4 [M+1]
$^1$H NMR (400 MHz, $CD_3OD$): δ 7.36-7.30(m, 1H), 7.29-7.21(m, 1H), 7.17-7.02(m, 2H), 6.83-6.79(m, 2H), 4.81-4.71(m, 2H), 4.69-4.55 (m, 2H), 4.25-4.15(m, 1H), 4.13-4.04(m, 1H), 3.96-3.85(m, 2H), 3.70-3.61(m, 1H), 3.55-3.46(m, 3H), 3.40-3.21 (m, 4H), 3.18-3.10(m, 2H), 3.07-2.96(m, 4H), 2.67-2.56(m, 2H), 2.54-2.34(m, 3H), 2.29-2.17(m, 2H), 2.10-1.99(m, 1H), 1.89-1.57(m, 7H), 1.52-1.28(m, 6H), 1.21-1.11 (m, 4H), 1.07-0.96(m, 6H), 0.95-0.81(m, 12H), 0.80-0.69(m, 1H).

3. Preparation of antibody-toxin conjugate

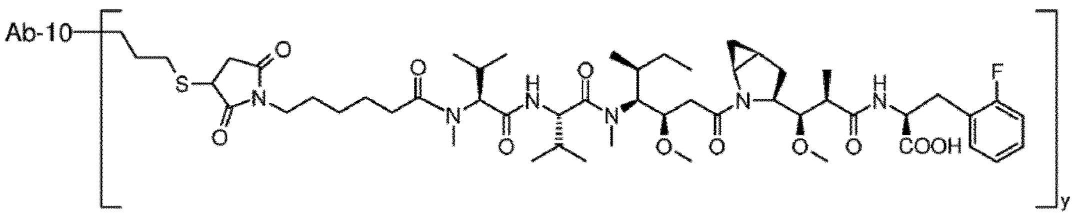

ADC-12

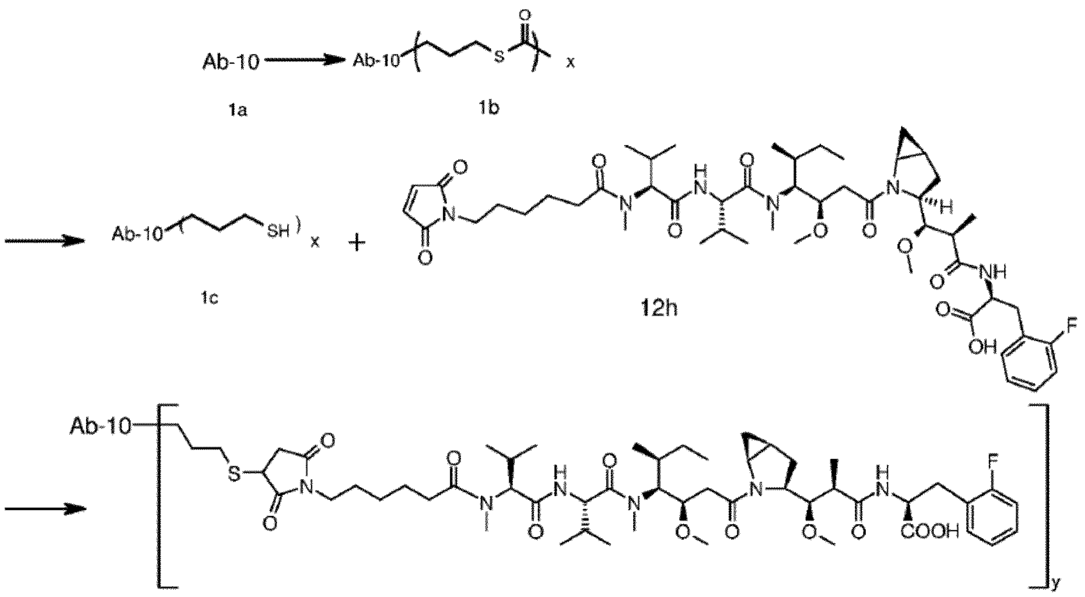

1a

1b

1c

12h

ADC-12

Compound **12h** (1.2mg, 1.2μmol) was dissolved in acetonitrile (0.3mL). Ab-10 monoclonal antibody-propylmercaptan **1c** solution (6.17mg/mL, 3.0mL) was added with shaking for 4 hours at 25°C, and then desalination and purification were done on Sephadex G25 gel column (Elution phase: 0.05*M* of PBS solution which pH is 6.5). The captioned product compound ADC-**12** in PBS buffer (3.3mg/mL, 5.0 mL) was obtained by filtration through 0.2μm filter under aseptic condition, and then frozen stored at 4°C.
Q-TOF LC/MS: characteristic peak: 148119.6 ($M_{Ab}$+0D), 149150.5 ($M_{Ab}$+1D), 150221.1 ($M_{Ab}$+2D), 151265.1 ($M_{Ab}$+3D), 152314.3 ($M_{Ab}$+4D).
Mean value: y=1.6.

With reference to examples 9-18, No. 11-12 ADC compounds were prepared.

| | |
|---|---|
| 11 | 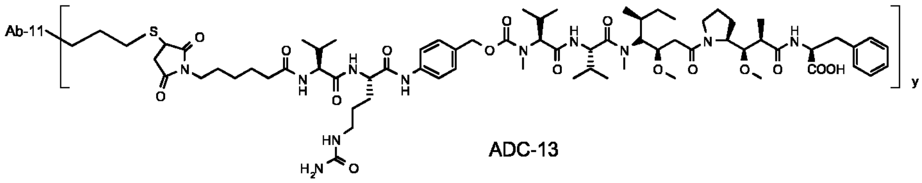 ADC-13 |
| | Anti-c-Met antibody Ab-11 conjugated toxin MC-VC-PAB-MMAF |
| 12 | 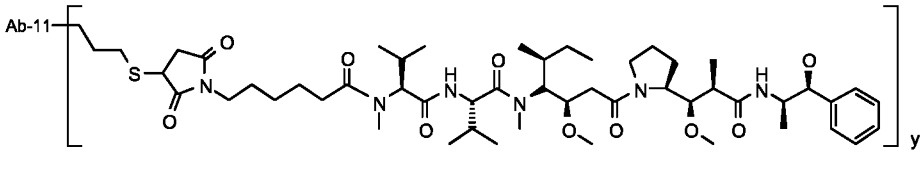 ADC-14 |
| | Anti-c-Met antibody Ab-11 conjugated toxin MC-MMAE |

**Example 19. The inhibitory effect of anti-c-Met antibody toxin conjugate (ADC) molecular on the proliferation of hepatic carcinoma cells**

Test sample: certain antibody compounds of the present invention, the chemical name and preparation method can be found in the preparation example of each compound.

The inhibitory effect of molecules of the present invention on cell proliferation was tested by the CCK method, and the *in vitro* activity of ADC molecules of the present invention was evaluated according to $IC_{50}$.

Cell proliferation was measured using Cell Counting Kit (Dojindo Chemical Technologies, LTD Cat# CK04) (operated according to the instructions). The cells and the corresponding media used are shown in the table below:

| cell line | culture media | Cat. No. |
|---|---|---|
| HepG2 | EMEM+10%FBS | Cell bank, the Chinese academy of science, Cat# TCHu 72 |
| Hep3B | EMEM+10%FBS | Cell bank, the Chinese academy of science, Cat# TCHu106 |
| SK-HEP-1 | EMEM+10%FBS | Cell bank, the Chinese academy of science, Cat# TCHu109 |
| HCCLM3 | DMEM+10%FBS | Jiangsu Howson pharmaceutical co., LTD |
| QGY-7701 | DMEM+10%FBS | Shanghai Bioleaf biotechnology co., LTD |
| SMMC-7721 | DMEM+10%FBS | Shanghai Bioleaf biotechnology co., LTD |
| Bel-7402 | DMEM+10%FBS | Shanghai Bioleaf biotechnology co., LTD |

Experimental procedures:

During the experiment, 2-3 mL trypsin was introduced to perform digestion for 2-3 min. After the cells were completely digested, the digested cells were eluted by adding 10-15 mL of complete medium, centrifuged at 1000 rpm for 3 min, and the supernatant was discarded. Then, the cells were resuspended by adding 10-20 mL medium to prepare single cell suspension, and the cell density was adjusted to $4 \times 10^4$ cells/mL. 0.1 mL of the above cell suspension was added to each well of a 96-well cell culture plate, incubated at 37°C in 5% $CO_2$ incubator, the medium was removed 24 hours later, and 90 μL medium containing 2% FBS was added into each well. The samples to be tested were diluted with PBS to different concentration gradients, added with 10 μL per well, and incubated at 37°C in 5% $CO_2$ incubator for 72 hours. 10 μL of CCK8 was added to each well, continued incubating for another 2 hours in the incubator, and OD450 was detected by microplate reader (VICTOR 3, PerkinElmer), and data analysis was performed using GraphPad Prism (version 5.0) software.

**Experimental results:**

Table 8. The inhibitory effect of the molecule of the invention on proliferation of hepatic carcinoma cells

| cell line | tumor type | ADC-1 | | Ab-10 | |
|---|---|---|---|---|---|
| | | $IC_{50}$(nM) | Maximum Inhibition (%) | $IC_{50}$(nM) | Maximum Inhibition (%) |
| HepG2 | hepatic carcinoma | 81.1 | 25 | 80.90 | 23 |
| Hep3B | hepatic carcinoma | 109.4 | 27 | >1000 | 0 |
| Sk-hep-1 | hepatic carcinoma | 40.8 | 65 | >1000 | 0 |
| QGY-7701 | hepatic carcinoma | 9.1 | 100 | >1000 | 0 |
| SMMC-7721 | hepatic carcinoma | 21.7 | 100 | >1000 | 0 |
| Bel-7402 | hepatic carcinoma | 0.35 | 83 | 0.50 | 34 |
| HCCLM3 | hepatic carcinoma | 1.1 | 85 | 3.20 | 24 |

**Experimental conclusion:**

Based on the above table, the experimental results show that the ADC-1 drug molecule of the present invention has an inhibitory effect on the hepatic carcinoma cell line superior to that of anti-c-Met antibody of the present invention, indicating that the ADC drug of the present invention has a better inhibitory effect on the proliferation of hepatic carcinoma cells.

**Example 20: The efficacy of ADC drug of the present invention on subcutaneous xenografted tumor of human hepatic carcinoma HCCLM3 in nude mice**

Test sample: certain antibodies, ADC compounds of the present invention, the chemical name and preparation method can be found in the preparation example of each compound.

Experimental animals: BALB/cA-nude mice, 21-28 days, male, purchased from Shanghai Institute of Materia Medica, Chinese Academy of Sciences. Production license number: SCXK (Shanghai) 2013-0017, No311613700000089. Feeding environment: SPF level.

Preparation of test solutions:

The ADC-12 was dissolved as 20 mg/mL solution with water for injection, and each aliquot was stored in -80°C refrigerator, and diluted to the corresponding concentrations with 0.1% BSA in saline before use.

Ab-10 antibody stock solution (antibody concentration of 16.3 mg/mL) was diluted with 0.1% BSA in saline, and each aliquot was stored in -80 °C refrigerator.

Experimental procedures:

Nude mice were subcutaneously inoculated with human hepatic carcinoma HCCLM3 cells. Once the tumors were grown to 100-150 $mm^3$, the animals were randomly divided into groups (D0), 10 each group. The tumor volume was measured 2-3 times per week, the mice were weighed, and the data were recorded.

The tumor volume (V) is calculated according to formula:

$$V=1/2\times a\times b^2,$$

wherein a and b represent length and width, respectively;

$$T/C(\%)=(T-T_0)/(C-C_0)\times 100,$$

wherein T and C are tumor volume at the end of the test; $T_0$ and $C_0$ are the tumor volume at the start of the test.

**Experimental results:**

Table 9. Inhibitory test of molecules of the present invention on subcutaneous xenografted tumor of human hepatic carcinoma HCCLM3 in nude mice

| Compound Groups | administration | Mean volume of tumor ($mm^3$) D0 | SEM | Mean volume of tumor ($mm^3$) D21 | SEM | % T/C D21 | tumorinhibition rate D21% | P value (d21) (vs blank) | partial regression | complete regression |
|---|---|---|---|---|---|---|---|---|---|---|
| Solvent | D0 | 125.3 | ±4.0 | 2367.4 | ±193.3 | - | - | - | 0 | 0 |
| ADC-12 (1 mg/kg) | DO | 125.5 | ±3.1 | 1556.6 | ±99.0 | 64 | 36 | 0.002 | 0 | 0 |
| ADC-12 (3 mg/kg) | DO | 123.3 | ±3.8 | 425.8 | ±78.6 | 13 | 87 | **0.000 | 1 | 0 |
| ADC-12 (10 mg/kg) | DO | 121.5 | ±5.7 | 27.2 | ±12.8 | -78 | 178 | **0.000 | 3 | 6 |
| Ab-10 (10 mg/kg) | D0,3,7,1 0,14,17 | 123.9 | ±3.5 | 1565.1 | ±150.5 | 64 | 36 | 0.004 | 0 | 0 |

D0: first administration time; P value, compared with solvent; **P<0.01; all use Student's t test; number of mice at the start of the test: n=10; short line - indicates that the data is empty.

**Experimental conclusion:**

Based on the data in the above table, ADC-12 (1, 3, 10 mg/kg, iv, D0) inhibited the growth of subcutaneous xenografted tumor (c-Met-expressing human hepatic carcinoma HCCLM3) in nude mice in a dose-dependent manner; tumor inhibition rates were 36%, 87%, and 178% (D21) respectively, 1 out of 10 tumors exhibited partial regression in 3 mg/kg dose group, 3 out of 10 tumors exhibited partial regression and 6 out of 10 tumors exhibited complete regression in 10 mg/kg dose group (D21); By 43 days after the first administration (D42), there were still 7 out of 10 tumors showing complete regression in 10 mg/kg dose group. As for Ab-10 antibody stock solution (10 mg/kg, IV, twice per week for 6 times), tumor inhibition rate against HCCLM3 was 36%; tumor-bearing mice were well tolerated to the above drugs, symptoms such as loss of weight were not observed. In comparison, ADC-12 was significantly more effective against HCCLM3 than Ab-10 antibody stock solution.

Therefore, ADC-12 had a significant inhibitory effect on subcutaneous xenograft tumor of c-Met human hepatic carcinoma HCCLM3 in nude mice, effectively inhibited tumor growth, caused partial or complete regression of tumor, and the tumor inhibition rate was increased along with the increase of dosage, inhibitory effect was more significant; Ab-10 antibody stock solution significantly inhibited HCCLM3 and effectively inhibited tumor growth; With the same dosage, ADC-12 was significantly more effective against HCCLM3 than Ab-10 antibody stock solution.

**Example 21. Anti-tumor effect of ADC drugs of the present invention on LI-03-0022 HCC patient-derived tumor transplantation (PDX) model in BALB/c nude mice**

Test sample: certain antibodies, ADC compounds of the present invention, the chemical name and preparation method can be found in the preparation example of each compound.

Experimental animals: BALB/cA-nude mice, 6-8 weeks, 18 females, purchased from Shanghai Sippr-BK Lab Animal Co. Ltd., production license number: SCXK (Shanghai) 2013-0016, animal certificate No.: 2008001658891; Feeding environment: SPF level.

Preparation of test solutions:

The drug ADC-12 was dissolved as 20 mg/mL solution with water for injection, and each aliquot was stored in -80°C refrigerator, and diluted to the corresponding concentrations with 5% glucose solution before use;

Ab-10 antibody stock solution (antibody concentration of 16.3 mg/mL) was diluted with 5% glucose solution, each aliquot was stored in -80 °C refrigerator; diluted to corresponding concentration with 5% glucose solution before use.

The particular preparation method is shown in the table below:

| compound | Packing size | preparation method | concentration (mg/mL) | storage |
|---|---|---|---|---|
| Solvent | | 5% glucose solution | - | 4 °C |
| ADC-12 | 40 mg/vial | 2.0 mL sterile water was injected into vial, and ADC-12 powder was slowly added; the mixture was gently stirred until 20 mg/mL clear stock solution was obtained; the stock solution was divided into 20 aliquots which were stored at -80°C (0.1 mL /vial). | 20.0 | - 80°C |
| ADC-12 injection solution | 20 mg/mL | the stored stock solution (0.1 mL, 20 mg/mL) was diluted with 1.9 mL of 5% glucose solution; as for each time of injection, the solution was freshly prepared before use. | 1.0 | RT |
| Ab-10 antibody solution | 16.3mg/mL, 5mL/vial | Ab-10 antibody solution was evenly divided into several aliquots and stored at -80°C; 0.613mL stored stock solution was diluted with 1.386mL of 5% glucose solution; as for each time of injection, the solution was freshly prepared before use. | 5.0 | RT |
| Ab-10 antibody solution | 16.3mg/mL, 5 mL/vial | Ab-103 antibody solution was evenly divided into several aliquots and stored at -80°C; 0.12 mL stock solution was diluted with 1.836mL of 5% glucose solution; as for each time of injection, the solution was freshly prepared before use. | 1.0 | RT |

**Establishment of LI-03-0022 HCC PDX tumor model:**

LI-03-0022 HCC (hepatic celluler cancer, hepatic carcinoma) PDX tumor model (patient-derived tumor xenograft model, PDX) was originally established on clinical tissue sample surgically resected from patients with hepatic carcinoma (from Shanghai Oriental Hepatobiliary Hospital) and was implanted in nude mice and defined as generation 0 (P0); implantation of tumor generation 0 (P0) was defined as generation 1 (P1); the generation was thus defined according to the order continuously implanted in nude mice, FP3 tumors were recovered from P2T patients, the next generation from FP5 was defined as FP6, and so on; FP5 tumor tissue was used for this study.

Experimental procedure:

(1) Tumor implantation: Each mouse was s.c. implanted with LI-03-0022 FP5 tumor sections (about 30 $mm^3$) on right side to develop tumors, and 32 days after tumor implantation, the mean tumor size was close to 183.20 $mm^3$. Since then, treatment was started with 6 tumor-bearing mice in each group, the experimental procedure for mice was carried out according to the predetermined protocol in the experimental design of the following table:

| Group | N[a] | treatment | dosage mg/kg | administration volume, mL/kg[b] | concentration mg/mL | administration mode | administration time[c] |
|---|---|---|---|---|---|---|---|
| 1 | 6 | solvent | - | 10 | - | iv | BIW × 2 weeks |
| 2 | δ | ADC-12 injection solution | 10 | 10 | 1.0 | iv | BIW × 2 weeks |
| 3 | 6 | Ab-10 | 10 or 50 | 10 | 1.0<br>5.0 | iv | BIW × 2 weeks 1st-2nd dosing, 10 mg/kg; 3rd-4th dosing, 50 mg/kg |
| Note: a, N is the number of animals in each group; b, the administration volume is adjusted based on 10 μL/g body weight; c, BIW is twice a week; iv is intravenously; | | | | | | | |

After 2 hours, the last administration was performed, blood samples were collected from all mice without anticoagulation treatment, about 50 μL of serum was collected for PK analysis; at the end of the study, tumor samples were collected, 2 animals from the solvent group and 2 animals from the sample group. Animals were divided into two groups: one for FFPE (Formalin-Fixed and Parrffin-Embedded tissue was referred as FFPE sample) and IHC (Immunohistochemistry); the other for Frozen in liquid nitrogen.

(2) Observation and recording: The tumor volume was measured 2-3 times per week, the rats were weighed, and the data were recorded.

(3) Tumor measurement and endpoint

The endpoint is mainly dependent on whether the tumor growth is delayed or whether the mouse may be cured. The tumor volume is measured twice a week with caliper in two dimensions (in $mm^3$);

The tumor volume (V) is calculated as:

$$V=0.5\times a\times b^2,$$

wherein a and b represent the long and short diameter of the tumor, respectively;

The tumor volume was used to calculate T-C value, T/C value, T-C value by T (the mean time required for the tumor in treatment group to reach 1000 $mm^3$, in days) and C (the mean time required for the tumor in control group to reach the same size, in days); the T/C value (percentage) is used as an indicator of antitumor efficacy, in particular $T=T_i/T_0$, $C=C_i/C_0$, $T_i$ is the mean tumor volume of the treatment group on certain day, $T_0$ is the mean tumor volume of the treatment group at the beginning of treatment, $C_i$ is the mean tumor volume of the solvent control group at the same time as $T_i$, and $V_0$ is the mean tumor volume of the solvent group at the beginning of treatment.

(4) Data analysis: Summary statistics, including mean and standard error (SEM), volumetric analysis of differences in tumor volume between different groups, and volumetric analysis of drug interactions after the last administration (day

14 after grouping) performed by the data obtained at optimal treatment time point, one-way variance analysis was performed to compare tumour volume and tumor weight between groups; when non-significant F-statistic was obtained (p=0.061, treatment variance vs. error variance), Dunnett's T (double-sided) inter-group comparison was performed; all data were analyzed using SPSS 17.0, $P < 0.05$ was considered statistically significant.

**Experimental results:**

Table 10-1. The change of tumor volume along with time

| Day [b] | tumor volume ($mm^3$)[a] | | |
|---|---|---|---|
| | solvent | ADC-12 injection solution 10 mg/kg | Ab-10 1st-2nd 10 mg/kg 3rd-4th 50 mg/kg |
| 0 | 184 ± 32 | 184 ± 28 | 164 ± 31[c] |
| 3 | 351 ± 62 | 242 ± 37 | 293 ± 52 |
| 7 | 606 ± 98 | 155 ± 29 | 569 ± 85 |
| 10 | 879 ± 193 | 107 ± 18 | 719 ± 108 |
| 14 | 1,605 ± 367 | 52 ± 12 | 1,067 ± 152 |
| 17 | - | 41 ± 11 | 1,725 ± 195 |
| 21 | - | 29 ± 10 | 1,896 ± 283 |
| 24 | - | 16 ± 8 | - |
| 28 | - | 7 ± 3 | - |
| 31 | - | 6 ± 3 | - |
| 34 | - | 4 ± 2 | - |
| 37 | - | 4 ± 2 | - |
| 41 | - | 14 ± 9 | - |
| 42 | | 15 ± 9 | - |

a is mean ± standard error; b is the number of days after beginning the treatment; c is n = 5; short line, "-", means that the animals in the corresponding group have been sacrificed at this time, no data were obtained.

Table 10-2. Analysis of the inhibitory effect of molecules of the present invention on tumor growth of human hepatic carcinoma subcutaneous xenograft tumor in BALB/c nude mice

| Sample | Day 14 | | | | Day 21 | |
|---|---|---|---|---|---|---|
| | tumor size ($mm^3$)[a] | T/C[b] (%) | T-C (day) at 1000 $mm^3$ | *p* value[c] | tumor size ($mm^3$)[a] | *p* value[c] |
| solvent | 1,605 ± 367 | - | - | - | - | - |
| ADC-12 injection solution (10 mg/kg) | 52 ± 12 | 3.25 | >32 | 0.001 | 29 ± 10 | - |
| Ab-10 (1st-2nd, 10 mg/kg; 3rd-4th, 50mg/kg)[d] | 1,067 ± 152 | 74.47 | 3 | 0.252 | 1,896 ± 283 | <0.001 |

a is mean ± standard error; b is tumor growth inhibition, calculated by dividing the mean tumor volume of the treatment group with the mean tumor volume of the control group, T/C must be less than or equal to 50%; c is the p value calculated on the basis of tumor size; d means 3-5 tumor-bearing animals in each group.

Table 10-3. Immunohistochemistry (IHC) of c-Met staining

| No. | PDX model | IHC score |
|---|---|---|
| 1 | LI-03-0010 (negative control) | 0 |
| 2 | LI-03-0022 | 2+ |

The particular staining area is shown in Figure 2 and Figure 3 of the specification.
Note: 0 means unstained, + means light staining, ++ means medium staining, +++ means dark staining; the entire section is read under microscope, the percentage of different staining intensity in cells is determined by visual evaluation, and the H score is calculated as 1 × (% of +cells) + 2 × (% of ++cells) + 3 × (% of +++cells); then we used the scoring standard to evaluate the figure: the score of 0-0.3 is weakly positive, 0.3-1.5 is moderate positive and 1.5-3 is strongly positive.

**Experimental conclusion:**

The results of the above table demonstrate that the ADC drug of the present invention is significantly stronger than the antibody for inhibiting the proliferation of hepatic carcinoma cells; treatment with ADC-12 injection solution results in significant antitumor activity with an mean tumor volume of 52.3 $mm^3$ (T/C value = 3.25%, p=0.001), when compared with the solvent group; the tumor growth volume is controlled within 1000 $mm^3$ and delayed by 32 days; however, treatment with Ab-10 antibody solution results in only minimal antitumor activity, the mean tumor size is controlled within 1,067 $mm^3$ (T/C value =74.47%) and delayed by 3 days; there is no statistically significant difference when compared with the solvent group (p = 0.252); The LI-03-0022 HCC PDX model has an impression score of 2+ for c-Met protein expression, indicating that the expression level of c-Met protein in the model is strongly positive and can be used for further research *in vivo.* Therefore, the ADC drug of the present invention has significant antitumor activity in LI-03-0022 HCC patient-derived tumor transplantation (PDX) model study, and is well tolerated in tumor-bearing animals.

**Example 22. Anti-tumor effect of ADC drug of the present invention on LI-03-0240 HCC patient-derived tumor transplantation (PDX) model in BALB/c nude mice**

Test sample: certain antibodies, ADC compounds of the present invention, the chemical name and preparation method can be found in the preparation example of each compound.

Experimental animals: BALB/cA-nude mice, 6-8 weeks, 30 females, purchased from Shanghai Sippr-BK Lab Animal Co. Ltd., production license number: SCXK (Shanghai) 2013-0016, animal certificate No.: 2008001658004; Feeding environment: SPF level.

Preparation of test solutions:

The drug ADC-12 lyophilized powder was dissolved as 20 mg/mL solution with water for injection, and each aliquot was stored in -80°C refrigerator, and diluted to corresponding concentrations with 5% glucose solution before use;

Ab-10 antibody stock solution (concentration of 16.3 mg/mL) was diluted with 5% glucose solution, each aliquot was stored in -80°C refrigerator; diluted to corresponding concentrations with 5% glucose solution before use.

The particular preparation method is shown in the table below:

| compound | packing size | preparation method | concentration mg/mL | storage |
|---|---|---|---|---|
| solvent | - | 5% glucose solution | - | RT |
| ADC-12 | 40mg/vial | 2.0 mL sterile water was injected into vial, and ADC-12 lyophilized powder was slowly added; the mixture was gently stirred until 20 mg/mL clear stock solution was obtained; the stock solution was divided into 20 aliquots which were stored at -80°C | 20 | -80 °C |
| ADC-12 injection solution | 20 mg/mL | 0.14 mL ADC-12 was added into vial, and diluted with 2.66 mL 0.5% glucose solution to obtain 1 mg/mL working solution. | 1 | RT |

(continued)

| compound | packing size | preparation method | concentration mg/mL | storage |
|---|---|---|---|---|
| ADC-12 injection solution | 1 mg/mL | 0.2 mL ADC-12 injection solution was added into vial, and diluted with 1.8 mL 0.5% glucose solution to obtain 0.1 mg/mL working solution. The solution was prepared before each administration. | 0.1 | RT |
| ADC-12 injection solution | 1 mg/mL | 0.6 mL ADC-12 injection solution was added into vial, and diluted with 1.4 mL 0.5% glucose solution to obtain 0.3 mg/mL working solution. The solution was prepared before each administration. | 0.3 | RT |
| Ab-10 antibody solution | 16.3mg/mL, 5 mL/vial | Ab-10 antibody solution was evenly divided into several aliquots and stored at -80°C . 0.12 mL Ab-10 antibody solution was diluted with 1.836mL of 5% glucose solution to obtain 1 mg/mL working solution. The solution was prepared before each administration. | 1 | RT |

**Establishment of LI-03-0240 HCC PDX tumor model:**

LI-03-0240 HCC (hepatic celluler cancer, hepatic carcinoma) PDX tumor model (patient-derived tumor xenograft model, PDX) was originally established on clinical tissue sample surgically resected from patients with hepatic carcinoma (from Shanghai Oriental Hepatobiliary Hospital) which was implanted in nude mice and defined as generation 0 (P0); implantation of tumor generation 0 (P0) was defined as generation 1 (P1); the generation was thus defined according to the order continuously implanted in nude mice, FP3 tumors were recovered from P2T patients, the next generation from FP5 was defined as FP6, and so on; FP5 tumor tissue was used for this study.

**Experimental procedure:**

(1) Tumor implantation: Each mouse was s.c. implanted with LI-03-0240 FP5 tumor sections (about 30 $mm^3$) on right side to develop tumors, and 15 days after tumor implantation, the mean tumor size was close to 151.79 $mm^3$. Since then, treatment was started with 6 tumor-bearing mice in each group, the experimental procedure for mice was carried out according to the predetermined protocol in the experimental design of the following table

| group | $N^a$ | treatment | dosage mg/kg | administration volume, $mL/kg^b$ | concentration mg/mL | administration mode | administration $time^c$ |
|---|---|---|---|---|---|---|---|
| 1 | δ | solvent | - | 10 | - | iv | BIW × 2 weeks |
| 2 | 6 | ADC-12 injection solution | 1 | 10 | 0.1 | iv | BIW × 2 weeks |
| 3 | 6 | ADC-12 injection solution | 3 | 10 | 0.3 | iv | BIW × 2 weeks |
| 4 | 6 | ADC-12 injection solution | 10 | 10 | 1.0 | iv | BIW × 2 weeks |
| 5 | 6 | Ab-10 | 10 | 10 | 1.0 | iv | BIW × 2 weeks |
| Note: a, N is the number of animals in each group; b, the dosage volume is adjusted based on 10 μL/g body weight; c, BIW is twice a week; iv is intravenously; | | | | | | | |

After 2 hours, the last administration was performed, blood samples were collected from all mice without anticoagulation treatment, about 50 μL of serum was collected for PK analysis; at the end of the study, tumor samples were collected, 2 animals from the solvent group and 2 animals from the sample group. Animals were divided into two groups: one for FFPE (Formalin-Fixed and Parrffin-Embedded tissue was referred as FFPE sample) and IHC (Immunohistochemistry);

the other for Frozen in liquid nitrogen.

(2) Observation and recording: The tumor volume was measured 2-3 times per week, the rats were weighed, and the data were recorded.

(3) Tumor measurement and endpoint

The endpoint is mainly dependent on whether the tumor growth is delayed or whether the mouse may be cured. The tumor volume is measured twice a week with caliper in two dimensions (in $mm^3$);

The tumor volume (V) is calculated as:

$$V=0.5\times a\times b^2,$$

wherein a and b represent length and width, respectively;

The tumor volume was used to calculate T-C value, T/C value, T-C value by T (the mean time required for the tumor in treatment group to reach 1000 $mm^3$, in days) and C (the mean time required for the tumor in control group to reach the same size, in days); the T/C value (percentage) is used as an indicator of antitumor efficacy, in particular $T=T_i/T_0$, $C=C_i/C_0$, $T_i$ is the mean tumor volume of the treatment group on certain day, $T_0$ is the mean tumor volume of the treatment group at the beginning of treatment, $C_i$ is the mean tumor volume of the solvent control group at the same time as $T_i$, and $V_0$ is the mean tumor volume of the solvent group at the beginning of treatment.

(4) Data analysis: Summary statistics, including mean and standard error (SEM), statistic analysis of differences in tumor volume between different groups, and data analysis of drug interactions after the last administration (day 17 after grouping) performed by the data obtained at optimal treatment time point, one-way variance analysis was performed to compare tumour volume and tumor weight between groups; when non-significant F-statistic was obtained ($p<0.001$, treatment variance vs. error variance), Games-Howell inter-group comparison was performed; all data were analyzed using SPSS 17.0, $P < 0.05$ was considered statistically significant.

**Experimental results:**

Table 11-1. the change of tumor volume along with time

| Day [b] | tumor volume ($mm^3$)[a] | | | | |
|---|---|---|---|---|---|
| | solvent | ADC-12 injection solution 1.0 mg/kg | ADC-12 injection solution 3.0 mg/kg | ADC-12 injection solution 10 mg/kg | Ab-10 10 mg/kg |
| 0 | 151 ± 20 | 152 ± 10 | 151 ± 17 | 152 ± 27 | 152 ± 16 |
| 4 | 268 ± 47 | 189 ± 10 | 121 ± 17 | 129 ± 27 | 259 ± 22 |
| 7 | 425 ± 74 | 140 ± 10 | 60 ± 9 | 63 ± 19 | 384 ± 43 |
| 11 | 677 ± 105 | 96 ± 10 | 41 ± 8 | 43 ± 16 | 574 ± 91 |
| 14 | 972 ± 162 | 76 ± 9 | 31 ± 5 | 31 ± 11 | 815 ± 141 |
| 17 | 1,320 ± 201 | 60 ± 7 | 22 ± 4 | 24 ± 9 | 1,159 ± 221 |
| 20 | - | 53 ± 6 | 20 ± 4 | 20 ± 8 | 1,556 ± 280 |
| 24 | - | 43 ± 7 | 16 ± 4 | 15 ± 6 | - |
| 27 | - | 41 ± 6 | 8 ± 2 | 14 ± 5 | - |
| 31 | - | 50 ± 11 | 3 ± 2 | 9 ± 5 | - |
| 34 | - | 75 ± 28 | 2 ± 1 | 7 ± 4 | - |
| 38 | - | 114 ± 56 | 0 ± 0 | 4 ± 3 | - |
| 41 | - | 177 ± 84 | 0 ± 0 | 2 ± 2 | - |
| 45 | - | 248 ± 130 | 0 ± 0 | 1 ± 1 | - |

a is mean ± standard error; b is the number of days after beginning the treatment; c means n = 5; short line, "-" means that the animals in corresponding group have been sacrificed at this time, no data were obtained.

Table 11-2. Analysis of the inhibitory effect of molecules of the present invention on tumor growth of human hepatic carcinoma subcutaneous xenograft tumor in BALB/c nude mice

| sample | Day 17 | | | | Day 21 | |
|---|---|---|---|---|---|---|
| | tumor size ($mm^3$)[a] | T/C[b] (%) | T-C (day) at 1000 $mm^3$ | *p* value[c] | tumor size ($mm^3$)[a] | *p* value[c] |
| solvent | 1,320 ± 201 | -- | -- | -- | - | - |
| ADC-12 injection solution (1 mg/kg) | 60±7 | 4.51 | >31 | 0.008 | - 53 ± 6 | 0.011 |
| ADC-12 injection solution (3 mg/kg) | 22±4 | 1.66 | >31 | 0.007 | 20 ± 4 | 0.010 |
| ADC-12 injection solution (10 mg/kg) | 24 ± 9 | 1.84 | >31 | 0.007 | 20 ± 8 | 0.010 |
| Ab-10 (10 mg/kg) | 1,159±221 | 87.75 | 1 | 0.980 | 1,556 ± 280 | - |

a is mean ± standard error; b is tumor growth inhibition, calculated by dividing the mean tumor volume of the treatment group by the mean tumor volume of the control group, T/C must be less than or equal to 50%; c is the p value calculated on the basis of tumor size; d means 3-5 tumor-bearing animals in each group.

Table 11-3. Immunohistochemistry (IHC) of c-Met staining

| No. | PDX model | IHC score |
|---|---|---|
| 1 | LI-03-0010 (negative control) | 0 |
| 2 | LI-03-0240 | 3+ |

The particular staining area is shown in Figure 5 and Figure 6 of the specification.
Note: 0 means unstained, + means light staining, ++ means medium staining, +++ means dark staining; the entire section is read under microscope, the percentage of different staining intensity in cells is determined by visual evaluation, and the H score is calculated as 1 × (% of +cells) + 2 × (% of ++cells) + 3 × (% of +++cells); then we used the scoring standard to evaluate the figure: the score of 0-0.3 is weakly positive, 0.3-1.5 is moderate positive and 1.5-3 is strongly positive.

**Experimental conclusion:**

The results of the above table demonstrate that the ADC drug of the present invention is significantly stronger than the antibody for inhibiting the proliferation of hepatic carcinoma cells; treatment with ADC-12 injection solution results in significant antitumor activity with mean tumor volume of 52.3 $mm^3$ (T/C value = 3.25%, p=0.001), when compared with the solvent group; the tumor growth volume is controlled within 1000 $mm^3$ and delayed by 32 days; however, treatment with Ab-10 antibody solution results in only minimal antitumor activity, the mean tumor size is controlled within 1,067 $mm^3$ (T/C value =74.47%) and delayed by 3 days; there is no statistically significant difference when compared with the solvent group (p = 0.252); The LI-03-0240 PDX model has an impression score of 3+ for c-Met protein expression, indicating that the expression level of c-Met protein in the model is strongly positive and can be used for further research *in vivo.* Therefore, the ADC drug of the present invention has significant antitumor activity in LI-03-0240 HCC patient-derived tumor transplantation (PDX) model study, and is well tolerated in tumor-bearing animals.

## Claims

**1.** use of an antibody-cytotoxic drug conjugate or pharmaceutically acceptable salt or solvate thereof in the preparation of a medicament for the treatment of hepatic carcinoma, wherein said antibody-cytotoxic drug conjugate has a structure shown as formula (I):

$$Ab\text{-}[(L_2)t\text{-}L_1\text{-}D)]y \qquad (I)$$

wherein:

D is cytotoxic drug;
$L_1$ and $L_2$ are linker units;
t is 0 or 1, preferably 1;
y is 1-8, preferably 2-5;
Ab is antibody or antigen-binding fragment thereof that specifically binds to c-Met receptor, comprising at least one CDR sequence selected from the following sequences or mutant sequence thereof:
antibody heavy chain variable region HCDR sequence: SEQ ID NO: 6, SEQ ID NO:7 or SEQ ID NO:8; and
antibody light chain variable region LCDR sequence: SEQ ID NO: 9, SEQ ID NO: 10 or SEQ ID NO: 11.

**2.** The use according to claim 1, wherein the antibody heavy chain variable region comprises at least one HCDR sequence selected from the following sequences or mutant sequence thereof: SEQ ID NO: 6, SEQ ID NO: 7 or SEQ ID NO: 8.

**3.** The use according to claim 1, wherein the antibody light chain variable region comprises at least one LCDR sequence selected from the following sequences or mutant sequence thereof: SEQ ID NO: 9, SEQ ID NO: 10 or SEQ ID NO: 11.

**4.** The use according to any one of claims 1-3, wherein the antibody comprises heavy chain variable region sequence of SEQ ID NO: 6, SEQ ID NO: 7 and SEQ ID NO: 8, or mutant sequence thereof; and light chain variable region sequence of SEQ ID NO:9, SEQ ID NO: 10 and SEQ ID NO: 11, or mutant sequence thereof.

**5.** The use according to any one of claims 1-4, wherein the mutant sequence is a sequence which has 1-3 amino acid mutation(s) located in CDR region for optimizing the antibody activity, wherein the mutant sequence of HCDR2 region preferably is SEQ ID NO: 12.

**6.** The use according to any one of claims 1-5, wherein the antibody or antigen-binding fragment thereof that specifically binds to c-Met receptor is murine antibody or fragment thereof.

**7.** The use according to claim 6, wherein the heavy chain variable region sequence of the murine antibody is shown as SEQ ID NO: 4.

**8.** The use according to claim 6, wherein the light chain variable region sequence of the murine antibody is shown as

SEQ ID NO: 5.

**9.** The use according to any one of claims 6 to 8, wherein the heavy chain variable region of the murine antibody is shown as SEQ ID NO: 4, and the light chain variable region of the murine antibody is shown as SEQ ID NO: 5.

**10.** The use according to any one of claims 1-5, wherein the antibody or antigen-binding fragment thereof that specifically binds to c-Met receptor is chimeric antibody or humanized antibody or fragment thereof.

**11.** The use according to claim 10, wherein the humanized antibody heavy chain variable region comprises a heavy chain FR region derived from human germline heavy chain sequence, preferably human germline heavy chain IGHV 3-33*01; wherein said heavy chain FR region comprises framework sequence of FR1, FR2, FR3 and FR4 regions of human germline heavy chain IGHV 3-33*01, or mutant sequence thereof, preferably the mutant sequence comprises 0-10 amino acid back-mutation(s).

**12.** The use according to claim 11, wherein the humanized antibody comprises heavy chain variable region sequence selected from SEQ ID NO: 13-15 or variant thereof.

**13.** The use according to claim 10, wherein the humanized antibody light chain variable region comprises a light chain FR region derived from human germline light chain sequence, preferably human germline light chain IGKV085 or IGKV4-1*01; wherein said light chain FR region comprises framework sequence of FR1, FR2, FR3 and FR4 regions of human germline light chain IGKV085 and IGKV4-1*01, or mutant sequence thereof, preferably the mutant sequence comprises 0-10 amino acid back-mutation(s).

**14.** The use according to claim 13, wherein the humanized antibody comprises light chain variable region sequence selected from SEQ ID NO: 16-18 or variant thereof.

**15.** The use according to any one of claims 10 to 14, wherein the humanized antibody comprises heavy chain variable region sequence selected from SEQ ID NO: 13-15 and light chain variable region sequence selected from SEQ ID NO: 16-18.

**16.** The use according to any one of claims 1-5 and 10-15, wherein said antibody or antigen-binding fragment thereof that specifically binds to c-Met receptor comprises a combination of heavy chain variable region sequence and light chain variable region sequence selected from any one of a) to c):

a) Heavy chain variable region sequence of SEQ ID NO: 13, and light chain variable region sequence of SEQ ID NO: 16;
b) Heavy chain variable region sequence of SEQ ID NO: 14, and light chain variable region sequence of SEQ ID NO: 17; or
c) Heavy chain variable region sequence of SEQ ID NO: 15, and light chain variable region sequence of SEQ ID NO: 18.

**17.** The use according to any one of claims 10-16, wherein the heavy chain constant region of humanized antibody comprises a constant region derived from human IgG1 or variant thereof, human IgG2 or variant thereof, human IgG3 or variant thereof, or human IgG4 or variant thereof, preferably comprises a constant region derived from human IgG1 or variant thereof, human IgG2 or variant thereof or human IgG4 or variant thereof, more preferably a constant region derived from human IgG2 or variant thereof.

**18.** The use according to claim 17, wherein said antibody or antigen-binding fragment thereof that specifically binds to c-Met receptor comprises a full length heavy chain sequence selected from SEQ ID NO: 23-25 or sequences having at least 90% identity to SEQ ID NO: 23-25.

**19.** The use according to any one of claims 10-16, wherein the light chain constant region of humanized antibody comprises a constant region selected from human $\kappa$ or $\lambda$ or variant thereof.

**20.** The use according to claim 19, wherein said antibody or antigen-binding fragment thereof that specifically binds to c-Met receptor comprises a full-length light chain sequence selected from SEQ ID NO: 26-28 or sequences having at least 90% identity to SEQ ID NO: 26-28.

**21.** The use according to any one of claims 10-20, wherein the humanized antibody comprises a combination of full-length light chain sequence and full-length heavy chain selected from:

Ab-9: heavy chain sequence of SEQ ID NO: 23 and light chain sequence of SEQ ID NO: 26;
Ab-10: heavy chain sequence of SEQ ID NO: 24 and light chain sequence of SEQ ID NO: 27; or
Ab-11: heavy chain sequence of SEQ ID NO: 25 and light chain sequence of SEQ ID NO: 28.

**22.** The use of a pharmaceutical composition in the preparation of a medicament for the treatment of hepatic carcinoma, wherein said pharmaceutical composition comprises the antibody or antigen-binding fragment thereof that specifically binds to c-Met receptor defined in any one of claims 1-21, said pharmaceutical composition comprises one or more pharmaceutically acceptable excipient, diluent or carrier.

**23.** The use according to claim 1, wherein said $-L_2-$ is a compound shown as formula ($-L_2-$):

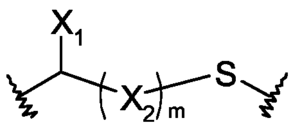

,

wherein:

$X_1$ is selected from the group consisting of hydrogen, halogen, hydroxyl, cyano, $C_{1-6}$ alkyl, $C_{1-6}$ alkoxy or 3-8 membered cycloalkyl;
$X_2$ is selected from the group consisting of $C_{1-6}$ alkyl, 3-8 membered cycloalkyl or 3-8 membered heterocyclyl;
m is 0-5, preferably 1-3;
S is sulfur atom.

**24.** The use according to claim 1, wherein said cytotoxic drug D is a cytotoxic agent selected from toxins, chemotherapeutic agents, antibiotics, radioisotopes or nucleolytic enzyme.

**25.** The use according to claim 24, wherein D is a compound shown as formula (D):

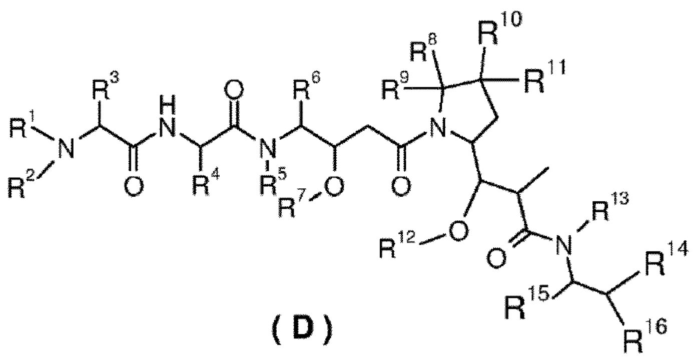

(D)

or a tautomer, a mesomer, a racemate, an enantiomer, a diastereomer, or mixture thereof, or pharmaceutically acceptable salt thereof:
wherein:

$R^1$, $R^2$, $R^3$, $R^4$, $R^5$, $R^6$, $R^7$ is each selected from the group consisting of hydrogen, halogen, hydroxyl, cyano, $C_{1-6}$ alkyl, $C_{1-6}$ alkoxy or 3-8 membered cycloalkyl;
$R^8$, $R^9$, $R^{10}$, $R^{11}$ is each selected from the group consisting of hydrogen, halogen, $C_{2-6}$ alkenyl, $C_{1-6}$ alkyl, $C_{1-6}$ alkoxy or 3-8 membered cycloalkyl;
or, any two of $R^8$, $R^9$, $R^{10}$ and $R^{11}$ form 3-8 membered cycloalkyl, the rest of two are each selected from the group consisting of hydrogen, $C_{1-6}$ alkyl or 3-8 membered cycloalkyl;
$R^{12}$ and $R^{13}$ are each selected from the group consisting of hydrogen, $C_{1-6}$ alkyl or halogen;
$R^{14}$ is selected from 6-8 membered aryl or 5-8 membered heteroaryl, wherein the aryl or heteroaryl is optionally

further substituted by substituent selected from the group consisting of hydrogen, halogen, hydroxy, $C_{1-6}$ alkyl, $C_{1-6}$ alkoxy or 3-8 membered cycloalkyl;

$R^{15}$ is selected from halogen, $C_{2-6}$ alkenyl, $C_{1-6}$ alkyl, 3-8 membered cycloalkyl, carboxyl, $C_{1-6}$ alkyl carbonyl or $C_{1-6}$ alkoxy carbonyl;

$R^{16}$ is selected from hydrogen, halogen, hydroxyl, cyano, alkyl, $C_{1-6}$ alkoxy or 3-8 membered cycloalkyl.

**26.** The use according to claim 25, wherein $L_2$ comprises a linker selected from Val-Cit, MC, PAB and MC-PAB, preferably MC.

**27.** The use according to claim 1, wherein D is maytansinoids; preferably DM1, DM3 and DM4; more preferably DM1.

**28.** The use according to claim 27, wherein $L_2$ is selected from the group consisting of N-succinimidyl 4-(2-pyridylthio) valerate, N-succinimidyl 4- (N-maleimidomethyl) -cyclohexane-1-carboxylate or N-succinimidyl (4-iodo-acetyl) aminobenzoate; preferably N-succinimidyl 4-(2-pyridylthio) valerate or succinimidyl 4- (N-maleimidomethyl) -cyclohexane-1-carboxylate.

**29.** The use according to claim 1, wherein D is a camptothecin alkaloid which is selected from CPT, 10-hydroxy-CPT, Irinotecan, SN-38 or topotecan, more preferably SN-38.

**30.** The use according to claim 29, wherein said linker $L_2$ is selected from Val-Cit, MC, PAB or MC-PAB; preferably MC or MC-vc-PAB.

**31.** The use according to claim 1, wherein said antibody-cytotoxic drug conjugate is a conjugated drug of formula (II) or pharmaceutically acceptable salt or solvate thereof:

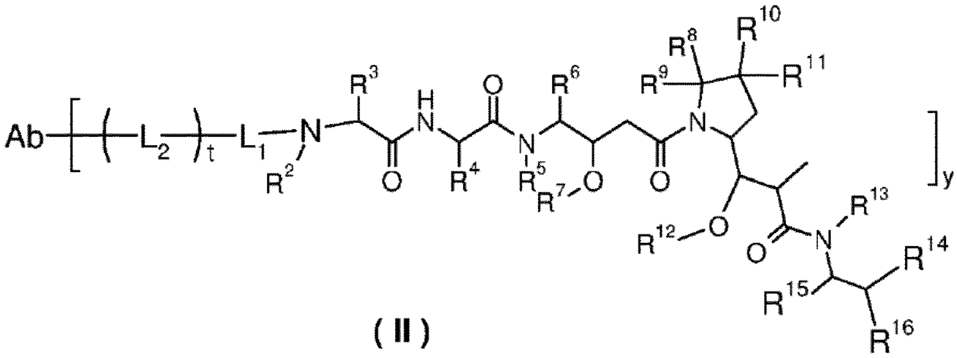

( II )

wherein:

$R^2$-$R^{16}$ are as defined in claim 25;
Ab, t, y, $L_1$ and $L_2$ are as defined in claim 1.

**32.** The use according to claim 1, wherein said antibody-cytotoxic drug conjugate is a conjugated drug of formula (III) or pharmaceutically acceptable salt or solvate thereof:

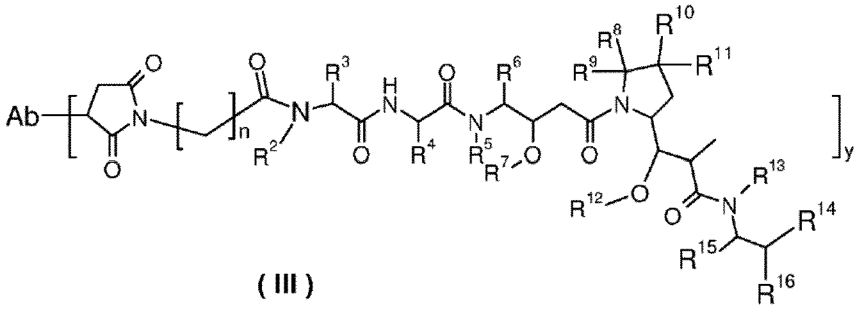

( III )

wherein:

$R^2$-$R^{16}$ are as defined in claim 25;
Ab and y are as defined in claim 1;
n is 3-6, preferably 5.

**33.** The use according to claim 1, wherein said antibody-cytotoxic drug conjugate is a conjugated drug of formula (IV) or pharmaceutically acceptable salt or solvate thereof:

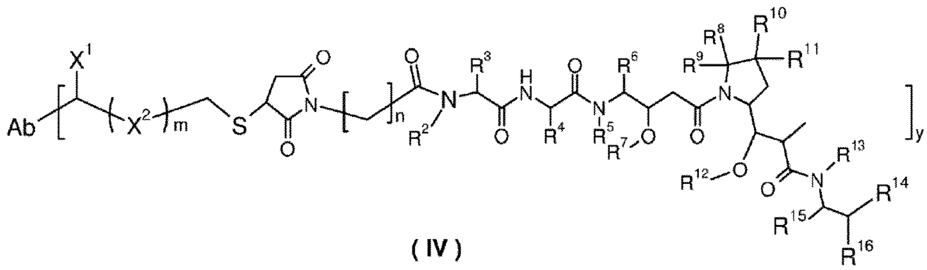

**( IV )**

wherein:

$R^2$-$R^{16}$ are as defined in claim 25;
Ab and y are as defined in claim 1;
n is as defined in claim 32;
$X^1$, $X^2$ and m are as defined in claim 23.

**34.** The use according to claim 1, wherein said antibody-cytotoxic drug conjugate is a conjugated drug of formula (V) or pharmaceutically acceptable salt or solvate thereof:

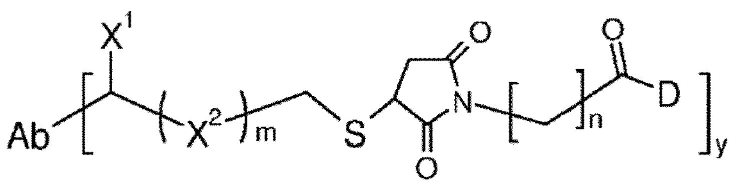

**( V )**

wherein:

Ab, D and y are as defined in claim 1;
n is as defined in claim 32;
$X^1$, $X^2$ and m are as defined in claim 23.

**35.** The use according to any one of claims 1-34, wherein said antibody-cytotoxic drug conjugate or pharmaceutically acceptable salt or solvate thereof is selected from the group consisting of:

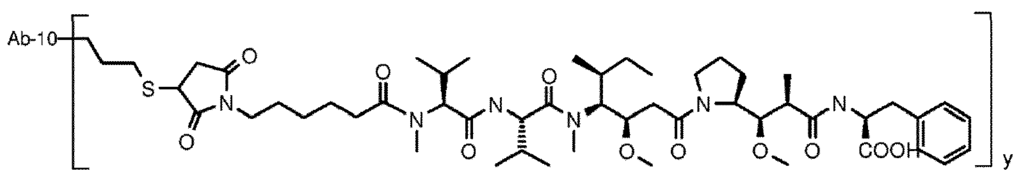

ADC-1

,

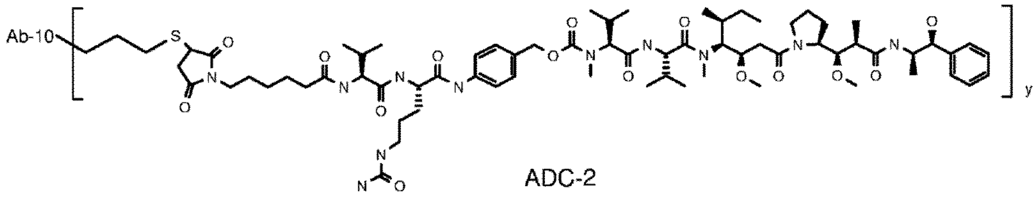

ADC-2

,

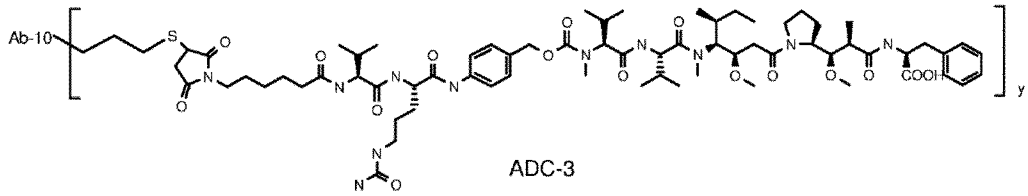

ADC-3

,

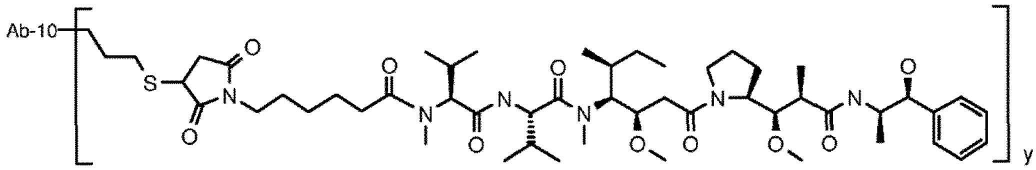

ADC-4

,

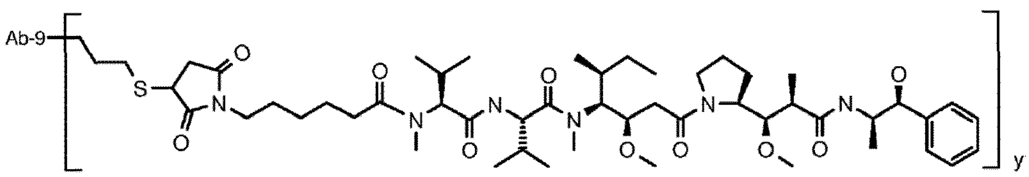

ADC-5

,

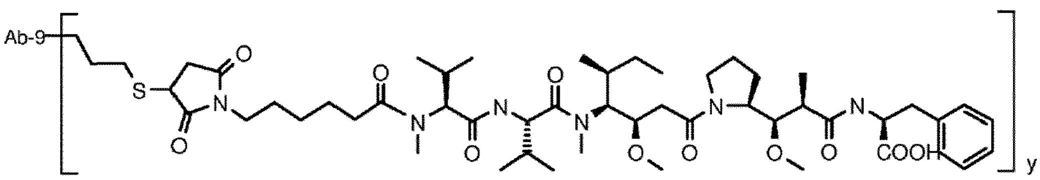

ADC-6

,

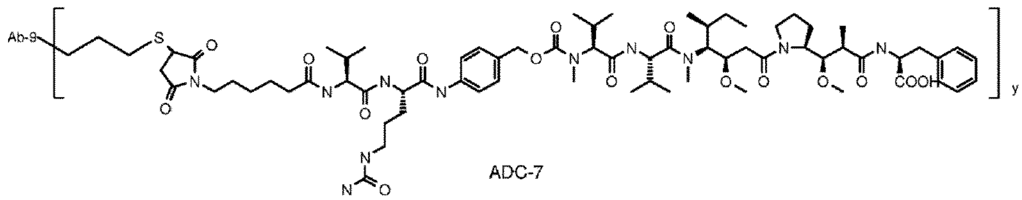

ADC-7

,

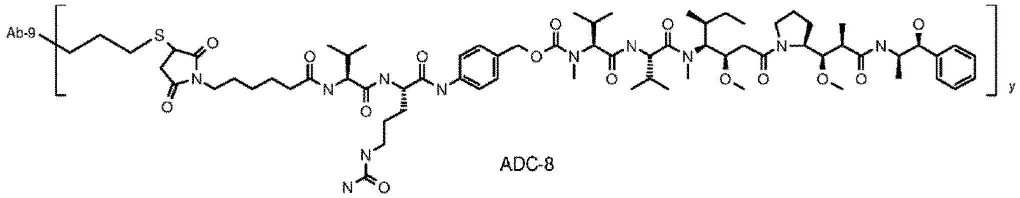

ADC-8

,

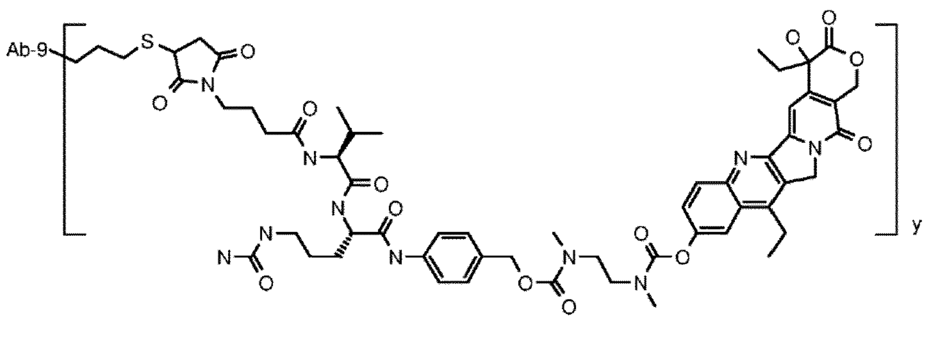

ADC-11

,

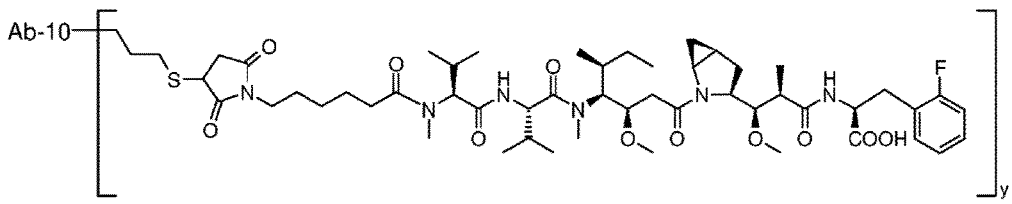

ADC-12

,

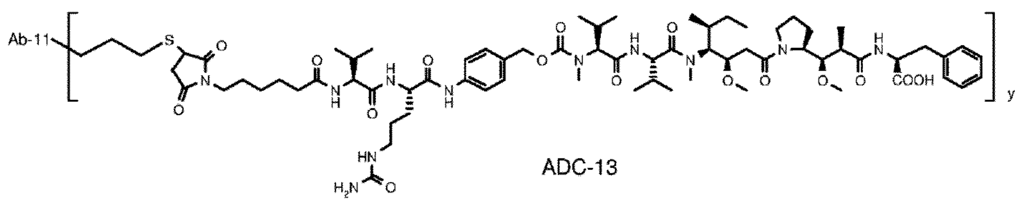

ADC-13

,

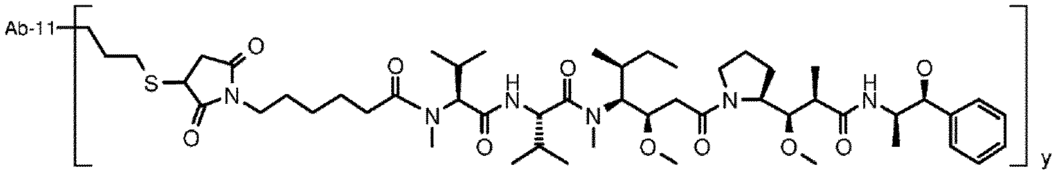

ADC-14

;

wherein Ab-9, Ab-10 and Ab-11 are as defined in claim 21, y ranges from 1-8, preferably 2-5.

**36.** The use according to claim 1, wherein said hepatic carcinoma is c-Met positive hepatic carcinoma or hepatic carcinoma which overexpresses c-Met.

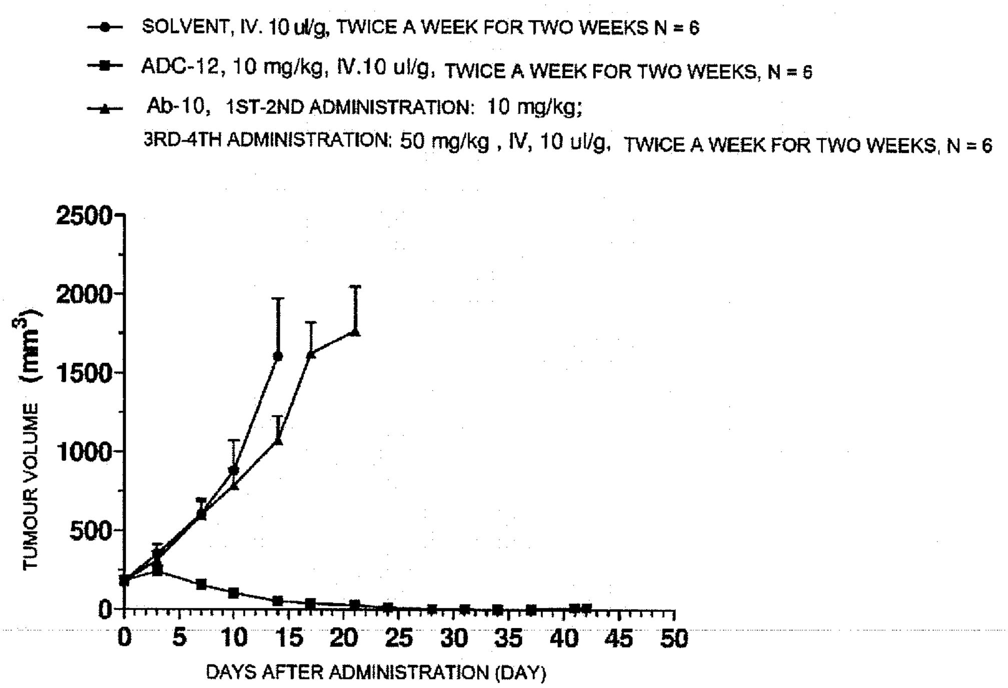


Figure 1

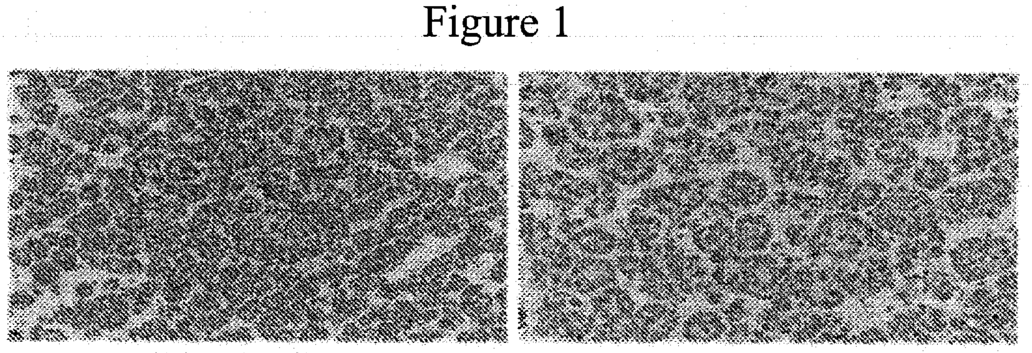

LI-03-0010 P4, 20X, IHC score 0 LI-03-0010 P4, 40X, IHC score 0

Figure 2

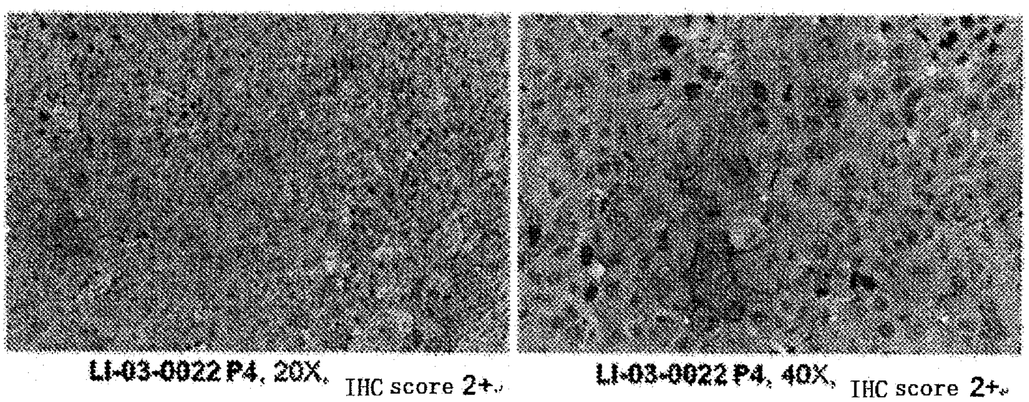

LI-03-0022 P4, 20X, IHC score 2+ LI-03-0022 P4, 40X, IHC score 2+

Figure 3

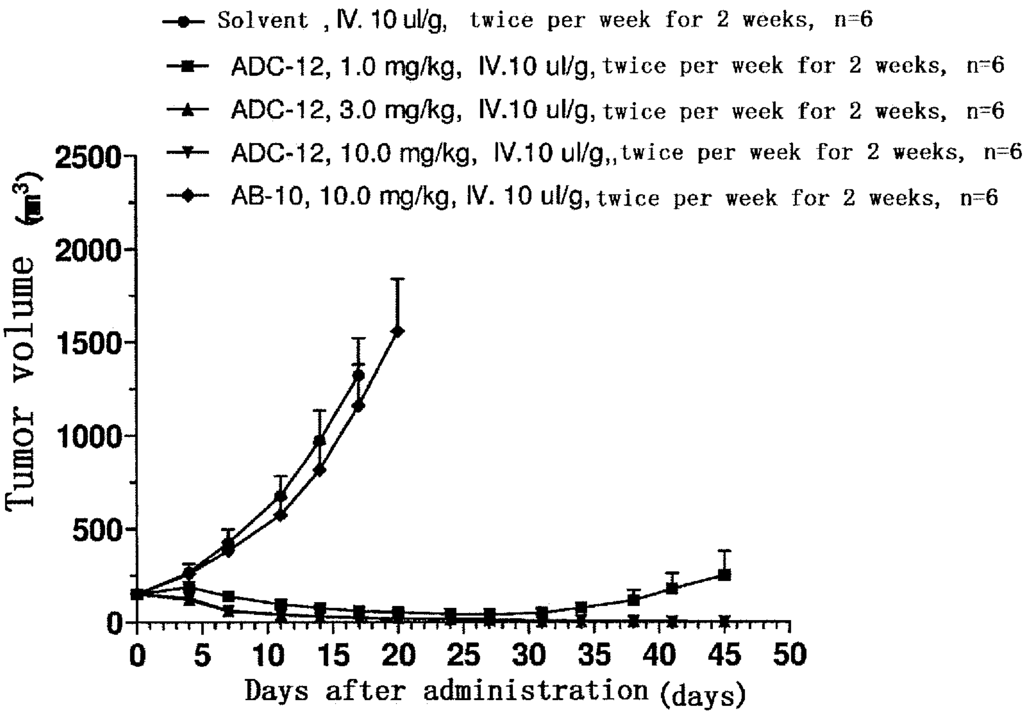


Figure 4

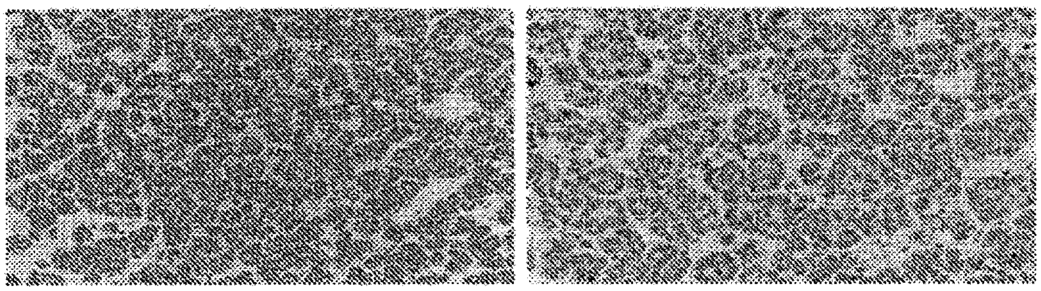

LI-03-0010 P4, 20X, IHC score 0 LI-03-0010 P4, 40X, IHC score 0

Figure 5

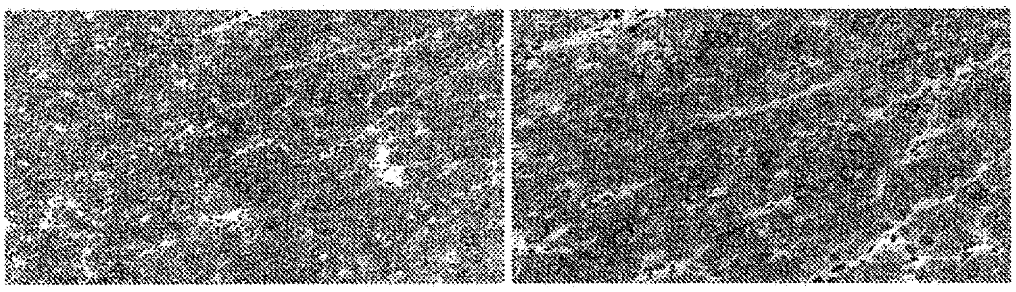

LI-03-0240-P4, 20X, IHC score 3+ LI-03-0240-P4, 40X, IHC score 3+

Figure 6

# INTERNATIONAL SEARCH REPORT

International application No. PCT/CN2017/106044

A. CLASSIFICATION OF SUBJECT MATTER

A61K 39/395 (2006.01) i; A61K 47/68 (2017.01) i; A61K 45/00 (2006.01) i; A61K 31/4745 (2006.01) i; A61K 31/537 (2006.01) i; A61K 38/08 (2006.01) i; A61K 38/06 (2006.01) i; A61P 35/00 (2006.01) i

According to International Patent Classification (IPC) or to both national classification and IPC

B. FIELDS SEARCHED

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNMED; CPRSABS; CNABS; DWPI; SIPOABS; CNTXT; WOTXT; EPTXT; JPTXT; CNKI; Pubmed; Google scholar: 肝细胞生长因子受体，单抗，抗体，人源化，偶联，接头，化疗药物，细胞毒，靶向，肝癌，互补决定区，可变区，c-MET, HGFR, monoclonal, antibody, mAb, humaniz+, coupl+, linker, adaptor, cytotox+, MC-MMAF, MC-MMAE, MC-VC-PAB-MMAE, MC-VC-PAB-MMAF, SN-38, targeting, liver cancer, CDR, 基于序列 SEQ ID NOs: 1-28 的检索

C. DOCUMENTS CONSIDERED TO BE RELEVANT

| Category* | Citation of document, with indication, where appropriate, of the relevant passages | Relevant to claim No. |
|---|---|---|
| PX | WO 2016165580 A1 (JIANGSU HENGRUI MEDICINE CO et al.), 20 October 2016 (20.10.2016), see entire document | 1-36 |
| A | US 2015071950 A1 (SAMSUNG ELECTRONICS CO., LTD.), 12 March 2015 (12.03.2015), see entire document | 1-36 |
| A | CN 105452297 A (AGENCY FOR SCIENCE, TECHNOLOGY AND RESEARCH), 30 March 2016 (30.03.2016), see entire document | 1-36 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 02 January 2018 | 11 January 2018 |

Name and mailing address of the ISA
State Intellectual Property Office of the P. R. China
No. 6, Xitucheng Road, Jimenqiao
Haidian District, Beijing 100088, China
Facsimile No. (86-10) 62019451

Authorized officer
LI, Kangqi
Telephone No. (86-10) 62411034

Form PCT/ISA/210 (second sheet) (July 2009)

## INTERNATIONAL SEARCH REPORT

International application No.
PCT/CN2017/106044

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing filed or furnished:

a. (means)

☐ on paper

☒ in electronic form

b. (time)

☒ in the international application as filed

☐ together with the international application in electronic form

☐ subsequently to this Authority for the purposes of search

2. ☐ In addition, in the case that more than one version or copy of a sequence listing has been filed or furnished, the required statements that the information in the subsequent or additional copies is identical to that in the application as filed or does not go beyond the application as filed, as appropriate, were furnished.

3. Additional comments:

Form PCT/ISA/210 (continuation of first sheet (1)) (July 2009)

**INTERNATIONAL SEARCH REPORT**
Information on patent family members

International application No.
PCT/CN2017/106044

| Patent Documents referred in the Report | Publication Date | Patent Family | Publication Date |
|---|---|---|---|
| WO 2016165580 A1 | 20 October 2016 | None | |
| US 2015071950 A1 | 12 March 2015 | KR 20150030829 A | 23 March 2015 |
| CN 105452297 A | 30 March 2016 | EP 2992019 A4 | 28 December 2016 |
| | | WO 2014178791 A1 | 06 November 2014 |
| | | EP 2992019 A1 | 09 March 2016 |
| | | JP 2016520578 A | 14 July 2016 |
| | | US 2016083479 A1 | 24 March 2016 |
| | | SG 11201508875T A | 27 November 2015 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2016078699 W
- US 7750116 B
- US 5208020 A
- US 20050238649 A1
- US 3896111 A
- US 4151042 A
- US 5635483 A
- US 5780588 A
- US 5663149 A
- WO 02088172 A
- US 20050238649 A
- WO 2006034488 A
- US 5362852 A
- WO 9411026 A
- WO 2005081711 A
- WO 2004010957 A
- US 7750116 B1
- WO 2013072813 A


### Non-patent literature cited in the description


- *J. biol. chem,* 1968, vol. 243, 3558
- **KABAT, EA et al.** Sequences of Proteins of Immunological Interest. 1991
- **QUEEN et al.** *Proc. Natl. Acad. Sci. USA,* 1991, vol. 88, 2869
- **JONES et al.** *Nature,* 1986, vol. 321, 522
- **RIECHMANN et al.** *Nature,* 1988, vol. 332, 323-327
- **VERHOEYEN et al.** *Science,* 1988, vol. 239, 1534
- **WATSON et al.** Molecular Biology of the Gene. The Benjamin/Cummings Pub. Co, 1987, 224
- **CHARI et al.** *Cancer Research,* 1992, vol. 52, 127-131
- **DORONINA et al.** *Nat. Biotechnol.,* 2003, vol. 21, 778-784
- **HAMANN et al.** *Expert Opin. Ther. Patents,* 2005, vol. 15, 1087-1103
- **YU et al.** *PNAS,* 2002, vol. 99, 7968-7973
- **WOYKE et al.** *Antimicrob. Agents and Chemother.,* 2001, vol. 45 (12), 3580-3584
- **PETTIT et al.** *Antimicrob. Agents Chemother.,* 1998, vol. 42, 2961-2965
- **SENTER et al.** *Proceedings of the American Association for CancerResearch,* 28 March 2004, vol. 45
- **DORONINA et al.** *Bioconjugate Chem.,* 2006, vol. 17, 114-124
- **E. SCHRODER ; K.LÜBKE.** The Peptides. Academic Press, 1965, vol. 1, 76-136
- **PETTIT et al.** *J. Am. Chem. Soc.,* 1989, vol. 111, 5463-5465
- **PETTIT et al.** *Anti-Cancer Drug Design,* 1998, vol. 13, 243-277
- **PETTIT, G. R. et al.** *Synthesis,* 1996, 719-725
- **PETTIT et al.** *J. Chem. Soc. Perkin Trans.,* 1996, vol. 15, 859-863
- **DORONINA.** *Nat. Biotechnol.,* 2003, vol. 21 (7), 778-784
- **DORONINA et al.** *Nat. Biotech.,* 2003, vol. 21, 778-784
- **GEOGHEGAN ; STROH.** *Bioconjugate Chem.,* 1992, vol. 3, 138-146
- 2003-2004 Application Manual and product catalog. Pierce Biotechnology, Inc, 2003
- 2003-2004 Applications Handbook and Catalog. 2003, 467-498
- **VITETTA et al.** *Science,* 1987, vol. 238, 1098
- **SAMBROOK et al.** Molecular Cloning, A Laboratory Manual. Cold Spring Harbor Laboratory
- **AUSUBEL et al.** Current Protocols in Molecular Biology. Greene Publishing Associates, Wiley Interscience
- *Advanced Synthesis & Catalysis,* 2012, vol. 354 (17), 3327-3332

Sequence listing

<110> SUZHOU SUNCADIA BIOPHARMACEUTICALS CO., LTD.
JIANGSU HENGRUI MEDICINE CO., LTD.
SHANGHAI HENGRUI PHARMACEUTICAL CO., LTD.

<120> MEDICAL USE OF ANTI-C MET ANTIBODY-CYTOTOXIC DRUG CONJUGATE

<130> GECBP/P68724EP

<140> EP 17861068.9
<141> 2017-10-13

<150> PCT/CN2017/106044
<151> 2017-10-13

<150> CN201610898963.7
<151> 2016-10-14

<160> 28

<170> SIPOSequenceListing 1.0

<210> 1
<211> 2796
<212> DNA
<213> artificial sequence

<220>
<221> CDS
<222> (1)..(2796)
<223> fusion protein of human c-Met ECD and murine Fc region

<400> 1
atgaaggccc ccgctgtgct tgcacctggc atcctcgtgc tcctgtttac cttggtgcag 60

aggagcaatg gggagtgtaa agaggcacta gcaaagtccg agatgaatgt gaatatgaag 120

tatcagcttc ccaacttcac cgcggaaaca cccatccaga atgtcattct acatgagcat 180

cacattttcc ttggtgccac taactacatt tatgttttaa atgaggaaga ccttcagaag 240

gttgctgagt acaagactgg gcctgtgctg gaacacccag attgtttccc atgtcaggac 300

tgcagcagca aagccaattt atcaggaggt gtttggaaag ataacatcaa catggctcta 360

gttgtcgaca cctactatga tgatcaactc attagctgtg gcagcgtcaa cagagggacc 420

tgccagcgac atgtctttcc ccacaatcat actgctgaca tacagtcgga ggttcactgc 480

atattctccc cacagataga agagcccagc cagtgtcctg actgtgtggt gagcgccctg 540

ggagccaaag tcctttcatc tgtaaaggac cggttcatca acttctttgt aggcaatacc 600

ataaattctt cttatttccc agatcatcca ttgcattcga tatcagtgag aaggctaaag 660

gaaacgaaag atggttttat gtttttgacg gaccagtcct acattgatgt tttacctgag 720

ttcagagatt cttaccccat taagtatgtc catgcctttg aaagcaacaa ttttatttac 780

ttcttgacgg tccaaaggga aactctagat gctcagactt ttcacacaag aataatcagg 840

```
ttctgttcca taaactctgg attgcattcc tacatggaaa tgcctctgga gtgtattctc      900
acagaaaaga gaaaaaagag atccacaaag aaggaagtgt ttaatatact tcaggctgcg      960
tatgtcagca agcctggggc ccagcttgct agacaaatag gagccagcct gaatgatgac     1020
attcttttcg ggtgttcgc acaaagcaag ccagattctg ccgaaccaat ggatcgatct     1080
gccatgtgtg cattccctat caaatatgtc aacgacttct tcaacaagat cgtcaacaaa     1140
aacaatgtga gatgtctcca gcatttttac ggacccaatc atgagcactg ctttaatagg     1200
acacttctga gaaattcatc aggctgtgaa gcgcgccgtg atgaatatcg aacagagttt     1260
accacagctt tgcagcgcgt tgacttattc atgggtcaat tcagcgaagt cctcttaaca     1320
tctatatcca ccttcattaa aggagacctc accatagcta atcttgggac atcagagggt     1380
cgcttcatgc aggttgtggt ttctcgatca ggaccatcaa cccctcatgt gaattttctc     1440
ctggactccc atccagtgtc tccagaagtg attgtggagc atacattaaa ccaaaatggc     1500
tacacactgg ttatcactgg gaagaagatc acgaagatcc cattgaatgg cttgggctgc     1560
agacatttcc agtcctgcag tcaatgcctc tctgccccac cctttgttca gtgtggctgg     1620
tgccacgaca aatgtgtgcg atcggaggaa tgcctgagcg ggacatggac tcaacagatc     1680
tgtctgcctg caatctacaa ggttttccca aatagtgcac cccttgaagg agggacaagg     1740
ctgaccatat gtggctggga ctttggattt cggaggaata ataaatttga tttaaagaaa     1800
actagagttc tccttggaaa tgagagctgc accttgactt taagtgagag cacgatgaat     1860
acattgaaat gcacagttgg tcctgccatg aataagcatt tcaatatgtc cataattatt     1920
tcaaatggcc acgggacaac acaatacagt acattctcct atgtggatcc tgtaataaca     1980
agtatttcgc cgaaatacgg tcctatggct ggtggcactt tacttacttt aactggaaat     2040
tacctaaaca gtgggaattc tagacacatt tcaattggtg gaaaaacatg tactttaaaa     2100
agtgtgtcaa acagtattct tgaatgttat accccagccc aaaccatttc aactgagttt     2160
gctgttaaat tgaaaattga cttagccaac cgagagacaa gcatcttcag ttaccgtgaa     2220
gatcccattg tctatgaaat tcatccaacc aaatctttta ttagtggtgg gagcacaata     2280
acaggtgttg ggaaaaacct gaattcagtt agtgtcccga gaatggtcat aaatgtgcat     2340
gaagcaggaa ggaactttac agtggcatgt caacatcgct ctaattcaga gataatctgt     2400
tgtaccactc cttccctgca acagctgaat ctgcaactcc ccctgaaaac caaagccttt     2460
ttcatgttag atgggatcct ttccaaatac tttgatctca tttatgtaca taatcctgtg     2520
tttaagcctt ttgaaaagcc agtgatgatc tcaatgggca atgaaaatgt actggaaatt     2580
aagggaaatg atattgaccc tgaagcagtt aaaggtgaag tgttaaaagt tggaaataag     2640
agctgtgaga atatacactt acattctgaa gccgttttat gcacggtccc caatgacctg     2700
ctgaaattga acagcgagct aaatatagag tggaagcaag caatttcttc aaccgtcctt     2760
```

ggaaaagtaa tagttcaacc agatcagaat ttcaca 2796

<210> 2
<211> 1758
<212> DNA
<213> artificial sequence

<220>
<221> CDS
<222> (1)..(1758)
<223> human cMet extracellular Sema region and Flag-his-tag

<400> 2
atgaaggccc ccgctgtgct tgcacctggc atcctcgtgc tcctgtttac cttggtgcag 60

aggagcaatg gggagtgtaa agaggcacta gcaaagtccg agatgaatgt gaatatgaag 120

tatcagcttc ccaacttcac cgcggaaaca cccatccaga atgtcattct acatgagcat 180

cacattttcc ttggtgccac taactacatt tatgttttaa atgaggaaga ccttcagaag 240

gttgctgagt acaagactgg gcctgtgctg gaacacccag attgtttccc atgtcaggac 300

tgcagcagca aagccaattt atcaggaggt gtttggaaag ataacatcaa catggctcta 360

gttgtcgaca cctactatga tgatcaactc attagctgtg gcagcgtcaa cagagggacc 420

tgccagcgac atgtctttcc ccacaatcat actgctgaca tacagtcgga ggttcactgc 480

atattctccc cacagataga agagcccagc cagtgtcctg actgtgtggt gagcgccctg 540

ggagccaaag tcctttcatc tgtaaaggac cggttcatca acttctttgt aggcaatacc 600

ataaattctt cttatttccc agatcatcca ttgcattcga tatcagtgag aaggctaaag 660

gaaacgaaag atggttttat gtttttgacg gaccagtcct acattgatgt tttacctgag 720

ttcagagatt cttaccccat taagtatgtc catgcctttg aaagcaacaa ttttatttac 780

ttcttgacgg tccaaaggga aactctagat gctcagactt ttcacacaag aataatcagg 840

ttctgttcca taaactctgg attgcattcc tacatggaaa tgcctctgga gtgtattctc 900

acagaaaaga gaaaaaagag atccacaaag aaggaagtgt ttaatatact tcaggctgcg 960

tatgtcagca agcctggggc ccagcttgct agacaaatag gagccagcct gaatgatgac 1020

attcttttcg gggtgttcgc acaaagcaag ccagattctg ccgaaccaat ggatcgatct 1080

gccatgtgtg cattccctat caaatatgtc aacgacttct tcaacaagat cgtcaacaaa 1140

aacaatgtga gatgtctcca gcatttttac ggacccaatc atgagcactg ctttaatagg 1200

acacttctga gaaattcatc aggctgtgaa gcgcgccgtg atgaatatcg aacagagttt 1260

accacagctt tgcagcgcgt tgacttattc atgggtcaat tcagcgaagt cctcttaaca 1320

tctatatcca ccttcattaa aggagacctc accatagcta atcttgggac atcagagggt 1380

cgcttcatgc aggttgtggt ttctcgatca ggaccatcaa cccctcatgt gaattttctc 1440

ctggactccc atccagtgtc tccagaagtg attgtggagc atacattaaa ccaaaatggc 1500

tacacactgg ttatcactgg gaagaagatc acgaagatcc cattgaatgg cttgggctgc 1560

agacatttcc agtcctgcag tcaatgcctc tctgccccac cctttgttca gtgtggctgg 1620

tgccacgaca aatgtgtgcg atcggaggaa tgcctgagcg ggacatggac tcaacagatc 1680

tgtctgcctg caatctacaa ggactacaag gacgacgacg acaagcatgt ccaccatcat 1740

caccatcact gattcgaa 1758

<210> 3
<211> 2823
<212> DNA
<213> artificial sequence

<220>
<221> CDS
<222> (1)..(2823)
<223> recombinant protein of human c-Met ECD and his-tag

<400> 3
atgaaggccc ccgctgtgct tgcacctggc atcctcgtgc tcctgtttac cttggtgcag 60

aggagcaatg gggagtgtaa agaggcacta gcaaagtccg agatgaatgt gaatatgaag 120

tatcagcttc ccaacttcac cgcggaaaca cccatccaga atgtcattct acatgagcat 180

cacattttcc ttggtgccac taactacatt tatgttttaa atgaggaaga ccttcagaag 240

gttgctgagt acaagactgg gcctgtgctg gaacacccag attgtttccc atgtcaggac 300

tgcagcagca aagccaattt atcaggaggt gtttggaaag ataacatcaa catggctcta 360

gttgtcgaca cctactatga tgatcaactc attagctgtg gcagcgtcaa cagagggacc 420

tgccagcgac atgtctttcc ccacaatcat actgctgaca tacagtcgga ggttcactgc 480

atattctccc cacagataga agagcccagc cagtgtcctg actgtgtggt gagcgccctg 540

ggagccaaag tcctttcatc tgtaaaggac cggttcatca acttctttgt aggcaatacc 600

ataaattctt cttatttccc agatcatcca ttgcattcga tatcagtgag aaggctaaag 660

gaaacgaaag atggttttat gtttttgacg gaccagtcct acattgatgt tttacctgag 720

ttcagagatt cttaccccat taagtatgtc catgcctttg aaagcaacaa ttttatttac 780

ttcttgacgg tccaaaggga aactctagat gctcagactt ttcacacaag aataatcagg 840

ttctgttcca taaactctgg attgcattcc tacatggaaa tgcctctgga gtgtattctc 900

acagaaaaga gaaaaaagag atccacaaag aaggaagtgt ttaatatact tcaggctgcg 960

tatgtcagca agcctggggc ccagcttgct agacaaatag gagccagcct gaatgatgac 1020

attcttttcg gggtgttcgc acaaagcaag ccagattctg ccgaaccaat ggatcgatct 1080

gccatgtgtg cattccctat caaatatgtc aacgacttct tcaacaagat cgtcaacaaa 1140

aacaatgtga gatgtctcca gcatttttac ggacccaatc atgagcactg ctttaatagg 1200

acacttctga gaaattcatc aggctgtgaa gcgcgccgtg atgaatatcg aacagagttt 1260

accacagctt tgcagcgcgt tgacttattc atgggtcaat tcagcgaagt cctcttaaca 1320

tctatatcca ccttcattaa aggagacctc accatagcta atcttgggac atcagagggt 1380

cgcttcatgc aggttgtggt ttctcgatca ggaccatcaa cccctcatgt gaattttctc 1440

ctggactccc atccagtgtc tccagaagtg attgtggagc atacattaaa ccaaaatggc 1500

tacacactgg ttatcactgg gaagaagatc acgaagatcc cattgaatgg cttgggctgc 1560

agacatttcc agtcctgcag tcaatgcctc tctgccccac cctttgttca gtgtggctgg 1620

tgccacgaca aatgtgtgcg atcggaggaa tgcctgagcg ggacatggac tcaacagatc 1680

tgtctgcctg caatctacaa ggttttccca aatagtgcac cccttgaagg agggacaagg 1740

ctgaccatat gtggctggga ctttggattt cggaggaata ataaatttga tttaaagaaa 1800

actagagttc tccttggaaa tgagagctgc accttgactt taagtgagag cacgatgaat 1860

acattgaaat gcacagttgg tcctgccatg aataagcatt tcaatatgtc cataattatt 1920

tcaaatggcc acgggacaac acaatacagt acattctcct atgtggatcc tgtaataaca 1980

agtatttcgc cgaaatacgg tcctatggct ggtggcactt tacttacttt aactggaaat 2040

tacctaaaca gtgggaattc tagacacatt tcaattggtg gaaaaacatg tactttaaaa 2100

agtgtgtcaa acagtattct tgaatgttat accccagccc aaaccatttc aactgagttt 2160

gctgttaaat tgaaaattga cttagccaac cgagagacaa gcatcttcag ttaccgtgaa 2220

gatcccattg tctatgaaat tcatccaacc aaatctttta ttagtggtgg gagcacaata 2280

acaggtgttg ggaaaaacct gaattcagtt agtgtcccga gaatggtcat aaatgtgcat 2340

gaagcaggaa ggaactttac agtggcatgt caacatcgct ctaattcaga gataatctgt 2400

tgtaccactc cttccctgca acagctgaat ctgcaactcc ccctgaaaac caaagccttt 2460

ttcatgttag atgggatcct ttccaaatac tttgatctca tttatgtaca taatcctgtg 2520

tttaagcctt ttgaaaagcc agtgatgatc tcaatgggca atgaaaatgt actggaaatt 2580

aagggaaatg atattgaccc tgaagcagtt aaaggtgaag tgttaaaagt tggaaataag 2640

agctgtgaga atatacactt acattctgaa gccgttttat gcacggtccc caatgacctg 2700

ctgaaattga acagcgagct aaatatagag tggaagcaag caatttcttc aaccgtcctt 2760

ggaaaagtaa tagttcaacc agatcagaat ttcacacacc atcatcacca tcactgattc 2820

gaa 2823

<210> 4
<211> 120
<212> PRT
<213> Mus musculus

<400> 4
Gln Val Gln Leu Lys Gln Ser Gly Pro Gly Leu Val Gln Pro Ser Gln

```
1               5                   10                  15
Ser Leu Ser Ile Thr Cys Thr Val Ser Gly Phe Ser Leu Pro Asn Tyr
            20                  25                  30
Gly Val His Trp Val Arg Gln Ser Pro Gly Lys Gly Leu Glu Trp Leu
        35                  40                  45
Gly Val Ile Trp Ser Gly Gly Ser Thr Asn Tyr Ala Ala Ala Phe Val
    50                  55                  60
Ser Arg Leu Arg Ile Ser Lys Asp Asn Ser Lys Ser Gln Val Phe Phe
65                  70                  75                  80
Glu Met Asn Ser Leu Gln Ala Asp Asp Thr Ala Val Tyr Tyr Cys Ala
                85                  90                  95
Arg Asn His Asp Asn Pro Tyr Asn Tyr Ala Met Asp Tyr Trp Gly Gln
            100                 105                 110
Gly Thr Thr Val Thr Val Ser Ser
        115                 120
```

<210> 5
<211> 112
<212> PRT
<213> Mus musculus

<400> 5

```
Asp Ile Val Leu Thr Gln Ser Pro Gly Ser Leu Ala Val Tyr Leu Gly
1               5                   10                  15
Gln Arg Ala Thr Ile Ser Cys Arg Ala Asn Lys Ser Val Ser Thr Ser
            20                  25                  30
Thr Tyr Asn Tyr Leu His Trp Tyr Gln Gln Lys Pro Gly Gln Pro Pro
        35                  40                  45
Lys Leu Leu Ile Tyr Leu Ala Ser Asn Leu Ala Ser Gly Val Pro Ala
    50                  55                  60
Arg Phe Ser Gly Ser Gly Ser Gly Thr Asp Phe Thr Leu Asn Ile His
65                  70                  75                  80
Pro Leu Glu Glu Glu Asp Ala Ala Thr Tyr Tyr Cys Gln His Ser Arg
                85                  90                  95
Asp Leu Pro Pro Thr Phe Gly Ala Gly Thr Lys Leu Glu Leu Lys Arg
            100                 105                 110
```

<210> 6
<211> 5
<212> PRT
<213> Mus musculus

<400> 6

```
Asn Tyr Gly Val His
1               5
```

<210> 7
<211> 16
<212> PRT
<213> Mus musculus

<400> 7

```
Val Ile Trp Ser Gly Gly Ser Thr Asn Tyr Ala Ala Ala Phe Val Ser
1               5                   10                  15
```

<210> 8
<211> 12
<212> PRT
<213> Mus musculus

<400> 8

```
Asn His Asp Asn Pro Tyr Asn Tyr Ala Met Asp Tyr
1               5                   10
```

<210> 9
<211> 15
<212> PRT
<213> Mus musculus

<400> 9
Arg Ala Asn Lys Ser Val Ser Thr Ser Thr Tyr Asn Tyr Leu His
1 5 10 15

<210> 10
<211> 7
<212> PRT
<213> Mus musculus

<400> 10
Leu Ala Ser Asn Leu Ala Ser
1 5

<210> 11
<211> 9
<212> PRT
<213> Mus musculus

<400> 11
Gln His Ser Arg Asp Leu Pro Pro Thr
1 5

<210> 12
<211> 15
<212> PRT
<213> artificial sequence

<220>
<221> PEPTIDE
<222> (1)..(15)
<223> optimized light chain CDR1

<400> 12
Arg Ala Asp Lys Ser Val Ser Thr Ser Thr Tyr Asn Tyr Leu His
1 5 10 15

<210> 13
<211> 120
<212> PRT
<213> artificial sequence

<220>
<221> PEPTIDE
<222> (1)..(120)
<223> Ab-9 heavy chain variable region

<400> 13
Gln Val Thr Leu Lys Glu Ser Gly Pro Val Leu Val Lys Pro Thr Glu
1 5 10 15
Thr Leu Thr Leu Thr Cys Thr Val Ser Gly Phe Ser Leu Pro Asn Tyr
20 25 30
Gly Val His Trp Val Arg Gln Pro Pro Gly Lys Ala Leu Glu Trp Leu
35 40 45
Ala Val Ile Trp Ser Gly Gly Ser Thr Asn Tyr Ala Ala Ala Phe Val
50 55 60

```
Ser Arg Leu Arg Ile Ser Lys Asp Thr Ser Lys Ser Gln Val Val Phe
65                  70                  75                  80
Thr Met Asn Asn Met Asp Pro Val Asp Thr Ala Thr Tyr Tyr Cys Ala
                85                  90                  95
Arg Asn His Asp Asn Pro Tyr Asn Tyr Ala Met Asp Tyr Trp Gly Gln
            100                 105                 110
Gly Thr Thr Val Thr Val Ser Ser
        115                 120
```

<210> 14
<211> 120
<212> PRT
<213> artificial sequence

<220>
<221> PEPTIDE
<222> (1)..(120)
<223> Ab-10 heavy chain variable region

<400> 14

```
Gln Val Gln Leu Val Glu Ser Gly Gly Gly Val Val Gln Pro Gly Arg
1               5                   10                  15
Ser Leu Arg Leu Ser Cys Ala Ala Ser Gly Phe Ser Leu Ser Asn Tyr
            20                  25                  30
Gly Val His Trp Val Arg Gln Ala Pro Gly Lys Gly Leu Glu Trp Leu
        35                  40                  45
Ala Val Ile Trp Ser Gly Gly Ser Thr Asn Tyr Ala Ala Ala Phe Val
    50                  55                  60
Ser Arg Leu Thr Ile Ser Lys Asp Asn Ser Lys Asn Thr Val Tyr Leu
65                  70                  75                  80
Gln Met Asn Ser Leu Arg Ala Glu Asp Thr Ala Val Tyr Tyr Cys Ala
                85                  90                  95
Arg Asn His Asp Asn Pro Tyr Asn Tyr Ala Met Asp Tyr Trp Gly Gln
            100                 105                 110
Gly Thr Thr Val Thr Val Ser Ser
        115                 120
```

<210> 15
<211> 120
<212> PRT
<213> artificial sequence

<220>
<221> PEPTIDE
<222> (1)..(120)
<223> Ab-11 heavy chain variable region

<400> 15

```
Gln Val Gln Leu Val Glu Ser Gly Gly Gly Val Val Gln Pro Gly Arg
1               5                   10                  15
Ser Leu Arg Leu Ser Cys Ala Ala Ser Gly Phe Thr Leu Pro Asn Tyr
            20                  25                  30
Gly Val His Trp Val Arg Gln Ala Pro Gly Lys Gly Leu Glu Trp Leu
        35                  40                  45
Ala Val Ile Trp Ser Gly Gly Ser Thr Asn Tyr Ala Ala Ala Phe Val
    50                  55                  60
Ser Arg Leu Thr Ile Ser Lys Asp Asn Ser Lys Asn Thr Val Tyr Leu
65                  70                  75                  80
Gln Met Asn Ser Leu Arg Ala Glu Asp Thr Ala Val Tyr Tyr Cys Ala
                85                  90                  95
Arg Asn His Asp Asn Pro Tyr Asn Tyr Ala Met Asp Tyr Trp Gly Gln
```

```
            100                 105                 110
Gly Thr Thr Val Thr Val Ser Ser
        115                 120

<210>  16
<211>  112
<212>  PRT
<213>  artificial sequence

<220>
<221>  PEPTIDE
<222>  (1)..(112)
<223>  Ab-9 light chain variable region


<400>  16
Asp Ile Val Leu Thr Gln Ser Pro Ala Ser Leu Ala Val Ser Pro Gly
1               5                   10                  15
Gln Arg Ala Thr Ile Thr Cys Arg Ala Asn Lys Ser Val Ser Thr Ser
            20                  25                  30
Thr Tyr Asn Tyr Leu His Trp Tyr Gln Gln Lys Pro Gly Gln Pro Pro
        35                  40                  45
Lys Leu Leu Ile Tyr Leu Ala Ser Asn Leu Ala Ser Gly Val Pro Ala
    50                  55                  60
Arg Phe Ser Gly Ser Gly Ser Gly Thr Asp Phe Thr Leu Thr Ile Asn
65                  70                  75                  80
Pro Val Glu Ala Asn Asp Thr Ala Asn Tyr Tyr Cys Gln His Ser Arg
                85                  90                  95
Asp Leu Pro Pro Thr Phe Gly Gln Gly Thr Lys Leu Glu Ile Lys Arg
            100                 105                 110

<210>  17
<211>  112
<212>  PRT
<213>  artificial sequence

<220>
<221>  PEPTIDE
<222>  (1)..(112)
<223>  Ab-10 light chain variable region


<400>  17
Asp Ile Val Leu Thr Gln Ser Pro Asp Ser Leu Ala Val Ser Leu Gly
1               5                   10                  15
Glu Arg Ala Thr Ile Asn Cys Arg Ala Asp Lys Ser Val Ser Thr Ser
            20                  25                  30
Thr Tyr Asn Tyr Leu His Trp Tyr Gln Gln Lys Pro Gly Gln Pro Pro
        35                  40                  45
Lys Leu Leu Ile Tyr Leu Ala Ser Asn Leu Ala Ser Gly Val Pro Asp
    50                  55                  60
Arg Phe Ser Gly Ser Gly Ser Gly Thr Asp Phe Thr Leu Thr Ile Ser
65                  70                  75                  80
Ser Leu Gln Ala Glu Asp Val Ala Val Tyr Tyr Cys Gln His Ser Arg
                85                  90                  95
Asp Leu Pro Pro Thr Phe Gly Gln Gly Thr Lys Leu Glu Ile Lys Arg
            100                 105                 110

<210>  18
<211>  112
<212>  PRT
<213>  artificial sequence
```

```
<220>
<221>  PEPTIDE
<222>  (1)..(112)
<223>  Ab-11 light chain variable region


<400>  18
Asp Ile Val Leu Thr Gln Ser Pro Asp Ser Leu Ala Val Ser Leu Gly
1               5                   10                  15
Glu Arg Ala Thr Ile Asn Cys Arg Ala Asn Lys Ser Val Ser Thr Ser
            20                  25                  30
Thr Tyr Asn Tyr Leu His Trp Tyr Gln Gln Lys Pro Gly Gln Pro Pro
        35                  40                  45
Lys Leu Leu Ile Tyr Leu Ala Ser Asn Leu Ala Ser Gly Val Pro Asp
    50                  55                  60
Arg Phe Ser Gly Ser Gly Ser Gly Thr Asp Phe Thr Leu Thr Ile Ser
65                  70                  75                  80
Ser Leu Gln Ala Glu Asp Val Ala Val Tyr Tyr Cys Gln His Ser Arg
                85                  90                  95
Asp Leu Pro Pro Thr Phe Gly Gln Gly Thr Lys Leu Glu Ile Lys Arg
            100                 105                 110

<210>  19
<211>  330
<212>  PRT
<213>  Homo sapiens

<400>  19
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125
Lys Pro Lys Asp Thr Leu Met Ile Ser Arg Thr Pro Glu Val Thr Cys
    130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu
            180                 185                 190
His Gln Asp Trp Leu Asn Gly Lys Glu Tyr Lys Cys Lys Val Ser Asn
        195                 200                 205
Lys Ala Leu Pro Ala Pro Ile Glu Lys Thr Ile Ser Lys Ala Lys Gly
    210                 215                 220
Gln Pro Arg Glu Pro Gln Val Tyr Thr Leu Pro Pro Ser Arg Glu Glu
225                 230                 235                 240
Met Thr Lys Asn Gln Val Ser Leu Thr Cys Leu Val Lys Gly Phe Tyr
                245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
```

```
        275                 280                 285
Leu Tyr Ser Lys Leu Thr Val Asp Lys Ser Arg Trp Gln Gln Gly Asn
    290                 295                 300
Val Phe Ser Cys Ser Val Met His Glu Ala Leu His Asn His Tyr Thr
305                 310                 315                 320
Gln Lys Ser Leu Ser Leu Ser Pro Gly Lys
                325                 330

<210>  20
<211>  326
<212>  PRT
<213>  Homo sapiens

<400>  20
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Asn Phe Gly Thr Gln Thr
65                  70                  75                  80
Tyr Thr Cys Asn Val Asp His Lys Pro Ser Asn Thr Lys Val Asp Lys
                85                  90                  95
Thr Val Glu Arg Lys Cys Cys Val Glu Cys Pro Pro Cys Pro Ala Pro
            100                 105                 110
Pro Val Ala Gly Pro Ser Val Phe Leu Phe Pro Pro Lys Pro Lys Asp
        115                 120                 125
Thr Leu Met Ile Ser Arg Thr Pro Glu Val Thr Cys Val Val Val Asp
    130                 135                 140
Val Ser His Glu Asp Pro Glu Val Gln Phe Asn Trp Tyr Val Asp Gly
145                 150                 155                 160
Val Glu Val His Asn Ala Lys Thr Lys Pro Arg Glu Glu Gln Phe Asn
                165                 170                 175
Ser Thr Phe Arg Val Val Ser Val Leu Thr Val Val His Gln Asp Trp
            180                 185                 190
Leu Asn Gly Lys Glu Tyr Lys Cys Lys Val Ser Asn Lys Gly Leu Pro
        195                 200                 205
Ala Pro Ile Glu Lys Thr Ile Ser Lys Thr Lys Gly Gln Pro Arg Glu
    210                 215                 220
Pro Gln Val Tyr Thr Leu Pro Pro Ser Arg Glu Glu Met Thr Lys Asn
225                 230                 235                 240
Gln Val Ser Leu Thr Cys Leu Val Lys Gly Phe Tyr Pro Ser Asp Ile
                245                 250                 255
Ala Val Glu Trp Glu Ser Asn Gly Gln Pro Glu Asn Asn Tyr Lys Thr
            260                 265                 270
Thr Pro Pro Met Leu Asp Ser Asp Gly Ser Phe Phe Leu Tyr Ser Lys
        275                 280                 285
Leu Thr Val Asp Lys Ser Arg Trp Gln Gln Gly Asn Val Phe Ser Cys
    290                 295                 300
Ser Val Met His Glu Ala Leu His Asn His Tyr Thr Gln Lys Ser Leu
305                 310                 315                 320
Ser Leu Ser Pro Gly Lys
                325

<210>  21
<211>  327
<212>  PRT
<213>  Homo sapiens

<400>  21
```

Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys
                85                  90                  95
Arg Val Glu Ser Lys Tyr Gly Pro Pro Cys Pro Pro Cys Pro Ala Pro
            100                 105                 110
Glu Phe Leu Gly Gly Pro Ser Val Phe Leu Phe Pro Pro Lys Pro Lys
        115                 120                 125
Asp Thr Leu Met Ile Ser Arg Thr Pro Glu Val Thr Cys Val Val Val
    130                 135                 140
Asp Val Ser Gln Glu Asp Pro Glu Val Gln Phe Asn Trp Tyr Val Asp
145                 150                 155                 160
Gly Val Glu Val His Asn Ala Lys Thr Lys Pro Arg Glu Glu Gln Phe
                165                 170                 175
Asn Ser Thr Tyr Arg Val Val Ser Val Leu Thr Val Leu His Gln Asp
            180                 185                 190
Trp Leu Asn Gly Lys Glu Tyr Lys Cys Lys Val Ser Asn Lys Gly Leu
        195                 200                 205
Pro Ser Ser Ile Glu Lys Thr Ile Ser Lys Ala Lys Gly Gln Pro Arg
    210                 215                 220
Glu Pro Gln Val Tyr Thr Leu Pro Pro Ser Gln Glu Glu Met Thr Lys
225                 230                 235                 240
Asn Gln Val Ser Leu Thr Cys Leu Val Lys Gly Phe Tyr Pro Ser Asp
                245                 250                 255
Ile Ala Val Glu Trp Glu Ser Asn Gly Gln Pro Glu Asn Asn Tyr Lys
            260                 265                 270
Thr Thr Pro Pro Val Leu Asp Ser Asp Gly Ser Phe Phe Leu Tyr Ser
        275                 280                 285
Arg Leu Thr Val Asp Lys Ser Arg Trp Gln Glu Gly Asn Val Phe Ser
    290                 295                 300
Cys Ser Val Met His Glu Ala Leu His Asn His Tyr Thr Gln Lys Ser
305                 310                 315                 320
Leu Ser Leu Ser Leu Gly Lys
                325

<210> 22
<211> 106
<212> PRT
<213> Homo sapiens

<400> 22
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Val Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu Cys
            100                 105

<210> 23
<211> 446
<212> PRT
<213> artificial sequence

<220>
<221> PEPTIDE
<222> (1)..(446)
<223> Ab-9 heavy chain

<400> 23
Gln Val Thr Leu Lys Glu Ser Gly Pro Val Leu Val Lys Pro Thr Glu
1 5 10 15
Thr Leu Thr Leu Thr Cys Thr Val Ser Gly Phe Ser Leu Pro Asn Tyr
20 25 30
Gly Val His Trp Val Arg Gln Pro Pro Gly Lys Ala Leu Glu Trp Leu
35 40 45
Ala Val Ile Trp Ser Gly Gly Ser Thr Asn Tyr Ala Ala Ala Phe Val
50 55 60
Ser Arg Leu Arg Ile Ser Lys Asp Thr Ser Lys Ser Gln Val Val Phe
65 70 75 80
Thr Met Asn Asn Met Asp Pro Val Asp Thr Ala Thr Tyr Tyr Cys Ala
85 90 95
Arg Asn His Asp Asn Pro Tyr Asn Tyr Ala Met Asp Tyr Trp Gly Gln
100 105 110
Gly Thr Thr Val Thr Val Ser Ser Ala Ser Thr Lys Gly Pro Ser Val
115 120 125
Phe Pro Leu Ala Pro Cys Ser Arg Ser Thr Ser Glu Ser Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Asn Phe Gly Thr Gln Thr Tyr Thr Cys Asn Val Asp His Lys
195 200 205
Pro Ser Asn Thr Lys Val Asp Lys Thr Val Glu Arg Lys Cys Cys Val
210 215 220
Glu Cys Pro Pro Cys Pro Ala Pro Pro Val Ala Gly Pro Ser Val Phe
225 230 235 240
Leu Phe Pro Pro Lys Pro Lys Asp Thr Leu Met Ile Ser Arg Thr Pro
245 250 255
Glu Val Thr Cys Val Val Val Asp Val Ser His Glu Asp Pro Glu Val
260 265 270
Gln Phe Asn Trp Tyr Val Asp Gly Val Glu Val His Asn Ala Lys Thr
275 280 285
Lys Pro Arg Glu Glu Gln Phe Asn Ser Thr Phe Arg Val Val Ser Val
290 295 300
Leu Thr Val Val His Gln Asp Trp Leu Asn Gly Lys Glu Tyr Lys Cys
305 310 315 320
Lys Val Ser Asn Lys Gly Leu Pro Ala Pro Ile Glu Lys Thr Ile Ser
325 330 335
Lys Thr Lys Gly Gln Pro Arg Glu Pro Gln Val Tyr Thr Leu Pro Pro
340 345 350
Ser Arg Glu Glu Met Thr Lys Asn Gln Val Ser Leu Thr Cys Leu Val
355 360 365
Lys Gly Phe Tyr Pro Ser Asp Ile Ala Val Glu Trp Glu Ser Asn Gly
370 375 380
Gln Pro Glu Asn Asn Tyr Lys Thr Thr Pro Pro Met Leu Asp Ser Asp
385 390 395 400

```
Gly Ser Phe Phe Leu Tyr Ser Lys Leu Thr Val Asp Lys Ser Arg Trp
                405                 410                 415
Gln Gln Gly Asn Val Phe Ser Cys Ser Val Met His Glu Ala Leu His
            420                 425                 430
Asn His Tyr Thr Gln Lys Ser Leu Ser Leu Ser Pro Gly Lys
        435                 440                 445

<210>  24
<211>  446
<212>  PRT
<213>  artificial sequence

<220>
<221>  PEPTIDE
<222>  (1)..(446)
<223>  Ab-10 heavy chain


<400>  24
Gln Val Gln Leu Val Glu Ser Gly Gly Gly Val Val Gln Pro Gly Arg
1               5                   10                  15
Ser Leu Arg Leu Ser Cys Ala Ala Ser Gly Phe Ser Leu Ser Asn Tyr
            20                  25                  30
Gly Val His Trp Val Arg Gln Ala Pro Gly Lys Gly Leu Glu Trp Leu
        35                  40                  45
Ala Val Ile Trp Ser Gly Gly Ser Thr Asn Tyr Ala Ala Ala Phe Val
    50                  55                  60
Ser Arg Leu Thr Ile Ser Lys Asp Asn Ser Lys Asn Thr Val Tyr Leu
65                  70                  75                  80
Gln Met Asn Ser Leu Arg Ala Glu Asp Thr Ala Val Tyr Tyr Cys Ala
                85                  90                  95
Arg Asn His Asp Asn Pro Tyr Asn Tyr Ala Met Asp Tyr Trp Gly Gln
            100                 105                 110
Gly Thr Thr Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Asn Phe Gly Thr Gln Thr Tyr Thr Cys Asn Val Asp His Lys
        195                 200                 205
Pro Ser Asn Thr Lys Val Asp Lys Thr Val Glu Arg Lys Cys Cys Val
    210                 215                 220
Glu Cys Pro Pro Cys Pro Ala Pro Pro Val Ala Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser His Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Phe Arg Val Val Ser Val
    290                 295                 300
Leu Thr Val Val His Gln Asp Trp Leu Asn Gly Lys Glu Tyr Lys Cys
305                 310                 315                 320
Lys Val Ser Asn Lys Gly Leu Pro Ala Pro Ile Glu Lys Thr Ile Ser
                325                 330                 335
Lys Thr Lys Gly Gln Pro Arg Glu Pro Gln Val Tyr Thr Leu Pro Pro
            340                 345                 350
```

```
Ser Arg Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Met Leu Asp Ser Asp
385                 390                 395                 400
Gly Ser Phe Phe Leu Tyr Ser Lys Leu Thr Val Asp Lys Ser Arg Trp
                405                 410                 415
Gln Gln Gly Asn Val Phe Ser Cys Ser Val Met His Glu Ala Leu His
            420                 425                 430
Asn His Tyr Thr Gln Lys Ser Leu Ser Leu Ser Pro Gly Lys
        435                 440                 445

<210>  25
<211>  446
<212>  PRT
<213>  artificial sequence

<220>
<221>  PEPTIDE
<222>  (1)..(446)
<223>  Ab-11 heavy chain


<400>  25
Gln Val Gln Leu Val Glu Ser Gly Gly Gly Val Val Gln Pro Gly Arg
1               5                   10                  15
Ser Leu Arg Leu Ser Cys Ala Ala Ser Gly Phe Thr Leu Pro Asn Tyr
            20                  25                  30
Gly Val His Trp Val Arg Gln Ala Pro Gly Lys Gly Leu Glu Trp Leu
        35                  40                  45
Ala Val Ile Trp Ser Gly Gly Ser Thr Asn Tyr Ala Ala Ala Phe Val
    50                  55                  60
Ser Arg Leu Thr Ile Ser Lys Asp Asn Ser Lys Asn Thr Val Tyr Leu
65                  70                  75                  80
Gln Met Asn Ser Leu Arg Ala Glu Asp Thr Ala Val Tyr Tyr Cys Ala
                85                  90                  95
Arg Asn His Asp Asn Pro Tyr Asn Tyr Ala Met Asp Tyr Trp Gly Gln
            100                 105                 110
Gly Thr Thr Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Asn Phe Gly Thr Gln Thr Tyr Thr Cys Asn Val Asp His Lys
        195                 200                 205
Pro Ser Asn Thr Lys Val Asp Lys Thr Val Glu Arg Lys Cys Cys Val
    210                 215                 220
Glu Cys Pro Pro Cys Pro Ala Pro Pro Val Ala Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser His Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Phe Arg Val Val Ser Val
    290                 295                 300
```

```
Leu Thr Val Val His Gln Asp Trp Leu Asn Gly Lys Glu Tyr Lys Cys
305                 310                 315                 320
Lys Val Ser Asn Lys Gly Leu Pro Ala Pro Ile Glu Lys Thr Ile Ser
                325                 330                 335
Lys Thr Lys Gly Gln Pro Arg Glu Pro Gln Val Tyr Thr Leu Pro Pro
            340                 345                 350
Ser Arg Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Met Leu Asp Ser Asp
385                 390                 395                 400
Gly Ser Phe Phe Leu Tyr Ser Lys Leu Thr Val Asp Lys Ser Arg Trp
                405                 410                 415
Gln Gln Gly Asn Val Phe Ser Cys Ser Val Met His Glu Ala Leu His
            420                 425                 430
Asn His Tyr Thr Gln Lys Ser Leu Ser Leu Ser Pro Gly Lys
        435                 440                 445

<210>  26
<211>  218
<212>  PRT
<213>  artificial sequence

<220>
<221>  PEPTIDE
<222>  (1)..(218)
<223>  Ab-9 light chain


<400>  26
Asp Ile Val Leu Thr Gln Ser Pro Ala Ser Leu Ala Val Ser Pro Gly
1               5                   10                  15
Gln Arg Ala Thr Ile Thr Cys Arg Ala Asn Lys Ser Val Ser Thr Ser
            20                  25                  30
Thr Tyr Asn Tyr Leu His Trp Tyr Gln Gln Lys Pro Gly Gln Pro Pro
        35                  40                  45
Lys Leu Leu Ile Tyr Leu Ala Ser Asn Leu Ala Ser Gly Val Pro Ala
    50                  55                  60
Arg Phe Ser Gly Ser Gly Ser Gly Thr Asp Phe Thr Leu Thr Ile Asn
65                  70                  75                  80
Pro Val Glu Ala Asn Asp Thr Ala Asn Tyr Tyr Cys Gln His Ser Arg
                85                  90                  95
Asp Leu Pro Pro Thr Phe Gly Gln Gly Thr Lys Leu Glu Ile Lys Arg
            100                 105                 110
Thr Val Ala Ala Pro Ser Val Phe Ile Phe Pro Pro Ser Asp Glu Gln
        115                 120                 125
Leu Lys Ser Gly Thr Ala Ser Val Val Cys Leu Leu Asn Asn Phe Tyr
    130                 135                 140
Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
        195                 200                 205
Val Thr Lys Ser Phe Asn Arg Gly Glu Cys
    210                 215

<210>  27
<211>  218
<212>  PRT
```

<213> artificial sequence

<220>
<221> PEPTIDE
<222> (1)..(218)
<223> Ab-10 light chain

<400> 27

```
Asp Ile Val Leu Thr Gln Ser Pro Asp Ser Leu Ala Val Ser Leu Gly
1               5                   10                  15
Glu Arg Ala Thr Ile Asn Cys Arg Ala Asp Lys Ser Val Ser Thr Ser
            20                  25                  30
Thr Tyr Asn Tyr Leu His Trp Tyr Gln Gln Lys Pro Gly Gln Pro Pro
        35                  40                  45
Lys Leu Leu Ile Tyr Leu Ala Ser Asn Leu Ala Ser Gly Val Pro Asp
    50                  55                  60
Arg Phe Ser Gly Ser Gly Ser Gly Thr Asp Phe Thr Leu Thr Ile Ser
65                  70                  75                  80
Ser Leu Gln Ala Glu Asp Val Ala Val Tyr Tyr Cys Gln His Ser Arg
                85                  90                  95
Asp Leu Pro Pro Thr Phe Gly Gln Gly Thr Lys Leu Glu Ile Lys Arg
            100                 105                 110
Thr Val Ala Ala Pro Ser Val Phe Ile Phe Pro Pro Ser Asp Glu Gln
        115                 120                 125
Leu Lys Ser Gly Thr Ala Ser Val Val Cys Leu Leu Asn Asn Phe Tyr
    130                 135                 140
Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
        195                 200                 205
Val Thr Lys Ser Phe Asn Arg Gly Glu Cys
    210                 215
```

<210> 28
<211> 218
<212> PRT
<213> artificial sequence

<220>
<221> PEPTIDE
<222> (1)..(218)
<223> Ab-11 light chain

<400> 28

```
Asp Ile Val Leu Thr Gln Ser Pro Asp Ser Leu Ala Val Ser Leu Gly
1               5                   10                  15
Glu Arg Ala Thr Ile Asn Cys Arg Ala Asn Lys Ser Val Ser Thr Ser
            20                  25                  30
Thr Tyr Asn Tyr Leu His Trp Tyr Gln Gln Lys Pro Gly Gln Pro Pro
        35                  40                  45
Lys Leu Leu Ile Tyr Leu Ala Ser Asn Leu Ala Ser Gly Val Pro Asp
    50                  55                  60
Arg Phe Ser Gly Ser Gly Ser Gly Thr Asp Phe Thr Leu Thr Ile Ser
65                  70                  75                  80
Ser Leu Gln Ala Glu Asp Val Ala Val Tyr Tyr Cys Gln His Ser Arg
                85                  90                  95
Asp Leu Pro Pro Thr Phe Gly Gln Gly Thr Lys Leu Glu Ile Lys Arg
```

```
                100                 105                 110
Thr Val Ala Ala Pro Ser Val Phe Ile Phe Pro Pro Ser Asp Glu Gln
            115                 120                 125
Leu Lys Ser Gly Thr Ala Ser Val Val Cys Leu Leu Asn Asn Phe Tyr
    130                 135                 140
Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
        195                 200                 205
Val Thr Lys Ser Phe Asn Arg Gly Glu Cys
    210                 215
```